# EUROPEAN PATENT APPLICATION

(11) **EP 4 663 752 A1**
(43) Date of publication of application: **17.12.2025**
(21) Application number: 24750627.2
(22) Date of filing: 02.02.2024
(51) Int. Cl.: C12N 9/12, C12N 5/0783, C07K 14/725

(54) **HIGHLY ACTIVE TRANSPOSASE PROTEIN OF TRANSPOSON SYSTEM, AND USE THEREOF**

(30) Priority: 02.02.2023 KR 20230014296
(71) Applicant: Neogentc Corp., Seoul 05505 (KR)
(72) Inventor: LIM, Chae Lyul, Hanam-si, Gyeonggi-do 12904 (KR); CHUNG, Jiwon, Seoul 04746 (KR); KIM, Young-Ae, Seoul 05532 (KR); PARK, Seeun, Seoul 05388 (KR)
(74) Representative: Nederlandsch Octrooibureau
(86) International application number: PCT/KR2024/001633
(87) International publication number: WO 2024/162830

(57) **Abstract**

The present invention relates to a highly active transposase protein of a transposon system, and a use thereof. Genes can be effectively transferred through improvement in transposase activity such that the present invention can be effectively used in the development of genome-modified cell lines expressing various genes. When T cells are transformed using a highly active transposase, according to the present invention, the proportion of cytotoxic T (CD8+ T) cells having anticancer activity increases, and the proportion of memory type T cells of T_{CM} (central memory T cells) and T_{SCM} (stem cell-like memory T cells), having good persistence in the body, increases, and thus it is expected that TCR-T cells and CAR-T cells having good persistence in the body can be produced using the transposon system of the present invention.

## Description

### [Technical Field]

The present invention relates to a highly active transposase protein of a transposon system and use thereof.

This application claims priority to and the benefit of Korean Patent Application No. 10-2023-0014296, filed on February 2, 2023, the disclosure of which is incorporated herein by reference in its entirety.

### [Background Art]

A chimeric antigen receptor (CAR)-T cell is a cell therapeutic that combines the sequence of an antibody that binds to tumor antigens (e.g. CD19) with a domain required for T cell signaling such as CD3/4-1BB/CD28 and inserts them into a T cell. There are various methods of inserting these CAR genes into T cells but most of them use a lentivirus delivery system. A characteristic of lentivirus is that it is capable of continuously expressing genes because it integrates into cellular chromosomes. These lentiviruses are expensive to produce, which is a major factor in increasing the price of the therapeutic, but they have the advantage that they can be used for multiple patients once they are produced.

On the other hand, a personalized T cell receptor-modified T cell therapy (TCR-T) is produced by finding the TCR sequence that reacts to the neoantigen that each patient has and delivering this sequence into the T cell through a gene delivery system. However, since it is personalized, the TCR sequence that is applied to each patient is different, so it is almost impossible to apply it with a lentivirus. Therefore, it is necessary to develop TCR-T cells using a transposon, which is a non-viral vector that is easier to produce than lentivirus, has lower production costs, and can be integrated into the chromosome to continuously express genes.

Accordingly, to meet the above-described needs, the present inventors developed a transposon as a gene delivery vector capable of inserting an exogenous gene into the chromosome (genome) of a target cell, particularly an immune cell, (Korea Patent Publication No. 10-2602485).

Furthermore, as a result of extensive research to increase the gene delivery efficiency through a transposon system, the present inventors improved the activity of the transposase in the transposon system and confirmed that excellent gene transfer efficiency is exhibited, thereby completing the present invention.

### [Disclosure]

### [Technical Problem]

The present inventors completed the present invention by confirming that when the activity of a transposase is improved by optimizing the nucleic acid sequence of the transposase, the gene delivery efficiency through a transposon system is increased.

An object of the present invention is to provide a transposase expression vector including a nucleic acid sequence encoding a transposase represented by SEQ ID NO: 2 or SEQ ID NO: 3.

Another object of the present invention is to provide an mRNA encoding a transposase represented by SEQ ID NO: 12.

Still another object of the present invention is to provide a transposase expressed from the transposase expression vector of the present invention or the mRNA encoding the transposase of the present invention.

Yet another object of the present invention is to provide a transposon system for delivering a target DNA, including: a) a transposon vector including a target DNA inserted therein; and b) a transposase expression vector comprising a nucleic acid sequence encoding the transposase represented by SEQ ID NO: 2 or SEQ ID NO: 3, or an mRNA encoding the transposase represented by SEQ ID NO: 12, or the transposase of the present invention.

Yet another object of the present invention is to provide a transposon kit for delivering a target DNA, including the transposon system for delivering a target DNA according to the present invention and instructions therefor.

Yet another object of the present invention is to provide: A cell into which:
a) a transposon vector including a target DNA inserted therein; and
b) a transposase expression vector including a nucleic acid sequence encoding a transposase represented by SEQ ID NO: 2 or SEQ ID NO: 3, or

an mRNA encoding a transposase represented by SEQ ID NO: 12, or
the transposase of the present invention, is introduced.

Yet another object of the present invention is to provide A method of inserting a target DNA sequence into a genome of a cell, comprising: a step of introducing a) and b) into the cell.
a) a transposon vector including a target DNA inserted therein; and
b) a transposase expression vector including a nucleic acid sequence encoding a transposase represented by SEQ ID NO: 2 or SEQ ID NO: 3, or

an mRNA encoding a transposase represented by SEQ ID NO: 12, or
the transposase of the present invention.

However, technical problems to be solved in the present invention are not limited to the above-described problems, and other problems which are not described herein will be fully understood by those of ordinary skill in the art from the following descriptions.

### [Technical Solution]

To achieve the above object, the present invention provides a transposase expression vector including a nucleic acid sequence encoding the transposase represented by SEQ ID NO: 2 or SEQ ID NO: 3.

In one embodiment of the present invention, the nucleic acid sequence encoding the transposase may further includes a nucleic acid sequence encoding a nuclear localization signal (NLS), but is not limited thereto.

In another embodiment of the present invention, the nucleic acid sequence encoding the nuclear localization signal may be one or more of the nucleic acid sequences represented by SEQ ID NOs: 4 to 11, but is not limited thereto.

In yet another embodiment of the present invention, the nucleic acid sequence encoding the nuclear localization signal may be included in a 5' to 3' direction at a 5' end or 3' end of the nucleic acid sequence encoding the transposase, but is not limited thereto.

Additionally, the present invention provides an mRNA encoding the transposase represented by SEQ ID NO: 12.

In one embodiment of the present invention, the mRNA may be produced by performing *in vitro* transcription using the transposase expression vector of the present invention, but is not limited thereto.

In another embodiment of the present invention, the transposase expression vector may be a transposase expression vector including a nucleic acid sequence encoding the transposase represented by SEQ ID NO: 3, but is not limited thereto.

Additionally, the present invention provides a transposase expressed from the transposase expression vector of the present invention or from the mRNA encoding the transposase of the present invention.

In one embodiment of the present invention, when the transposase expression vector is a transposase expression vector comprising a nucleic acid sequence encoding the transposase represented by SEQ ID NO: 3, the transposase may be expressed from an mRNA prepared by performing *in vitro* transcription using the transposase expression vector, but is not limited thereto.

In another embodiment of the present invention, the transposase may include the amino acid sequence represented by SEQ ID NO: 13, but is not limited thereto.

Additionally, the present invention provides a transposon system for delivering a target DNA, including: a) a transposon vector including a target DNA inserted therein; and b) a transposase expression vector including a nucleic acid sequence encoding the transposase represented by SEQ ID NO: 2 or SEQ ID NO: 3, or an mRNA encoding the transposase represented by SEQ ID NO: 12, an mRNA encoding the transposase of the present invention, or the transposase of the present invention.

In one embodiment of the present invention, the transposon vector and the transposase expression vector, or the mRNA encoding the transposase, or the transposase may be included at a mass ratio of 0.1 to 10:1, but is not limited thereto.

Additionally, the present invention provides a transposon kit for delivering a target DNA, including the transposon system for delivering a target DNA according to the present invention and instructions therefor.

Additionally, the present invention provides: a cell into which:
a) a transposon vector including a target DNA inserted therein; and
b) a transposase expression vector including a nucleic acid sequence encoding a transposase represented by SEQ ID NO: 2 or SEQ ID NO: 3, or

an mRNA encoding a transposase represented by SEQ ID NO: 12, or an mRNA encoding the transposase of the present invention, or
the transposase of the present invention, is introduced.

In one embodiment of the present invention, the target DNA may be cleaved from the transposon vector by the transposase within the cell, and the cleaved target DNA may be inserted into the genome of the cell, but is not limited thereto.

In another embodiment of the present invention, the cell may be selected from the group consisting of T cells, NK cells, B cells, dendritic cells, macrophages, and mast cells, but is not limited thereto.

In yet another embodiment of the present invention, the cell may be co-cultured with feeder cells after the transposon vector is introduced, but is not limited thereto.

In yet another embodiment of the present invention, the feeder cells may be cells irradiated with radiation, but is not limited thereto.

In yet another embodiment of the present invention, the cell may express the target DNA for more than 7 days after introduction of the transposon vector, but is not limited thereto.

Additionally, the present invention a method of inserting a target DNA sequence into a genome of a cell, comprising: a step of introducing a) and b) into the cell.
a) a transposon vector including a target DNA inserted therein; and
b) a transposase expression vector including a nucleic acid sequence encoding a transposase represented by SEQ ID NO: 2 or SEQ ID NO: 3, or

an mRNA encoding a transposase represented by SEQ ID NO: 12, or an mRNA encoding the transposase of the present invention, or
the transposase of the present invention.

In one embodiment of the present invention, the introduction may be performed by electroporation, but is not limited thereto.

In another embodiment of the present invention, the method may further comprise a step of co-culturing the cell into which the transposon vector is inserted with feeder cells after the introduction step, but is not limited thereto.

In yet another embodiment of the present invention, the step of co-culturing with the feeder cells may be performed immediately after the introduction step, but is not limited thereto.

### [Advantageous Effects]

The present invention relates to a highly active transposase protein of a transposon system and use thereof, and may be usefully applied to the development of genome-modified cell lines expressing various genes, as genes may be effectively delivered by improving the activity of the transposase.

In addition, when T cells are transduced using the highly active transposase according to the present invention, the ratio of cytotoxic T (CD8⁺ T) cells having anticancer activity increases, and the ratio of memory-type T cells having excellent *in vivo* persistence, such as T_{CM} (central memory T cells) and T_{SCM} (stem cell-like memory T cells), increases. Therefore, it is expected that TCR-T cells and CAR-T cells with excellent *in vivo* persistence may be produced using the transposon system of the present invention.

### [Description of Drawings]

FIGs. 1 to 3 show the expression of 1G4 TCR in T cells on day 7 (FIG. 1), day 10 (FIG. 2), and day 14 (FIG. 3) after electroporation in each of the following groups: a group in which only electroporation (EP) was performed without plasmids in PBMCs (EP only); a group into which a pBat transposon containing the 1G4 TCR gene and the existing transposase were introduced (existing transposase); and a group into which a pBat transposon containing the 1G4 TCR gene and the optimized transposase were introduced (optimized transposase).
FIG. 4 shows a comparison of the proportion of 1G4 TCR-expressing T cells on day 7, day 10, and day 14 after electroporation in each of the following groups: a group in which only electroporation (EP) was performed without plasmids in PBMCs (EP only); a group into which a pBat transposon containing the 1G4 TCR gene and the existing transposase were introduced (existing transposase); and a group into which a pBat transposon containing the 1G4 TCR gene and the optimized transposase were introduced (optimized transposase).
FIG. 5 shows the CD4⁺ and CD8⁺ cell populations within the 1G4 TCR-expressing T cells on day 7, day 10, and day 14 after electroporation in each of the following groups: a group in which only electroporation (EP) was performed without plasmids in PBMCs (EP only); a group into which a pBat transposon containing the 1G4 TCR gene and the existing transposase were introduced (existing transposase); and a group into which a pBat transposon containing the 1G4 TCR gene and the optimized transposase were introduced (optimized transposase).
FIG. 6 shows a comparison of the proportions of CD4⁺ and CD8⁺ cells within the 1G4 TCR-expressing T cells on day 7, day 10, and day 14 after electroporation in each of the following groups: a group into which a pBat transposon containing the 1G4 TCR gene and the existing transposase were introduced (existing transposase), and a group into which a pBat transposon containing the 1G4 TCR gene and the optimized transposase were introduced (optimized transposase).
FIG. 7 shows the identification of memory-type T cells using CD45RA and CCR7 markers within the 1G4 TCR-expressing T cells on day 7, day 10, and day 14 after electroporation in each of the following groups: a group in which only electroporation (EP) was performed without plasmids in PBMCs (EP only); a group into which a pBat transposon containing the 1G4 TCR gene and the existing transposase were introduced (existing transposase); and a group into which a pBat transposon containing the 1G4 TCR gene and the optimized transposase were introduced (optimized transposase).
FIGs. 8A to 10B show the results of FACS analysis of GFP expression levels on day 1 (FIGs. 8A and 8B), day 7 (FIGs. 9A and 9B), and day 14 (FIGs. 10A and 10B) after electroporation of Jurkat cells with a pBat transposon containing the GFP gene and the existing transposase (P5081 + existing transposase) or with a pBat transposon containing the GFP gene and the optimized transposase (P5081 + optimized transposase).
FIG. 11 shows the results of PCR amplification to obtain a transposase gene optimized for the mRNA format.
FIG. 12 shows the results of enzyme mapping for confirming the structure after performing infusion cloning between an mRNA template (vector) and a transposase (insert) to construct an mRNA template plasmid.
FIG. 13 shows the total number of 1G4 TCR-T cells produced according to the form of transposase gene delivery.
FIG. 14 shows the viability of 1G4 TCR-T cells produced according to the form of transposase gene delivery.
FIG. 15 illustrates the FACS analysis method for comparing GFP gene expression according to the form of transposase gene delivery after electroporation.
FIG. 16 shows the results of FACS analysis of GFP gene expression according to the form of the transposase after electroporation.
FIG. 17 illustrates the FACS analysis method for comparing 1G4 TCR gene expression according to the form of transposase gene delivery after electroporation.
FIG. 18 shows the expression of the 1G4 TCR gene according to the form of the transposase after electroporation.
FIG. 19 shows the classification of memory T cells based on the expression of CD45RO/CD62L markers.
FIG. 20 shows the proportion of memory T cells within the 1G4 TCR-expressing T cells according to the form of transposase gene delivery after electroporation.
FIG. 21 shows the CD4 and CD8 ratios within the 1G4 TCR-expressing T cells according to the form of transposase gene delivery after electroporation.
FIGs. 22 and 23 show portions of the synthesized sequences used as templates for cloning to add an NLS (nuclear localization sequence) gene to the 5' end (FIG. 22) or 3' end (FIG. 23) of the optimized transposase.
FIGs. 24A to 26 show the results of evaluating the efficiency of GFP gene delivery on day 1 (FIGs. 24A and 24B), day 7 (FIGs. 25A and 25B), and day 14 (FIG. 26) after transfection (electroporation) of the optimized transposase to which an NLS (nuclear localization sequence) gene was added at the 5' end or 3' end.
FIG. 27 graphically shows the results of FACS analysis of GFP gene expression on day 1 and day 7 after transfection (electroporation) of the optimized transposase to which an NLS (nuclear localization sequence) gene was added at the 5' end or 3' end.
FIG. 28 graphically shows the results of FACS analysis of high-intensity GFP gene expression on day 1 and day 7 after transfection (electroporation) of the optimized transposase to which an NLS (nuclear localization sequence) gene was added at the 5' end or 3' end.
FIGs. 29A and 29B show the results of evaluating GFP gene delivery efficiency after sorting GFP-expressing cells on day 15, following transfection (electroporation) on day 14 of the optimized transposase to which an NLS (nuclear localization sequence) gene was added at the 5' end or 3' end, and additionally culturing the sorted cells for 9 days.
FIGs. 30A to 31D show the results of evaluating the 1G4 TCR gene delivery efficiency and the CD4/CD8 ratio within 1G4 TCR-expressing T cells on day 7 (FIGs. 30A to 30D) and day 14 (FIGs. 31A to 31D) after transfection (electroporation) of the optimized transposase to which an NLS (nuclear localization sequence) gene was added at the 5' end or 3' end.

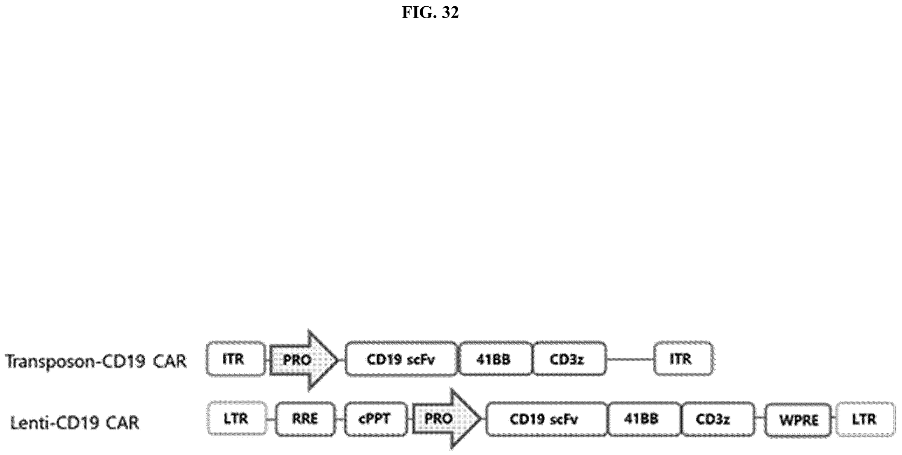

FIG. 32 shows the structure of CD19 CAR vectors of a transposon and a lentivirus.
FIG. 33 shows the co-culture conditions for *in vitroin vitro* killing analysis.
FIG. 34 shows the results of enzyme mapping to confirm the structure of the pBat CD19 CAR transposon plasmid vector.
FIGs. 35 and 36 show the total number of cultured T cells (FIG. 35) and cell viability (FIG. 36) after delivering genes into LK032 PBMCs and LK053 PBMCs using lentivirus and transposon by transduction and electroporation, respectively, for the production of CD19 CAR-T cells and culturing the cells for 7 and 14 days.
FIG. 37 illustrates the FACS analysis method for comparing CD19 CAR protein expression depending on the gene delivery vehicle: lentivirus or transposon.
FIG. 38 shows the results of evaluating CD19 CAR expression in LK032 PBMCs on day 7 and day 14 depending on the gene delivery vehicle: lentivirus or transposon.
FIG. 39 shows the results of evaluating CD19 CAR expression in LK053 PBMCs on day 7 and day 14 depending on the gene delivery vehicle: lentivirus or transposon.
FIG. 40 shows the classification of memory T cells based on the expression of CD45RA/CD62L markers.
FIG. 41 shows the results of evaluating the proportion of memory T cells in T cells from LK032 PBMCs depending on the gene delivery vehicle: lentivirus or transposon.
FIG. 42 shows the results of evaluating the proportion of memory T cells within CD19 CAR-expressing T cells from LK053 PBMCs depending on the gene delivery vehicle: lentivirus or transposon.
FIG. 43 shows the results of evaluating the proportion of memory T cells within CD19 CAR-expressing T cells from LK032 PBMCs and LK053 PBMCs depending on the gene delivery vehicle: lentivirus or transposon.
FIGs. 44 and 45 show the results of evaluating the proportions of CD4⁺-expressing T cells (FIG. 44) and CD8⁺-expressing T cells (FIG. 45) within CD19 CAR-expressing T cells from LK032 PBMCs and LK053 PBMCs depending on the gene delivery vehicle: lentivirus or transposon.
FIGs. 46 and 47 show the average killing percentage of FLAG (CD19 CAR)-expressing T cells transduced using lentivirus (FIG. 46) or transposon (FIG. 47) as the gene delivery vehicle.

### [Modes of the Invention]

The present inventors completed the present invention by confirming that gene delivery efficiency through a transposon system is improved when the activity of a transposase is enhanced by optimizing the nucleic acid sequence of the transposase.

Hereinafter, the present invention will be described in detail.

The present invention provides a transposase expression vector comprising a nucleic acid sequence encoding the transposase represented by SEQ ID NO: 2 or SEQ ID NO: 3.

In the present invention, the term "transposase" refers to an enzyme that recognizes and binds to both ends (in particular, inverted repeat sequences) of a transposon, cleaves the region, and then moves and inserts the gene fragment between the two ends (i.e., the region containing the target DNA) into another location in the chromosome.

The transposase may be introduced into the cell in the form of the protein itself, or in the form of a vector comprising a sequence encoding the transposase protein ("transposase vector," "transposase plasmid," "transposase expression vector," or "transposase expression plasmid") or a nucleic acid molecule (DNA or RNA molecule) comprising a sequence encoding the transposase protein, and may be expressed in the cell after being introduced.

In the present invention, the term "vector" refers to a nucleic acid molecule capable of transporting another linked nucleic acid molecule. Specifically, the vector refers to any vehicle for introducing and/or transferring a nucleotide into a host cell *in vitro,* ex vivo, or *in vivo,* and may be a replicon that can lead to replication of a linked fragment upon binding with another DNA fragment. The term "replicon" refers to any genetic unit that functions as an autonomous unit of DNA replication *in vivo,* i.e., that is capable of replicating under its own control, such as a plasmid, phage, cosmid, chromosome, or virus. The vector may include, but is not limited to, a bacterium, plasmid, phage, cosmid, episome, virus, or an insertable DNA fragment, i.e., a fragment capable of being inserted into the genome of a host cell via homologous recombination.

In the present invention, the term "transposase expression vector" refers to a nucleic acid molecule capable of transporting a nucleic acid sequence encoding a transposase or a template nucleic acid sequence for producing an mRNA encoding the transposase, and may be referred to as a "transposase vector," "transposase plasmid," or "transposase expression plasmid."

According to one example of the present invention, the transposase expression vector comprising the nucleic acid sequence encoding the transposase represented by SEQ ID NO: 2 may comprise the nucleic acid sequence of SEQ ID NO: 14, and the transposase expression vector comprising the nucleic acid sequence encoding the transposase represented by SEQ ID NO: 3 may comprise the nucleic acid sequence of SEQ ID NO: 15, but is not limited thereto.

The vector according to the present invention may be double-stranded DNA in the form of plasmid DNA, linear DNA, hairpin DNA, or minicircle DNA, or may be a recombinant viral vector, but is not limited thereto. As long as the vector comprises a nucleic acid sequence encoding a transposase or a template nucleic acid sequence for producing an mRNA encoding a transposase and is capable of delivering the sequence into a target cell, it may be used without limitation, and one of ordinary skill in the art may select from among various vectors known in the art.

The vector of the present invention may preferably include a promoter as a transcription initiation factor to which an RNA polymerase binds, an optional operator sequence for regulating transcription, an enhancer sequence, a sequence encoding an appropriate mRNA ribosome binding site, and sequences for regulating the termination of transcription and translation, such as a terminator. More preferably, it may further include a polyhistidine tag (an amino acid motif consisting of at least five histidine residues), a signal peptide gene, an endoplasmic reticulum retention signal peptide, and a cloning site, and may also include additional genes such as tag genes and selectable marker genes, for example, antibiotic resistance genes for selecting transformants. In the vector, the sequences of the respective genes are operatively linked to the promoter. As used herein, the term "operatively linked" refers to a functional connection between a nucleotide expression regulatory sequence, such as a promoter sequence, and another nucleotide sequence, whereby the regulatory sequence controls the transcription and/or translation of the other nucleotide sequence.

The vector of the present invention may be constructed using a prokaryotic or eukaryotic cell as a host. For example, when the vector of the present invention is an expression vector and uses a prokaryotic cell as a host, it generally comprises a strong promoter capable of initiating transcription (e.g., pLλ promoter, trp promoter, lac promoter, tac promoter, T7 promoter), a ribosome binding site for initiating translation, and a transcription/translation termination sequence. When a eukaryotic cell is used as the host, the vector may include a eukaryotic origin of replication such as the f1 origin, SV40 origin, pMB1 origin, adenoviral origin, AAV origin, or BBV origin, but is not limited thereto. In addition, a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, or HSV tk promoter) may be used, and the vector generally comprises a polyadenylation sequence as a transcription termination sequence. The vector may also include a signal sequence such as a poly A signal, but is not limited thereto.

Examples of tag genes include, but are not limited to, Avi tag, calmodulin tag, polyglutamate tag, E tag, FLAG tag, HA tag, His tag (polyhistidine tag), myc tag, S tag, SBP tag, IgG-Fc tag, CTB tag, Softag 1 tag, Softag 3 tag, Strep tag, TC tag, V5 tag, VSV tag, and Xpress tag. Preferably, the vector according to the present invention may comprise a myc tag.

In the present invention, the vector may be delivered into a prokaryotic or eukaryotic host cell through various techniques commonly used for introducing exogenous nucleic acids (DNA or RNA) into cells. For example, the vector of the present invention may be introduced into cells by calcium phosphate coprecipitation; electroporation; microfluidics gene editing; nucleofection; cell squeezing; sonoporation; optical transfection; impalefection; gene gun; magnetofection; viral transduction; DEAE-dextran transfection; lipofection; or transfection using a dendrimer, liposome, or cationic polymer, but is not limited thereto.

According to one example of the present invention, the nucleic acid sequence encoding the transposase represented by SEQ ID NO: 2 is a codon-optimized sequence in which the DNA sequence of the existing transposase (pBat transposase, SEQ ID NO: 1) is optimized to enhance the expression of the transposase, particularly in cells such as T cells, and is characterized in that it provides the transposase in the form of (plasmid) DNA, i.e., a vector comprising a sequence encoding the transposase protein.

In the present invention, the nucleic acid sequence encoding the transposase represented by SEQ ID NO: 3 is an optimized sequence in which the DNA sequence of the existing transposase (pBat transposase, SEQ ID NO: 1) is adapted for the mRNA format, and according to one example of the present invention, the nucleic acid sequence encoding the transposase represented by SEQ ID NO: 3 is used to produce an mRNA by performing *in vitro* transcription using a transposase expression vector comprising the sequence, thereby providing the transposase in the form of mRNA. Accordingly, the nucleic acid sequence encoding the transposase represented by SEQ ID NO: 3 is preferably a template nucleic acid sequence for producing an mRNA encoding the transposase.

In the present invention, the nucleic acid sequences represented by SEQ ID NO: 2 or SEQ ID NO: 3 are optimized at the nucleic acid sequence level, and the transposase proteins expressed therefrom are identical to the existing transposase protein.

In the present invention, the term "nucleic acid" or "nucleic acid molecule" broadly includes DNA (gDNA and cDNA) and RNA molecules, and the nucleotide, which is the basic structural unit of the nucleic acid, includes not only natural nucleotides but also analogues with modifications in the sugar or base portion. The nucleotide sequence of the nucleic acid according to the present invention may be modified, and such modifications include addition, deletion, non-conservative substitution, or conservative substitution of nucleotides. The nucleic acid of the present invention also includes nucleotide sequences that exhibit substantial identity to the above nucleotide sequence. Substantial identity refers to a nucleotide sequence that, when aligned with the nucleotide sequence of the present invention to maximize correspondence and analyzed using an algorithm commonly used in the art, exhibits at least 80% homology, more preferably at least 90% homology, and most preferably at least 95% homology.

That is, in the present invention, a polynucleotide consisting of a nucleotide sequence represented by a specific sequence number is not limited to that sequence alone, and variants of the sequence are included within the scope of the present invention. A nucleic acid molecule consisting of a nucleotide sequence represented by a specific sequence number in the present invention includes functional equivalents of the nucleic acid molecule, such as variants in which a portion of the nucleotide sequence of the nucleic acid molecule is modified by deletion, substitution, or insertion, but which are capable of performing the same function as the nucleic acid molecule. Specifically, the polynucleotide disclosed in the present invention may include a nucleotide sequence having at least 70%, more preferably at least 80%, even more preferably at least 90%, and most preferably at least 95% sequence identity with the nucleotide sequence represented by a specific sequence number. For example, the polynucleotide includes those having 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity. The "% sequence identity" for a polynucleotide is determined by comparing a comparison region between two optimally aligned sequences, and the comparison region may include additions or deletions (i.e., gaps) in part of the polynucleotide sequence relative to a reference sequence for optimal alignment of the two sequences (which does not include insertions or deletions).

According to one example of the present invention, the nucleic acid sequence encoding the transposase may further include a nucleic acid sequence encoding a nuclear localization signal (NLS).

In the present invention, a "nuclear localization signal (NLS)" refers to a peptide strand that facilitates the transport of various nuclear proteins into the nucleus after being synthesized in the cytoplasm, and the NLS is well known in the art and may be any known or functional NLS described extensively in the literature. According to one example of the present invention, the nuclear localization signal may be SV40 or C-myc.

In the present invention, the nucleic acid sequence encoding the nuclear localization signal may be one or more of the nucleic acid sequences represented by SEQ ID NOs: 4 to 11, and specifically, SEQ ID NOs: 4 to 9 are sequences encoding SV40, and SEQ ID NOs: 10 to 11 are sequences encoding C-myc. The nuclear localization signal may be included in one or more copies and may include a combination of two or more types of nuclear localization signals.

According to one example of the present invention, the nucleic acid sequences encoding the nuclear localization signals have also been optimized, and the optimized nucleic acid sequence for SV40 may comprise the nucleotide sequence of SEQ ID NO: 9, and the optimized nucleic acid sequence for C-myc may comprise the nucleotide sequence of SEQ ID NO: 11.

Since the optimization was performed at the nucleic acid sequence level, the SV40 proteins expressed from SEQ ID NOs: 4 to 9 and the C-myc proteins expressed from SEQ ID NOs: 10 to 11 are identical, respectively.

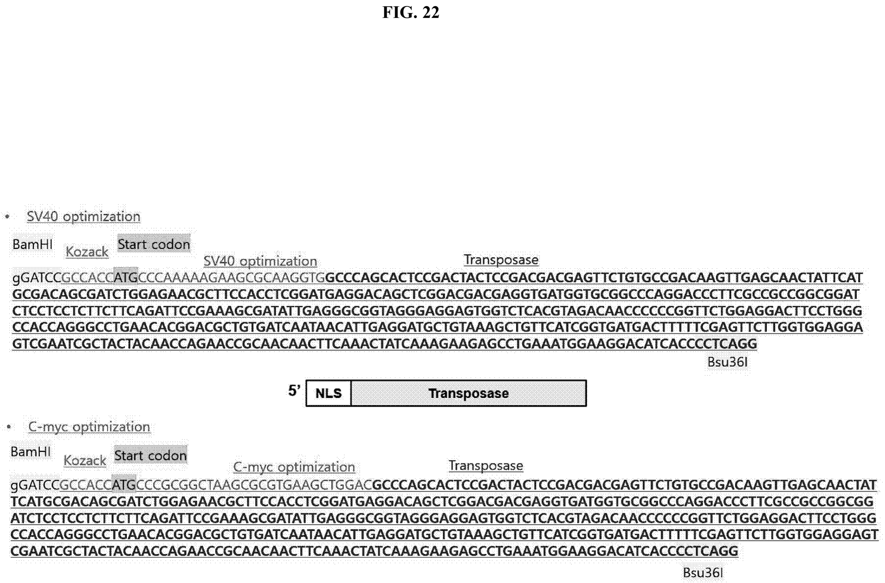

According to one example of the present invention, the nucleic acid sequence encoding the nuclear localization signal may be included in the 5' or 3' end of the nucleic acid sequence encoding the transposase in the 5' to 3' direction, Specifically, in order to enhance the activity of the transposase, when the SV40 or C-myc nucleic acid sequence is added to the 5' end of the transposase nucleic acid sequence, a Kozak sequence (5'-GCCACC-3') may be additionally included upstream of the start codon of the transposase, but is not limited thereto (see FIG. 22).

In the present invention, the nucleic acid sequence encoding the transposase represented by SEQ ID NO: 2, to which the optimized SV40 nucleic acid sequence (SEQ ID NO: 9) is added at the 5' end, may comprise or consist of the nucleic acid sequence of SEQ ID NO: 16.

In the present invention, the nucleic acid sequence encoding the transposase represented by SEQ ID NO: 2, to which the optimized C-myc nucleic acid sequence (SEQ ID NO: 11) is added at the 5' end, may comprise or consist of the nucleic acid sequence of SEQ ID NO: 18.

In the present invention, the nucleic acid sequence encoding the transposase represented by SEQ ID NO: 2, to which the optimized SV40 nucleic acid sequence (SEQ ID NO: 9) is added at the 3' end, may comprise or consist of the nucleic acid sequence of SEQ ID NO: 20.

In the present invention, the nucleic acid sequence encoding the transposase represented by SEQ ID NO: 2, to which the optimized C-myc nucleic acid sequence (SEQ ID NO: 11) is added at the 3' end, may comprise or consist of the nucleic acid sequence of SEQ ID NO: 22.

In the present invention, a transposase expression vector comprising a nucleic acid sequence encoding the transposase represented by SEQ ID NO: 2, in which an optimized SV40 nucleic acid sequence (SEQ ID NO: 9) is added at the 5' end and a Kozak sequence (5'-GCCACC-3') is included upstream of the start codon of the transposase, may comprise or consist of the nucleic acid sequence of SEQ ID NO: 17 (see FIG. 22).

In the present invention, a transposase expression vector comprising a nucleic acid sequence encoding the transposase represented by SEQ ID NO: 2, in which an optimized C-myc nucleic acid sequence (SEQ ID NO: 11) is added at the 5' end and a Kozak sequence (5'-GCCACC-3') is included upstream of the start codon of the transposase, may comprise or consist of the nucleic acid sequence of SEQ ID NO: 19 (see FIG. 22).

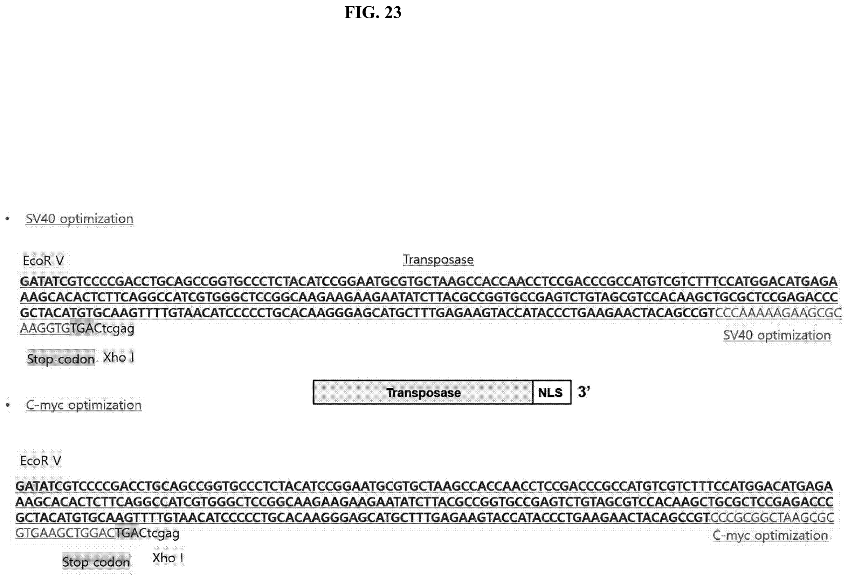

In the present invention, a transposase expression vector comprising a nucleic acid sequence encoding the transposase represented by SEQ ID NO: 2, to which the optimized SV40 nucleic acid sequence (SEQ ID NO: 9) is added at the 3' end, may comprise or consist of the nucleic acid sequence of SEQ ID NO: 21 (see FIG. 23).

In the present invention, a transposase expression vector comprising a nucleic acid sequence encoding the transposase represented by SEQ ID NO: 2, to which the optimized C-myc nucleic acid sequence (SEQ ID NO: 11) is added at the 3' end, may comprise or consist of the nucleic acid sequence of SEQ ID NO: 23 (see FIG. 23).

The present invention also provides an mRNA encoding the transposase represented by SEQ ID NO: 12, which may be produced by performing *in vitro* transcription using a transposase expression vector according to the present invention, and specifically, the transposase expression vector may be a transposase expression vector comprising a nucleic acid sequence encoding the transposase represented by SEQ ID NO: 3.

The present invention also provides a transposase expressed from a transposase expression vector according to the present invention or from an mRNA encoding the transposase according to the present invention, and according to one example of the present invention, since the nucleic acid sequence encoding the transposase included in the transposase expression vector or the template nucleic acid sequence for producing the mRNA encoding the transposase is optimized at the nucleic acid level, it comprises the amino acid sequence represented by SEQ ID NO: 13, which is identical to that of the existing transposase protein.

In the present invention, when the transposase expression vector comprises a nucleic acid sequence encoding the transposase represented by SEQ ID NO: 3, the transposase may be expressed from an mRNA produced by performing *in vitro* transcription using the transposase expression vector, but is not limited thereto.

The present invention also provides a transposon system for delivering a target DNA, comprising:
a) a transposon vector into which the target DNA is inserted; and
b) a transposase expression vector comprising a nucleic acid sequence encoding the transposase represented by SEQ ID NO: 2 or SEQ ID NO: 3, or an mRNA encoding the transposase represented by SEQ ID NO: 12, or a transposase according to the present invention.

In the present invention, the term "transposon" refers to a polynucleotide that is capable of changing its position within the genome and integrating into a target site (e.g., the genome of a cell or extrachromosomal DNA) by excising a specific gene from a donor polynucleotide (e.g., a vector). The transposon is a polynucleotide comprising a nucleic acid sequence flanked on both sides by cis-acting nucleotide sequences, wherein at least one cis-acting nucleotide sequence is located at the 5' end of the nucleic acid sequence and at least one cis-acting nucleotide sequence is located at the 3' end of the nucleic acid sequence. The cis-acting nucleotide sequence includes at least one inverted repeat (IR) at each end of the transposon, referred to as an inverted terminal repeat (ITR), to which the transposase binds.

According to one example of the present invention, the transposon vector and the transposase expression vector, or the mRNA encoding the transposase, or the transposase may be included in a mass ratio of 0.1 to 10:1, 0.1 to 8:1, 0.1 to 5:1, 0.1 to 3:1, 0.1 to 1:1, 0.5 to 10:1, 0.5 to 8:1, 0.5 to 5:1, 0.5 to 3:1, 0.5 to 1:1, 1 to 10:1, 1 to 8:1, 1 to 5:1, 1 to 4:1, 1 to 3:1, 1 to 2:1, or 1:1, but is not limited thereto.

The transposon vector of the present invention may be constructed according to conventional methods known in the art, with reference to Korean Patent No. 10-2602485.

In the present invention, the term "target DNA" refers to an exogenous DNA molecule to be delivered into a cell using a transposon. The target DNA only needs to be capable of being inserted into a transposon vector, delivered into a target cell, and expressed thereafter. That is, it is apparent that the target DNA is not limited to a specific type of DNA, and one of ordinary skill in the art may select any desired target DNA without limitation depending on the intended use.

In one embodiment of the present invention, the target DNA sequence may encode an antibiotic resistance protein, therapeutic polypeptide, siRNA, miRNA, reporter protein, cytokine, kinase, antigen, antigen-specific receptor, cytokine receptor, suicide polypeptide, recombinant antibody, neutralizing antibody against various viruses or other antigens, or a portion thereof, and for example, may encode a chimeric antigen receptor (CAR), a T cell receptor (TCR), or a portion thereof, but is not limited thereto.

In the present invention, the term "therapeutic polypeptide" refers to a polypeptide or peptide that is effective in preventing, ameliorating, and/or treating any disease, and one of ordinary skill in the art may appropriately select a polypeptide exhibiting therapeutic effects for a specific disease depending on the intended use. The disease is not limited to any specific type; however, in one embodiment, the disease may be cancer.

The present invention also provides a transposon kit for delivering a target DNA, comprising a transposon system for delivering a target DNA according to the present invention and instructions.

The instructions may include a pamphlet, recording, diagram, or other presentation medium (e.g., CD, VCD, DVD, USB) that may be used to communicate or inform how to use the transposon system of the present invention. The instructions may be affixed to a container or may be packaged separately from the container containing the transposon system of the present invention.

The kit may further include a container for containing the transposon system of the present invention.

The kit may further include a buffer solution for stabilizing the transposon system and/or for performing cell transfection. The buffer solution may be, for example, phosphate-buffered saline, Tris-based saline, Tris-EDTA buffer, piperazineethanesulfonic acid buffer, or N,N-bis(2-hydroxyethyl)-2-aminoethanesulfonic acid (BES) buffer, but is not limited thereto.

The present invention also provides:
A cell into which:
   a) a transposon vector including a target DNA inserted therein; and
   b) a transposase expression vector including a nucleic acid sequence encoding a transposase represented by SEQ ID NO: 2 or SEQ ID NO: 3, or
an mRNA encoding a transposase represented by SEQ ID NO: 12 or
the transposase of the present invention, is introduced.

In the present invention, the cell may be one in which the target DNA is excised from the transposon vector by the transposase within the cell and the excised target DNA is integrated into the genome of the cell. That is, the target DNA may be inserted into the genome of a target cell by the transposon and transposase of the present invention and stably expressed. The target DNA inserted into the genome of the cell may be expressed in the cell for 5 days or more, 7 days or more, 10 days or more, 15 days or more, 20 days or more, or 30 days or more after the transposon vector and the transposase are introduced into the cell, but is not limited thereto.

That is, the present invention provides a genetically modified cell in which the target DNA is inserted into the genome by the transposon. In the present invention, the term "modification" refers to any manipulation of a cell that causes a detectable change in the cell, and the modification includes, but is not limited to, inserting heterologous or homologous polynucleotides and/or polypeptides into the cell, and mutating endogenous polynucleotides and/or polypeptides of the cell.

In one embodiment of the present invention, the cell may be one or more immune cells selected from the group consisting of T cells, B cells, natural killer (NK) cells, monocytes, macrophages, eosinophils, mast cells, basophils, myeloid cells such as granulocytes, and dendritic cells; or may be a stem cell derived from bone marrow, adipose tissue, peripheral blood, umbilical cord blood, or dental pulp, but is not limited thereto. Additionally, the cell may be a cell derived from an insect, plant, fish, or mammal, particularly a human-derived cell, but is not limited thereto.

According to one embodiment of the present invention, the T cell may be activated by CD3/CD28 beads or the like prior to the introduction of the transposon vector, but is not limited thereto.

As used herein, the term "immune cell" collectively refers to cells that play a role in the immune response.

Additionally, the cell may be co-cultured with feeder cells after the introduction of the transposon vector and the transposase (protein or nucleic acid molecule). The feeder cell refers to a supporting cell that does not proliferate itself but provides extracellular secretions such as growth factors to enable the proliferation of the cell into which the target DNA has been introduced. The feeder cell is not limited to a specific type, and any cell known in the art to function as a feeder cell may be used without limitation. Non-limiting examples include fibroblasts, human bone marrow-derived mesenchymal cells, human amniotic epithelial cells, adipose-derived mesenchymal stem cells, and melanoma cells (A375 cells). Preferably, the feeder cells may be irradiated in advance prior to co-culture with the cell into which the target DNA has been introduced.

Co-culture with the feeder cells may particularly contribute to improving the efficiency of gene delivery, as well as the proliferation of the cells into which the gene has been introduced and the expression rate of the gene, when CAR or TCR is introduced into immune cells using the transposon system of the present invention. For example, when the cell is a T cell, it may be activated using TransAct or Dynabeads.

Co-culture with the feeder cells is preferably performed immediately after the transposon vector and the transposase are introduced into the cells by electroporation or the like, but is not limited thereto, and may be carried out within 1 to 10 days, 1 to 5 days, 1 to 3 days, 1 to 2 days, within 1 day, within 20 hours, within 10 hours, within 5 hours, within 3 hours, within 1 hour, within 30 minutes, or within 10 minutes after the introduction.

The present invention also provides
A method of inserting a target DNA sequence into a genome of a cell, comprising: a step of introducing a) and b) into the cell.
   a) a transposon vector including a target DNA inserted therein; and
   b) a transposase expression vector including a nucleic acid sequence encoding a transposase represented by SEQ ID NO: 2 or SEQ ID NO: 3, or
an mRNA encoding a transposase represented by SEQ ID NO: 12 or
the transposase of the present invention. The method may further comprise a step of co-culturing the cell into which the transposon vector has been introduced with feeder cells after the introduction step.

As used herein, the term "introduction" refers to the delivery of a polynucleotide (e.g., a transposon vector or a transposase vector) into a cell or organism. The nucleic acid of the polynucleotide may be in the form of naked DNA or RNA, may be associated with various proteins, or may be incorporated into a vector. The term "introduction" as used herein is intended to have the broadest possible meaning, and includes, for example, transfection methods (introducing a polynucleotide into a eukaryotic cell by physical and/or chemical treatment), transformation methods (introducing a polynucleotide into a prokaryotic cell by physical and/or chemical treatment), viral methods/viral transduction methods (introducing a polynucleotide into a eukaryotic and/or prokaryotic cell via a virus or viral vector), conjugation methods (introducing a polynucleotide from one cell into another through direct cell-to-cell contact or via a cytoplasmic bridge), and fusion methods (introducing by fusion of two cells, including homotypic or heterotypic cell fusion). Preferably, the introduction is performed by electroporation.

The present invention also provides compositions for various uses comprising, as an active ingredient, a cell into which a target DNA has been inserted into the genome by the transposon vector according to the present invention. The cell may be autologous or allogeneic.

In one embodiment of the present invention, a pharmaceutical composition for the prevention or treatment of an immune-related disease comprising the cell of the present invention as an active ingredient is provided.

As used herein, the term "immune-related disease" refers to a disease and/or condition in which the immune system is involved in the pathogenesis of the disease or in which proper stimulation or suppression of the immune system may lead to treatment and/or prevention of the disease. Examples of immune-related diseases that may be treated by the present invention include, but are not limited to, tumors, infectious diseases, allergies, autoimmune diseases, graft-versus-host disease, or inflammatory diseases.

The inventors confirmed through specific examples that the CAR T cells generated using the transposon of the present invention differentiate into cytotoxic T cells and memory T cells in response to an antigen. Therefore, those skilled in the art may generate genetically modified cells with enhanced immune function by delivering an appropriate antigen-specific CAR or TCR gene into immune cells using the transposon of the present invention, and such cells may be used for the prevention or treatment of immune-related diseases.

For example, a person skilled in the art may insert a gene encoding a target antigen into the transposon of the present invention and deliver it into immune cells to enhance the immune function of the cells against the antigen. Enhancing immune function may mean, for example, activating the functions of antigen-presenting cells, natural killer cells, and T cells (particularly cytotoxic T cells) against the antigen, or modulating the activities of regulatory T cells, myeloid-derived suppressor cells (MDSCs), or M2 macrophages, but is not limited thereto.

Hereinafter, preferred examples are presented to help understand the present invention. However, the following examples are provided only to help understand the present invention more easily, and the contents of the present invention are not limited by the following examples.

### [Examples]

### Example A. Optimization of pBat transposase

In order to further increase the gene delivery efficiency of the transposon system, the DNA sequence of the existing transposase was optimized using Genscript's "OptimumGene Codon Optimization" program for good expression in cells (especially, T cells).

Furthermore, in order to improve the gene delivery efficiency of the transposon system by solving problems that may occur due to the DNA type transposase, an mRNA type transposase, which has lower *in vivo* genotoxicity and a relatively short half-life compared to the DNA type of transposase, was prepared (Example D).

**[Table 1]**

| **transposase** | **SEQUENCE (5'→3')** |
|---|---|
| **Existing transposase (SEQ ID NO: 1)** | GCGCAACACTCAGATTACTCCGACGATGAATTTTGTGCTGACAAACTGT CCAATTATTCATGCGATAGCGACCTCGAAAACGCTTCCACGTCTGATGA AGATAGCAGCGATGATGAAGTAATGGTGAGGCCTCGCACCCTCCGCCG TCGCCGCATCAGCTCTTCGAGCTCTGATTCTGAATCCGATATTGAGGGT GGCCGCGAGGAGTGGTCCCACGTAGACAATCCGCCGGTGCTGGAGGAC TTCCTAGGCCACCAAGGTCTGAACACTGACGCAGTAATCAACAATATCG AAGATGCAGTTAAACTGTTTATCGGTGACGATTTCTTCGAGTTTCTGGT GGAGGAATCTAACCGGTACTATAACCAGAATCGTAATAACTTCAAGCTC TCTAAAAAGTCTCTGAAGTGGAAGGACATCACCCCTCAGGAGATGAAA AAGTTCCTCGGTCTGATCGTTCTGATGGGCCAAGTTCGCAAGGATCGTC GTGACGACTATTGGACTACCGAACCGTGGACGGAAACTCCATACTTTGG CAAGACCATGACTCGTGACCGTTTCCGTCAGATCTGGAAGGCCTGGCA CTTCAATAACAACGCTGACATTGTCAACGAGTCTGATCGTCTGTGTAAG GTTCGCCCTGTGCTGGATTACTTCGTTCCAAAATTCATTAACATTTACAA ACCACATCAGCAGCTGTCCCTGGATGAGGGCATCGTGCCGTGGCGCGG CCGCCTGTTCTTCCGTGTCTATAATGCTGGCAAGATTGTGAAGTACGGT ATCCTGGTTCGCCTGCTGTGCGAAAGCGACACTGGCTACATCTGTAACA TGGAGATCTACTGCGGCGAGGGCAAACGTCTCCTCGAAACTATCCAGA CCGTCGTGTCTCCATACACGGATTCCTGGTATCATATTTACATGGATAAC TATTATAACAGCGTGGCTAACTGTGAAGCTCTGATGAAAAATAAGTTCC GTATTTGCGGTACTATCCGTAAGAATCGTGGAATTCCGAAAGATTTCCA GACCATCTCCCTGAAAAAGGGTGAAACTAAGTTCATTCGCAAAAACGA CATCCTCCTGCAAGTCTGGCAGTCTAAAAAGCCTGTATATCTGATCTCAT CTATTCACAGCGCTGAAATGGAAGAATCTCAGAACATTGATCGCACCTC CAAGAAAAAGATCGTCAAACCGAATGCATTGATTGATTACAACAAGCA CATGAAGGGCGTTGATCGTGCTGACCAGTACCTGTCTTATTACTCTATCC TGCGCCGTACTGTGAAGTGGACTAAACGTCTCGCTATGTACATGATTAA TTGTGCGCTGTTCAATTCTTACGCTGTGTATAAAAGCGTGCGTCAGCGC AAAATGGGCTTTAAAATGTTCCTGAAGCAGACGGCTATTCACTGGCTGA CCGACGATATTCCGGAAGATATGGACATTGTCCCGGATCTCCAGCCGGT ACCGAGCACCAGCGGTATGCGTGCTAAACCTCCGACTAGTGATCCGCCT TGCCGTCTGTCTATGGATATGCGTAAGCATACCCTGCAGGCAATTGTGG GCTCTGGCAAAAAGAAAAATATCCTGCGTCGTTGCCGCGTATGCTCTGT ACACAAACTGCGTTCTGAGACTCGTTATATGTGTAAATTTTGCAATATTC CACTCCACAAGGGTGCGTGCTTCGAGAAGTACCATACGCTGAAGAACT ATTCTAGA |
| **plasmid DNA sequence of Optimized transposase (SEQ ID NO: 2)** | GCCCAGCACTCCGACTACTCCGACGACGAGTTCTGTGCCGACAAGTTG AGCAACTATTCATGCGACAGCGATCTGGAGAACGCTTCCACCTCGGATG AGGACAGCTCGGACGACGAGGTGATGGTGCGGCCCAGGACCCTTCGC CGCCGGCGGATCTCCTCCTCTTCTTCAGATTCCGAAAGCGATATTGAGG GCGGTAGGGAGGAGTGGTCTCACGTAGACAACCCCCCGGTTCTGGAGG ACTTCCTGGGCCACCAGGGCCTGAACACGGACGCTGTGATCAATAACA TTGAGGATGCTGTAAAGCTGTTCATCGGTGATGACTTTTTCGAGTTCTT GGTGGAGGAGTCGAATCGCTACTACAACCAGAACCGCAACAACTTCAA ACTATCAAAGAAGAGCCTGAAATGGAAGGACATCACCCCTCAGGAGAT GAAGAAGTTCCTTGGTCTCATCGTGCTCATGGGCCAGGTGCGGAAAGA CCGCAGGGACGATTATTGGACCACAGAACCGTGGACGGAGACCCCCTA TTTCGGGAAAACAATGACGCGCGACCGCTTCCGGCAAATTTGGAAGGC CTGGCACTTTAACAACAACGCGGACATCGTCAATGAGAGCGACCGCCT CTGTAAGGTGCGCCCCGTCCTGGACTACTTCGTCCCTAAGTTCATTAAC ATCTACAAGCCCCACCAGCAGCTGTCCTTGGATGAGGGCATCGTGCCGT GGAGAGGACGCCTGTTTTTCCGCGTGTACAATGCGGGCAAGATTGTGA AGTACGGCATCCTGGTGCGCCTGCTGTGCGAGTCCGACACCGGCTACAT CTGCAACATGGAGATCTATTGCGGGGAGGGGAAGCGGCTGCTGGAGAC TATCCAGACGGTGGTGTCCCCATACACCGACAGTTGGTACCATATATACA TGGATAACTACTACAACTCCGTGGCCAATTGCGAGGCCCTGATGAAAAA CAAGTTCCGTATCTGTGGCACCATTCGCAAAAACCGAGGCATCCCTAAA GACTTCCAGACCATCTCTCTGAAGAAAGGGGAGACCAAGTTCATCCGC AAGAACGACATCTTACTGCAGGTCTGGCAAAGTAAGAAGCCCGTTTAC CTAATCTCGTCCATTCATTCTGCGGAGATGGAAGAGTCTCAGAATATTG ACCGCACTTCTAAGAAGAAGATCGTTAAGCCTAACGCGCTCATTGACTA CAACAAGCACATGAAAGGTGTCGATCGCGCAGACCAGTACCTCTCGTA TTACAGCATCCTGCGTCGTACCGTGAAATGGACCAAGAGATTGGCCATG TATATGATCAACTGCGCCCTGTTCAACAGCTACGCGGTGTACAAGTCGG TGCGCCAGCGCAAGATGGGCTTTAAAATGTTCCTGAAGCAGACTGCTAT CCACTGGCTCACTGATGACATACCTGAAGACATGGATATCGTCCCCGAC CTGCAGCCGGTGCCCTCTACATCCGGAATGCGTGCTAAGCCACCAACCT CCGACCCGCCATGTCGTCTTTCCATGGACATGAGAAAGCACACTCTTCA GGCCATCGTGGGCTCCGGCAAGAAGAAGAATATCTTACGCCGGTGCCG AGTCTGTAGCGTCCACAAGCTGCGCTCCGAGACCCGCTACATGTGCAA GTTTTGTAACATCCCCCTGCACAAGGGAGCATGCTTTGAGAAGTACCAT ACCCTGAAGAACTACAGCCGT |
| **mRNA sequence of Optimized transposase (SEQ ID NO: 3)** | GCCCAGCATAGCGATTATTCTGATGACGAGTTTTGCGCTGATAAGCTGA GCAACTACTCCTGCGACTCCGACCTGGAAAACGCCTCTACCAGCGATG AAGACTCTAGCGACGATGAAGTGATGGTGAGACCTAGAACACTGAGAA GGCGGAGAATCTCTTCCTCTTCCAGCGACTCCGAGAGCGACATCGAGG GAGGCCGCGAGGAATGGAGCCACGTGGACAATCCTCCTGTGCTGGAAG ATTTTCTGGGCCACCAGGGCCTGAACACCGACGCCGTGATCAACAATAT CGAGGATGCTGTGAAACTGTTCATCGGTGACGACTTCTTCGAGTTCCTG GTTGAGGAATCTAATAGATACTACAACCAGAACCGGAACAACTTCAAA CTCTCCAAGAAGAGCCTGAAGTGGAAGGACATCACCCCTCAGGAGATG AAGAAATTCCTGGGACTGATCGTGCTGATGGGCCAGGTTCGCAAAGAC AGACGGGATGACTACTGGACAACCGAACCTTGGACCGAGACACCTTAC TTCGGCAAGACCATGACCAGAGACCGATTTAGACAAATCTGGAAGGCC TGGCACTTCAACAACAATGCTGACATCGTGAACGAGTCTGATAGACTGT GCAAGGTGCGGCCTGTGCTGGACTATTTCGTGCCTAAGTTCATCAACAT CTACAAGCCCCACCAGCAGCTGTCTCTGGACGAGGGCATCGTGCCCTG GCGGGGCAGACTGTTCTTCAGAGTGTACAACGCCGGCAAGATCGTGAA GTACGGCATTCTGGTGCGGCTACTGTGCGAGAGCGACACCGGCTACATC TGCAACATGGAAATCTACTGCGGCGAGGGCAAACGGCTCCTGGAAACC ATCCAGACCGTGGTGTCCCCTTATACAGACAGCTGGTATCACATCTACAT GGATAACTACTACAATAGCGTGGCCAACTGCGAGGCCCTGATGAAGAA CAAGTTCCGGATCTGTGGCACCATCAGAAAGAACCGGGGCATTCCTAA GGACTTCCAGACTATCAGCCTGAAGAAGGGCGAGACAAAGTTTATTAG GAAGAACGACATCCTGCTGCAAGTGTGGCAGAGCAAAAAACCCGTTTA CCTGATCAGCAGCATCCACAGCGCCGAGATGGAAGAGTCTCAGAACAT CGATAGAACCAGCAAGAAAAAGATCGTGAAACCAAACGCACTGATCG ACTACAACAAGCACATGAAGGGCGTGGATAGAGCCGATCAGTACCTGA GCTACTACAGCATCCTGAGAAGGACAGTCAAGTGGACCAAGAGACTTG CTATGTACATGATCAACTGTGCCCTGTTCAACAGCTACGCCGTGTACAA GAGCGTGCGGCAGAGAAAGATGGGATTTAAGATGTTCCTGAAACAGAC AGCCATCCACTGGCTGACCGACGACATCCCTGAGGACATGGACATCGT CCCCGACCTGCAACCCGTGCCTAGCACCAGCGGCATGCGGGCCAAGCC ACCTACCAGTGACCCCCCATGTAGACTGAGCATGGACATGAGAAAACA TACACTGCAAGCCATCGTCGGCAGCGGAAAGAAAAAGAATATCCTGAG AAGATGCAGAGTGTGCAGCGTGCACAAGCTGAGAAGCGAGACTCGGT ACATGTGCAAGTTCTGTAATATCCCCCTGCACAAGGGAGCCTGCTTCGA GAAGTACCACACCCTGAAGAACTACAGCAGA |

### Example B. Confirmation of gene delivery efficiency of pBat optimized transposase with optimized DNA sequence

In order to further increase the gene delivery efficiency of the pBat transposon system, the DNA sequence of the transposase was optimized for good expression in cells, and the gene delivery efficiency was compared according to the use of the existing transposase and the optimized transposase.

### [Method]

### 1. DNA and cells

To confirm the gene delivery efficiency of pBat optimized transposase with the optimized DNA sequence, pBat transposon 3M3-5M3-1G4 TCR (1.8 µg/µL) was used as a pBat transposon plasmid vector, and a pBat transposase plasmid (1.5 µg/µL) and a pBat optimized transposase plasmid (1.4 µg/µL) were used as pBat transposase plasmid vectors for the expression of transposase.

Peripheral blood mononuclear cells (PBMC) (LK045) isolated from a healthy human and A375 (ATCC, Cat no. CRL-1619, Lot no. 70032966) irradiated at 50 Gy were used as target cells.

### 2. PBMC electroporation

Gene transfer into cells was carried out via electroporation. The specific method is described below.
1) 1.5 mL tubes were prepared according to the electroporation conditions shown in Table 2 below, and 5 µg of each of the transposase plasmid and transposon plasmid was added per 5.0 x 10⁶ PBMCs according to the conditions.

**[Table 2]**

| **No.** | **Conditions** | **Transposon plasmid** | **Transposase plasmid** | **n** |
|---|---|---|---|---|
| 1 | Electroporation (EP) only | - | - | 2 |
| 2 | Existing transposase | 3M3-5M3-1G4 TCR | Existing plasmid | 2 |
| 3 | Optimized transposase | 3M3-5M3-1G4 TCR | Optimized plasmid | 2 |

2) 5.0 x 10⁶ PBMCs were added each to the 1.5 mL tubes of 1).
3) The cell suspension of 5.0 x 10⁶ cells/50 µL from 2) was carefully added to the OC100 x 2 assembly to avoid creating bubbles.
4) The Resting T cell 14-3 protocol was selected from the MaxCyte STx for electroporation.
5) The OC100 x 2 assembly from 3) was inserted into the STx, and the protocol was carried out for electroporation.
6) After electroporation, the cell suspension (5.0 x 10⁶ cells/50 µL/well) from the OC100 x 2 assembly was transferred to a T25 flask.
7) The OC100 x 2 assembly from 6) was washed with 50 µL of AlyS medium and added to each T25 flask.
8) After recovering the electroporated cells for 20 minutes in a 37 °C, 5% CO₂ incubator, 2.0 x 10⁶ irradiated A375 cells were added to each flask, and a medium (ALyS + 3% HS + 200 IU/mL IL-2) was added so that the total volume became 3 mL.
9) Culture was performed in a 37 °C, 5% CO₂ incubator.

### 3. T cell culture and storage after electroporation

1) Three days after electroporation, 5 mL of medium (ALyS + 3% HS + 200 IU/mL IL-2) was added, and culture was performed in an incubator at 37 °C, 5% CO₂.
2) Six days after electroporation, 10 mL of medium (ALyS + 3% HS + 200 IU/mL IL-2) was added, and culture was performed in an incubator at 37 °C, 5% CO₂.
3) Seven days after electroporation, the cultured cells were suspended, and 3 mL of the suspension was collected from each flask for fluorescence-activated cell sorting (FACS) analysis.
4) Eight days after electroporation, the cultured cells were suspended, and 8 mL of the suspension from each flask was transferred to a new T25 flask to split into two flasks, and 8 mL of medium (ALyS + 3% HS + 200 IU/mL IL-2) was added to each flask, and culture was performed in an incubator at 37 °C, 5% CO₂.
5) Ten days after electroporation, the cultured cells were suspended, and 2 mL of the suspension from each flask was collected for FACS analysis. The cells remaining in the T25 flask were transferred to each T75 flask, and 10 mL of medium (ALyS + 3% HS + 200 IU/mL IL-2) was added to each flask, and culture was performed in an incubator at 37 °C, 5% CO₂.
6) Thirteen days after electroporation, 10 mL of medium (ALyS + 3% HS + 200 IU/mL IL-2) was added, and culture was performed in an incubator at 37 °C, 5% CO₂.
7) Fourteen days after electroporation, the cultured cells were suspended, and 2 mL of the suspension from each flask was collected for FACS analysis.
8) Fourteen days after electroporation, the cells remaining in the flasks of 7) were transferred to each 50 mL conical tubes.
9) Centrifugation was performed at 1,500 rpm for five minutes at room temperature, and the supernatant was removed.
10) Cell pellet was suspended in 5 mL of ALyS medium, and cell counting was performed.
11) Centrifugation was performed at 1,500 rpm for five minutes at room temperature, and the supernatant was removed.
12) The cell pellet was suspended in 1 mL of CS10, and the suspension was frozen using Protocol 6 of controlled rate freezer (CRF) (ThermoFisher, CryoMed TSCM34PV, S/N 300503011, 300503015) and stored in a nitrogen tank.

### 4. FACS analysis

FACS analysis was performed 7, 10, and 14 days after electroporation.
1) Cells were suspended in each group's flask and transferred to FACS tubes in an amount of 2 to 3 mL each.
2) Centrifugation was performed at 4 °C and 1,500 rpm for five minutes, the supernatant was removed, and cells were suspended with 1 mL of FACS buffer (phosphate buffered saline (PBS) + 2% fetal bovine serum (FBS)).
3) Step 2) was repeated once more for washing.
4) Centrifugation was performed at 4 °C and 1,500 rpm for five minutes, and the supernatant was removed.
5) 5 µL of human TruStain FcX + 95 µL of FACS buffer was added to each tube, and incubation was performed at room temperature for five minutes.
6) 2 µL of each antibody (anti-CD3, anti-CD4, anti-CD8, anti-CD45RA, anti-CCR7, anti-mTCRβ) was added per tube, and incubation was performed for 30 minutes at room temperature in a dark place.
7) 1 mL of FACS buffer (PBS + 2% FBS) was added to each tube, and centrifugation was performed at 4 °C and 1,500 rpm for five minutes.
8) The supernatant removed, and then cells were suspended with 1 mL of FACS buffer (PBS + 2% FBS).
9) Centrifugation was performed at 4 °C and 1,500 rpm for five minutes, and the supernatant was removed.
10) Cells were suspended with 200 µL of FACS buffer (PBS + 2% FBS) with or without 1x 4',6-diamidino-2-phenylindole (DAPI), and a FACS analysis was performed.

### [Results]

### 1. Analysis of 1G4 TCR expression seven days after electroporation

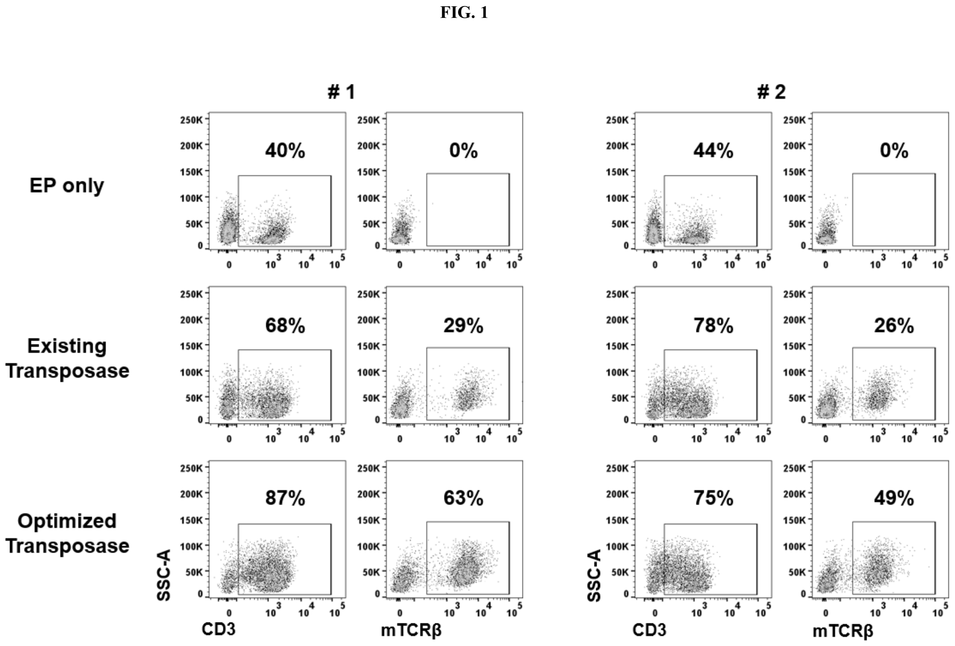

Since the constant region of 1G4 TCR consists of the constant gene sequence of mouse TCR, 1G4 TCR expression was confirmed by expression of mTCRβ using an anti-mTCRβ antibody. As a result of confirming the proportion of cells expressing 1G4 TCR in CD3+ T cells seven days after electroporation, as shown in FIG. 1, the proportion was 0% in the "EP only group (negative control group)" in which only electroporation (EP) was performed without a plasmid, and in the 3M3-5M3-1G4 TCR transposon, the proportion was 27.7% in the "existing transposase group" and 56.0% in the "optimized transposase group," confirming that the proportion of mTCRβ expressing cells was twice as high when the optimized transposase was used.

### 2. Analysis of 1G4 TCR expression ten days after electroporation

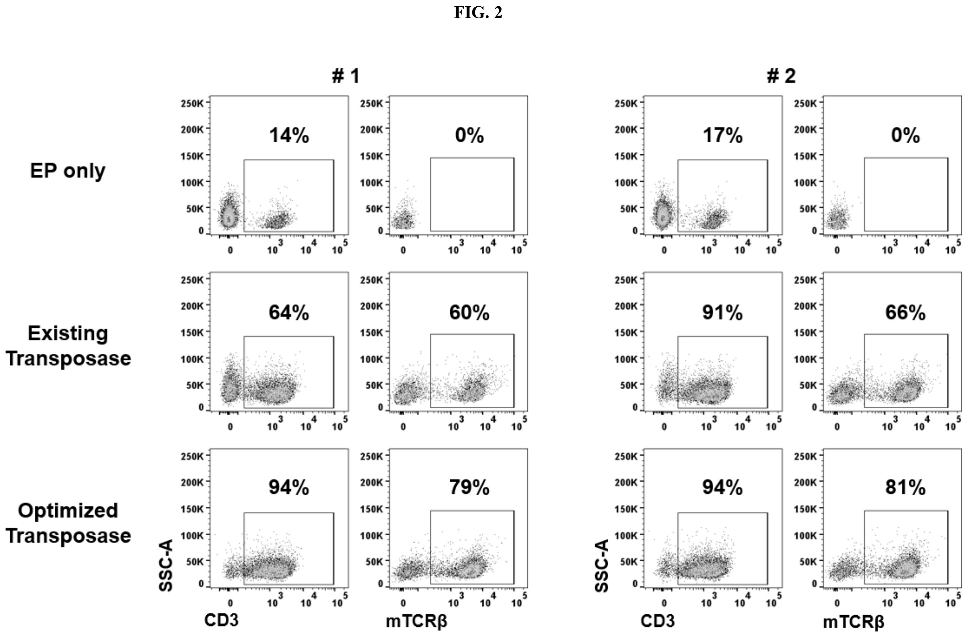

As a result of confirming the proportion of cells expressing 1G4 TCR in CD3+ T cells 10 days after electroporation, as shown in FIG. 2, the proportion was 0% in the "EP only group (negative control group)," and it was confirmed in the transposon group that the proportion increased overall compared to seven days after electroporation. Specifically, in the 3M3-5M3-1G4 TCR transposon, the proportion was 62.7% in the "existing transposase group" and 79.9% in the "optimized transposase group," and thus, similar to seven days after electroporation, the proportion of mTCRβ expressing cells was 1.3 times higher when the optimized transposase was used.

### 3. Analysis of 1G4 TCR expression 14 days after electroporation

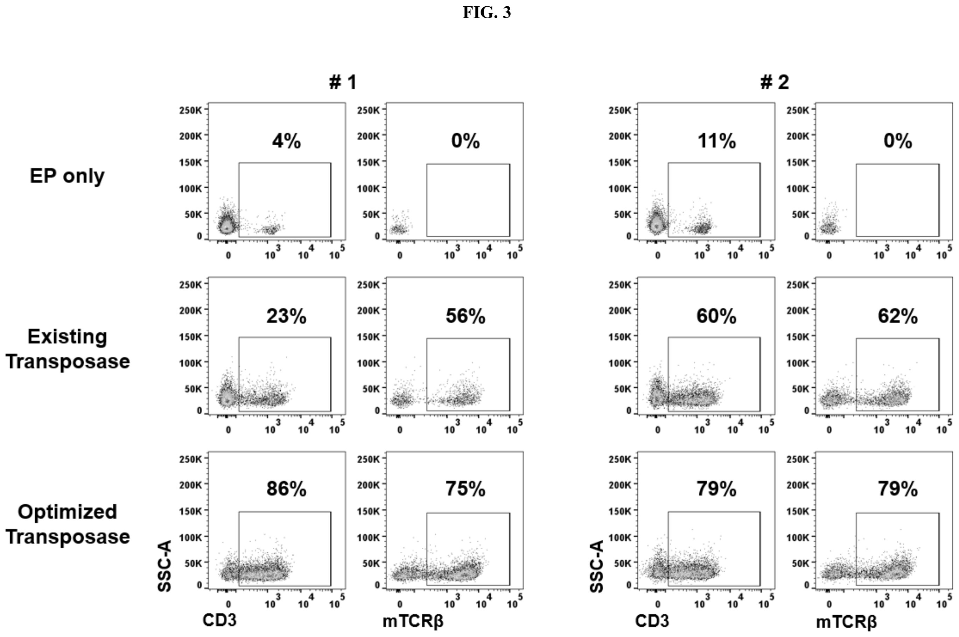

As a result of confirming the proportion of cells expressing 1G4 TCR in CD3+ T cells 14 days after electroporation, as shown in FIG. 3, the proportion was 0% in the "EP only group (negative control group)," and in the transposon group, it was similar overall to 10 days after electroporation. Specifically, in the 3M3-5M3-1G4 TCR transposon, the proportion was 59.0% in the "existing transposase group" and 76.7% in the "optimized transposase group," and thus, similar to seven days and 10 days after electroporation, the proportion of mTCRβ expressing cells was 1.3 times higher when the optimized transposase was used.

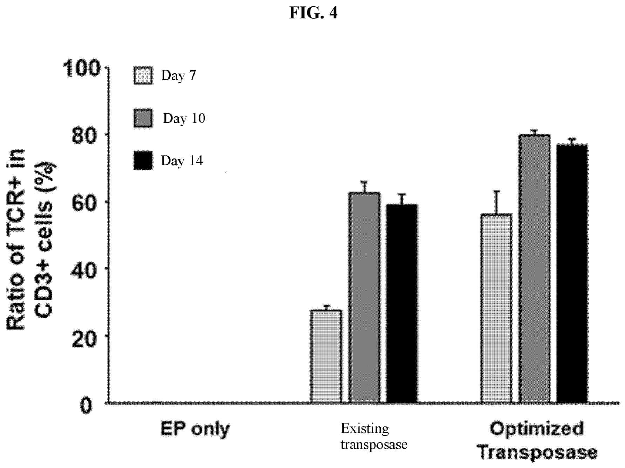

In addition, when comparing the proportion of 1G4 TCR expressing T cells between 7, 10, and 14 days after electroporation, as shown in FIG. 4, it was confirmed that the proportion of 1G4 TCR expressing T cells increased after both 10 days and 14 days compared to 7 days. In the comparison of the transposases, the proportion of 1G4 TCR expressing cells was higher in the "optimized transposase" than in the "existing transposase" at all of 7, 10, and 14 days after electroporation, indicating that the gene delivery efficiency of the optimized transposase was better.

### 4. Analysis of CD4+ cells and CD8+ cells in 1G4 TCR-expressing T cells

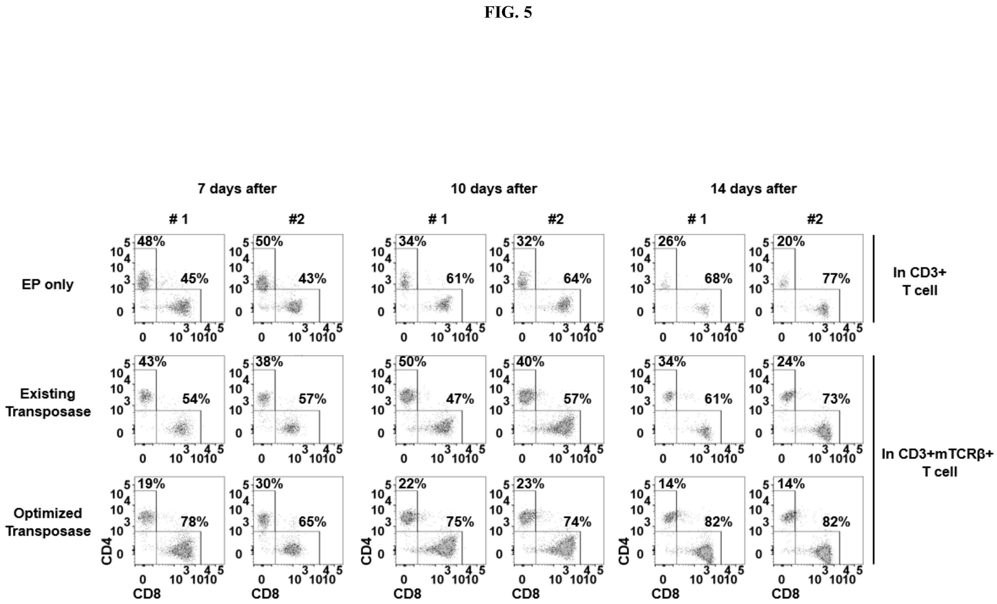

The proportion of helper T cells (CD4+ T cells) and cytotoxic T cells (CD8+ T cells) in 1G4 TCR-expressing T cells was compared, and in the "EP only group," CD4+ cells and CD8+ cells were confirmed in CD3+ T cells because there were no 1G4 TCR-expressing T cells. As a result, as shown in FIG. 5, the proportion of CD4+ cells and CD8+ cells in the "EP only group" was similar after 7 days, whereas after 10 days, CD4+ cells were 33.0% and CD8+ cells were 62.5%, showing that CD8+ cells were more abundant. After 14 days, CD4+ cells were 23% and CD8+ cells were 72.5%, showing that CD8+ cells were more abundant. This may be because the proportion of CD8+ cells in CD3+ T cells increased as CD8+ cells proliferated during the culture period.

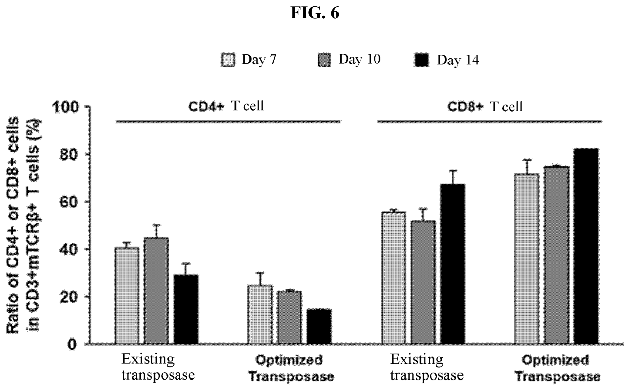

In addition, as a result of confirming CD4+ cells and CD8+ cells in CD3+mTCRβ+ T cells in the 3M3-5M3-1G4 TCR transposon, as shown in FIGS. 5 and 6, in the "existing transposase group," the proportion of CD4+ cells was 40.5% after 7 days, 44.9% after 10 days, and 29.0% after 14 days, and the proportion of CD8+ cells was 55.5% after 7 days, 51.8% after 10 days, and 67.2% after 14 days. It was confirmed that up to 10 days, the difference between CD4+ cells and CD8+ cells was minimal, but after 14 days, CD8+ cells were 2.3 times more. On the other hand, in the "optimized transposase group," the proportion of CD4+ cells was 24.7% after 7 days, 22.1% after 10 days, and 14.4% after 14 days, and the proportion of CD8+ cells was 71.4% after 7 days, 74.8% after 10 days, and 82.2% after 14 days. Therefore, it was confirmed that CD8+ cells gradually increased to be 2.9 times more after 7 days, 3.4 times more after 10 days, and 5.7 times more after 14 days compared to CD4+ cells.

### 5. Analysis of memory type T cells in 1G4 TCR expressing T cells

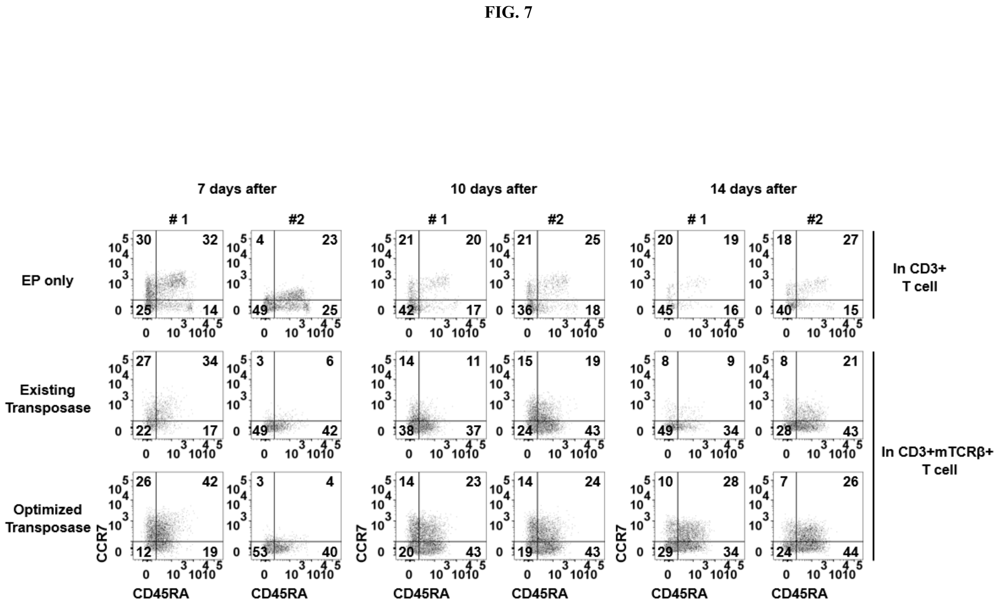

The proportion of memory type T cells was analyzed using CD45RA and CCR7 markers in 1G4 TCR expressing T cells. When memory type T cells are distinguished using CD45RA and CCR7 markers, CD45RA+CCR7- T cells are classified as T_{EFF} cells, CD45RA-CCR7- T cells are classified as T_{EM} cells, CD45RA-CCR7+ T cells are classified as T_{CM} cells, and CD45RA+CCR7+ T cells are classified as T_{SCM} cells. In the "EP only group," memory type T cells were confirmed in CD3+ T cells because there were no 1G4 TCR expressing T cells. As a result, as shown in FIG. 7, it was confirmed that T_{CM} and T_{SCM} decreased and T_{EM} increased as the culture period increased. As shown in FIG. 7 and Table 3, in the "existing transposase group," the proportion of memory type T cells in 1G4 TCR expressing T cells was 29.7% for T_{EFF}, 35.3% for T_{EM}, 15.1% for T_{CM}, and 20.0% for T_{SCM} after 7 days; 39.8% for T_{EFF}, 31.0% for T_{EM}, 14.2% for T_{CM}, and 15.1% for T_{SCM} after 10 days; and 38.3% for T_{EFF}, 38.8% for T_{EM}, 8.2% for T_{CM}, and 14.8% for T_{SCM} after 14 days, confirming that T_{CM} and T_{SCM} gradually decreased, while T_{EM} and T_{EFF} gradually increased. In the "optimized transposase group," the proportion was 29.7% for T_{EFF}, 32.8% for T_{EM}, 14.4% for T_{CM}, and 23.1% for T_{SCM} after 7 days; 42.8% for T_{EFF}, 19.5% for T_{EM}, 14.4% for T_{CM}, and 22.5% for T_{SCM} after 10 days; and 38.7% for T_{EFF}, 26.1% for T_{EM}, 8.4% for T_{CM}, and 27.0% for T_{SCM} after 14 days, confirming that T_{CM} decreased while T_{SCM} increased, and the sum of the T_{CM} and T_{SCM} cell proportions was higher than in the existing transposase.

**[Table 3]**

| Conditions | | T_{EFF} CD45RA+C CR7- | T_{EM} CD45RA-CCR7- | T_{CM} CD45RA-CCR7+ | T_{SCM} CD45RA+C CR7+ |
|---|---|---|---|---|---|
| 7 days | Existing transposase | 29.7% | 35.3% | 15.1% | 20.0% |
| | Optimized transposase | 29.7% | 32.8% | 14.4% | 23.1% |
| 10 days | Existing transposase | 39.8% | 31.0% | 14.2% | 15.1% |
| | Optimized transposase | 42.8% | 19.5% | 14.4% | 23.5% |
| 14 days | Existing transposase | 38.3% | 38.8% | 8.2% | 14.8% |
| | Optimized transposase | 38.7% | 26.1% | 8.4% | 27.0% |

As described above, when producing 1G4 TCR-T cells using the pBat transposon system in PBMCs, it was confirmed whether there was a difference in the delivery and expression efficiency of the 1G4 TCR gene according to the use of the existing transposase or optimized transposase, and the results confirmed that the gene delivery efficiency of the optimized transposase was higher in LK045 PBMCs. In addition, regarding the proportion of CD4+ or CD8+ cells in cells expressing 1G4 TCR, when the existing transposase was used, the proportion of CD8+ T cells was 2.3 times more than that of CD4+ T cells after 14 days, but when the optimized transposase was used, the proportion of CD8+ T cells was 2.9 times higher after 7 days, and the difference gradually increased thereafter. As a result of confirming the memory type using CD45RA and CCR7 markers in 1G4 TCR expressing T cells, it was confirmed that the proportion of cells containing T_{CM} and T_{SCM} was high in the optimized transposase. Therefore, when producing TCR-T cells using the pBat transposon system, TCR-T cells with a high proportion of cytotoxic T (CD8+ T) cells could be produced at a high yield when the optimized transposase was used.

### Example C. Confirmation of gene delivery efficiency of optimized transposase in Jurkat cells

### 1. Method

Jurkat cells (ATCC, Cat No. TIB-152, Lot no. 70017560) were used as target cells to confirm the gene delivery efficiency of the optimized transposase.

The pBat transposon 3M3-5M3-EGFP vector containing EGFP was used as a transposon plasmid vector, and the pBat transposase plasmid and the pBat optimized transposase plasmid were used for the comparison of transposase delivery efficiency.

Green fluorescent protein (GFP) fluorescence expressed in Jurkat cells was observed through FACS analysis on days 1, 7, and 14 after electroporation in the same manner as in the above-described Example B.

### 2. Results

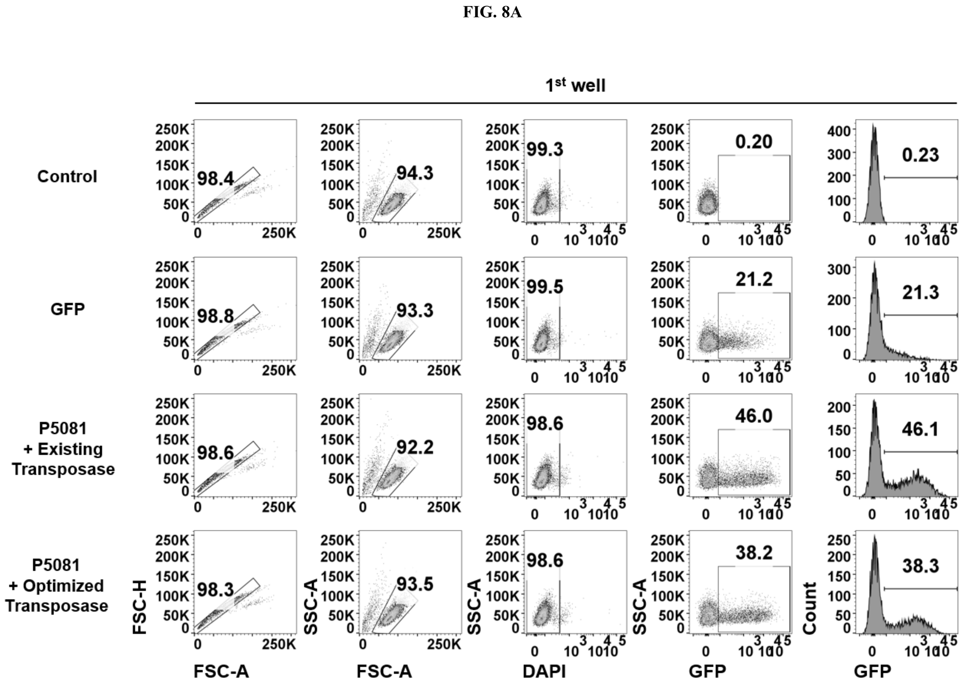

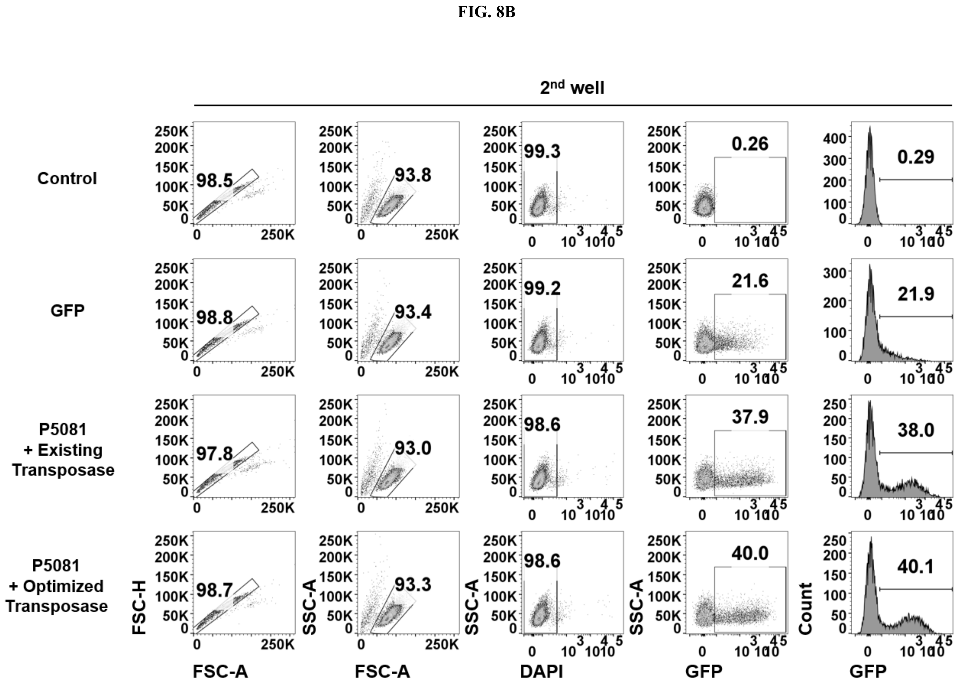

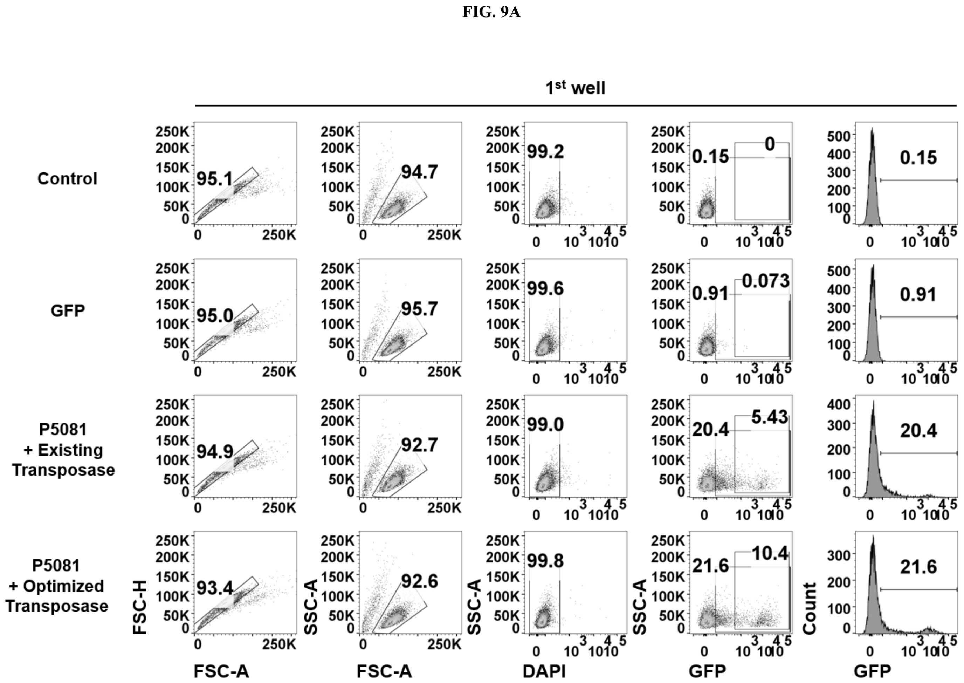

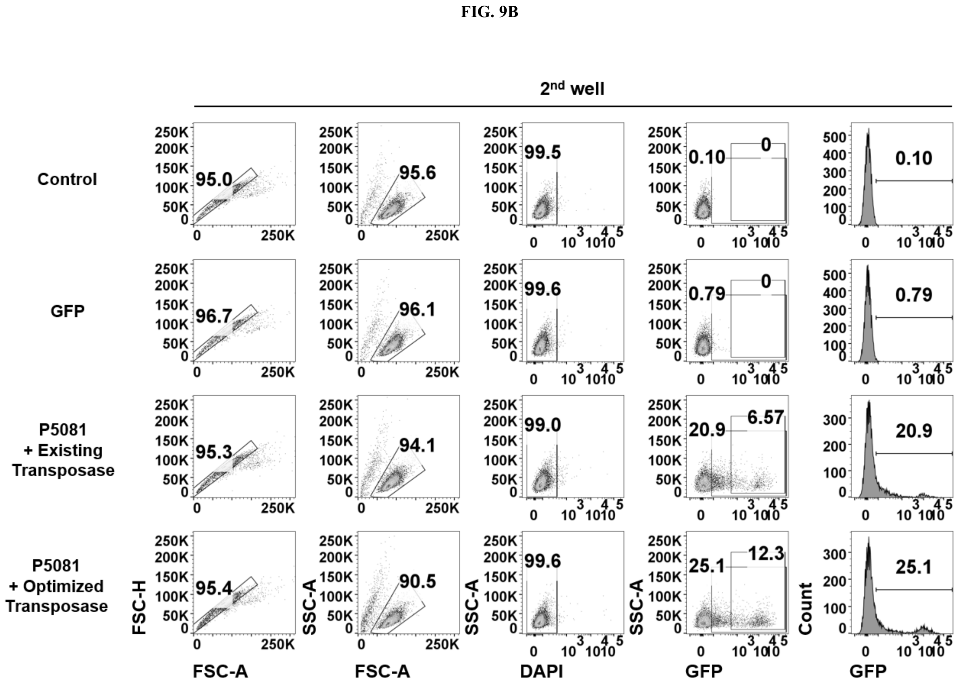

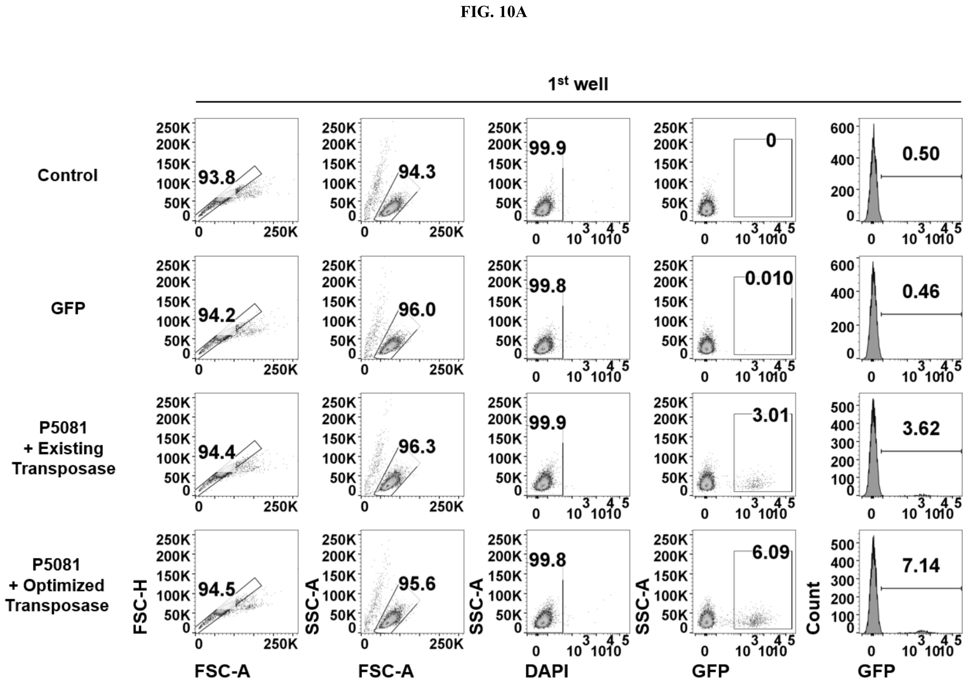

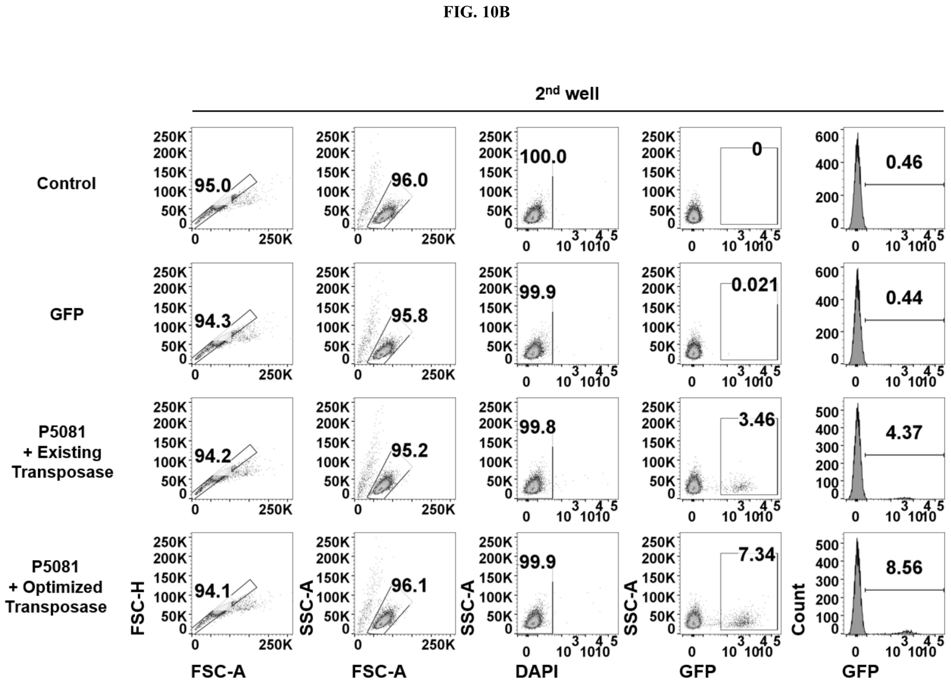

On day 1, as shown in FIGs. 8A and 8B, there was no difference in the proportion of GFP-expressing cells between the two types of transposases. On day 7, as shown in FIGs. 9A and 9B, there was no difference in the proportion of total GFP-expressing cells between the two types of transposases, but the proportion of highly expressed GFP cells was confirmed to be about twice as high in the optimized transposase. On day 14, as shown in FIGs. 10A and 10B, the proportions of total GFP-expressing cells and highly expressed GFP cells were confirmed to be about twice as high in the optimized transposase.

From the above-described results, it was confirmed that the optimized transposase exhibited a GFP expression rate twice as high as the existing transposase.

### Example D. Confirmation of increased gene expression efficiency of mRNA type pBat-optimized transposase

As a result of comparing the gene delivery efficiency of an existing transposase and a codon-optimized optimized transposase, it was confirmed that the codon-optimized optimized transposase exhibited higher gene delivery efficiency.

However, when an excessive amount of DNA type transposase is used for gene delivery, *in vivo* toxicity is induced due to the intracellular mechanism that recognizes foreign DNA, and when the transposase gene delivered into the cell in the form of DNA continues to be present in the cell and transposase expression continues for a long period, there is a possibility that the inserted foreign gene may be re-mobilized. Therefore, an mRNA type transposase with low *in vivo* toxicity and a relatively short half-life was produced, and the gene delivery efficiency was compared with that of the DNA type transposase.

### [Method]

### 1. Cells and DNA

Jurkat, Clone E6-1 (ATCC, Cat No. TIB-152, Lot no. 70017560) and PBMC LK053 isolated from a healthy human were used as target cells to confirm the increase in gene expression efficiency of an mRNA type pBat transposase.

A pBat transposon vector containing the GFP gene and a pBat transposon vector containing the 1G4 TCR gene were used as transposon plasmid vectors, and in order to confirm the gene delivery efficiency according to the delivery form of the transposase gene, a plasmid DNA type optimized transposase (pBat optimized transposase plasmid) and an mRNA type optimized transposase were used.

### 2. Production of template DNA for mRNA synthesis

Optimization of the existing transposase gene sequence (SEQ ID NO: 1) to fit the mRNA form was commissioned to GenScript CO., LTD., and synthesis of a DNA sequence (SEQ ID NO: 3) optimized for the mRNA form in GenSmart Optimization Report (Tool Version Beta 1.0) was commissioned to Bionics CO., LTD. The gene of the DNA sequence optimized for the mRNA type was cloned into the Cloning kit for mRNA Template (Cat. #6143) in the TaKaRa IVTpro^{™} T7 mRNA synthesis kit as described below.

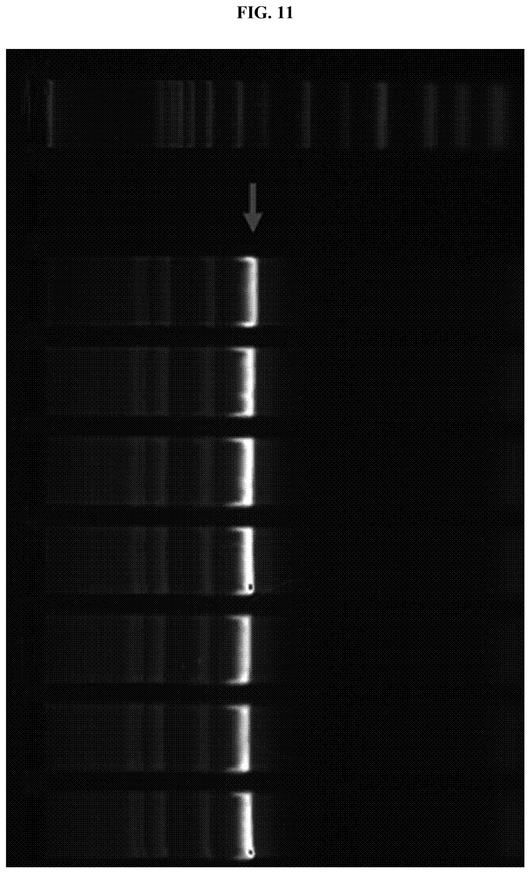

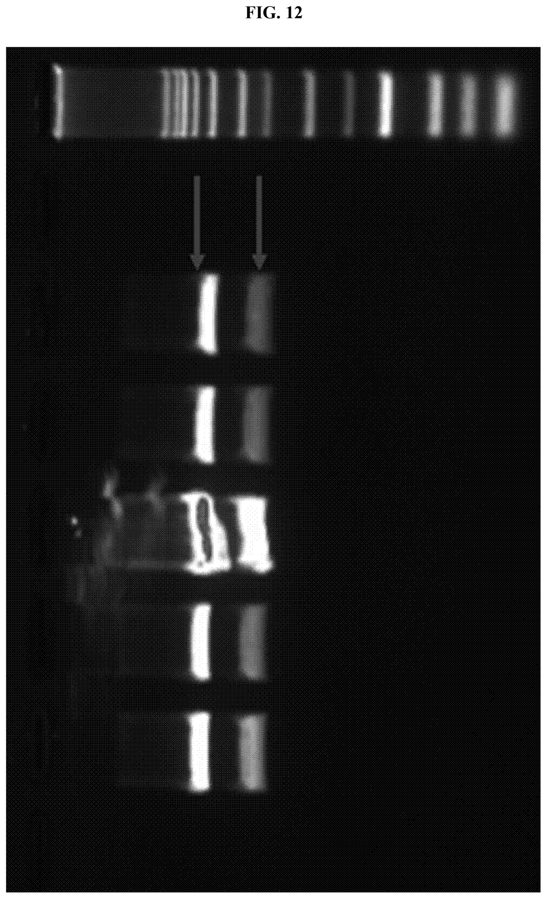

For infusion cloning of the DNA sequence optimized for the mRNA form, PCR for insert amplification was performed using the DNA sequence (SEQ ID NO: 3) optimized for the mRNA form synthesized by Bionics CO., LTD. as a template, and the PCR product was loaded onto an agarose gel. As shown in FIG. 11, the bands of the desired sizes were confirmed, and then gel extraction was performed. Infusion cloning was carried out between the Cloning kit for mRNA Template (Vector) included in the Takara IVTpro^{™} T7 mRNA synthesis system and the DNA sequence (Insert, SEQ ID NO: 3) optimized for the mRNA form, and as shown in FIG. 12, the structure was confirmed through enzyme mapping to produce the pBat optimized transposase mRNA template plasmid necessary for producing the optimized transposase in an mRNA form using the *in vitro* transcription method.

### 3. In vitro transcription (IVT) process for transforming pBat optimized transposase into mRNA type

In order to synthesize mRNA using the pBat optimized transposase mRNA template plasmid produced in '2.' above, the template plasmid was linearized with the Hind III restriction enzyme, and the IVT reaction was performed as described below.
1) The restriction enzyme Hind III was added to the pBat optimized transposase mRNA template plasmid and allowed to react at 37 °C for three hours for linearization.
2) The linearized mRNA template plasmid was purified using the EtOH precipitation method, and the process is described below:
2-1) 20 µL of 3 M sodium acetate was added to the reaction product in 1) and mixed well, and the resulting mixture was cooled at -20 °C for more than 15 minutes.
2-2) Centrifugation was performed at 12,000 rpm and 4 °C for 15 minutes.
2-3) The supernatant was carefully discarded, 1 mL of 70% EtOH was added, and centrifugation was performed at 12,000 rpm and 4 °C for 15 minutes.
2-4) The supernatant was carefully discarded, and the DNA pellet was dried thoroughly.
2-5) 200 µL of nuclease-free water was added to make a final concentration 0.5 to 1.0 µg/µL, and the resulting mixture was stored at -20 °C until use.

3) The reagents included in the Takara IVTpro^{™} T7 mRNA synthesis system were dissolved at room temperature, mixed well, and spun down. 10X Enzyme Mix was placed on ice.
4) The reagents were added to an E-tube in the order shown in Table 4 below, and the reaction was performed with a total amount of 120 µL.

**[Table 4]**

| Reagent | vol/rxn | Scale up |
|---|---|---|
| Nuclease-free water | 4.4 µL | 26.4 µL |
| 10X transcription buffer | 2.0 µL | 12 µL |
| 10X ATP | 2.0 µL | 12 µL |
| 10X CTP | 2.0 µL | 12 µL |
| 10X GTP | 2.0 µL | 12 µL |
| 10X UTP | 2.0 µL | 12 µL |
| CleanCap Reagent AG | 1.6 µL | 9.6 µL |
| mRNA template plasmid 1 ug | 2.0 µL | 12 µL |
| 10X Enzyme Mix | 2.0 µL | 12 µL |
| Total | 20 µL | 120 µL |

5) After mixing well, a reaction was performed at 37 °C for two hours, and 24 µL of DNase I was treated in the E-tube containing the reaction product of 4) and allowed to react at 37 °C for 15 minutes.
6) The mRNA type optimized transposase was purified through the LiCl precipitation process as described below.
6-1) 180 µL of nuclease-free water was added to 5), and 180 µL of LiCl was added.
6-2) After mixing well, the resulting mixture was allowed to react at -20 °C for at least 30 minutes, and then centrifugation was performed at 12,000 rpm and 4 °C for 15 minutes.
6-3) The supernatant was carefully discarded, 1 mL of 70% EtOH was added, and centrifugation was performed at 12,000 rpm and 4 °C for 15 minutes.
6-4) The supernatant was carefully discarded, the DNA pellet was well dried, and the DNA was dissolved in 200 µL of nuclease-free water.
6-5) 10 µL of each sample was aliquoted and stored at -80 °C until use.

### 4. PBMC activation

Using electroporator equipment, Neon and Maxyte, the gene delivery efficiency of two different genes (GFP/1G4 TCR) in PBMCs was compared according to the transposase type (see Table 5). When the GFP gene was delivered to PBMCs using the transposon system with the Neon equipment, the PBMCs were activated for three days with Miltenyi's TransAct^{™}, and feeder cells were not added during cell culture. On the other hand, when the 1G4 TCR gene was delivered to PBMCs using the transposon system with the MaxCyte equipment, the PBMCs were not activated, but feeder cells were added during cell culture.

**[Table 5]**

| Electroporator equipment | Expressed gene | PBMC activation | Feeder cells |
|---|---|---|---|
| Neon | GFP | Performed | Not added |
| MaxCyte | 1G4 TCR | Not performed | Added |

1) Roswell Park Memorial Institute (RPMI) medium (RPMI + 10% FBS + 1X P/S) was prepared by pre-warming in a 37 °C water bath.
2) PBMC LK053 was taken out from the nitrogen tank and quickly thawed in a 37 °C water bath.
3) 30 mL of culture medium was prepared in a 50 mL tube, and the thawed PBMCs were added.
4) Centrifugation was performed at 1,500 rpm for five minutes, the supernatant was removed, cells were suspended in 40 mL of RMPI medium, and cell counting was performed.
5) 1.2 x 10⁸ PBMCs were placed in a T175 flask, and RPMI medium was added to make 1.2 x 10⁸/120 mL.
6) IL-2 was added to a final concentration of 20 IU/mL, and 100 µL of Transact was added per 2.0 x 10⁷ cells (IL-2 was diluted 1/10 with the medium at a concentration of 1.0 x 10⁶ IU/mL to make 1.0 x 10⁵ IU/mL.).
7) Culture was performed in a 37 °C, 5% CO₂ incubator for two days.

### 5. PBMC electroporation

### 5-1. Neon equipment conditions (PBMC activated; no feeder cells added)

1) 3 mL of E2 buffer stored at 4 °C was dispensed into each of three Neon tubes.
2) 1.4 mL of medium (AlyS + 3% HS + IL-2 200 IU/mL) was plated into each well of a 12-well plate. At this time, four wells were prepared for each group.
3) PBMC cells that had been completely activated were harvested.
4) Centrifugation was performed at 1,500 rpm for five minutes at room temperature.
5) After removing the supernatant, the cell pellet was suspended in 1.0 mL of Opti-MEM buffer.
6) Centrifugation was performed at 1,500 rpm for five minutes at room temperature.
7) After removing the supernatant, the cell pellet was suspended in Opti-MEM buffer to 1.0 x 10⁶/100 µL.
8) For each condition, transposon and transposase plasmids were each added in an amount of 3 µg per 1.0 x 10⁶ cells as shown in Table 6 below (no vector was added to the control group). Thereafter, the mixture of cells and plasmid was mixed well by pipetting.

**[Table 6]**

| **No.** | **GROUP** | **Cell No.** | **OPTIMIZED TRANSPOSASE TYPE** |
|---|---|---|---|
| 1 | Electroporation only | 1.0 x 10⁶cells | - |
| 2 | pBat-GFP plasmid | 1.0 x 10⁶cells | - |
| 3 | pBat-GFP plasmid | 1.0 x 10⁶cells | Plasmid DNA type |
| 4 | pBat-GFP plasmid | 1.0 x 10⁶cells | mRNA type |

9) The Neon tubes containing E2 buffer from 1) were mounted on the Neon device.
10) Using the Neon pipette and Neon tip, 100 UI of the cell suspension from 8) was slowly sucked up and inserted into the Neon device.
11) After electroporation under the conditions of 1,700 V, 20 ms, and 1 pulse, the completed PBMCs were plated into each well of a 12-well plate containing a medium and cultured in an incubator at 37 °C, 5% CO₂.
12) One day after transfection, cells were harvested from two wells (observation plate after 1 day) under each condition, and FACS analysis was performed, and 2 mL of culture medium was added to each of the remaining second wells (observation plate after 7 days), and the resulting mixture was transferred to a T75 flask and cultured in an incubator at 37 °C, 5% CO₂.
13) Five days after transfection, 2 mL of culture medium was added, and culture was performed in a 37 °C, 5% CO₂ incubator.
14) Seven days after transfection, PBMCs were suspended by pipetting the culture medium of each flask for FACS analysis, and 1 mL of cells were collected out of the total 4 mL. 5 mL of culture medium was added to the remaining culture medium, and then culture was performed.
15) Eight, eleven, and thirteen days after transfection, new culture medium were added in an amount of 5 mL, 10 mL, and 20 mL, respectively, and culture was performed in a 37 °C, 5% CO₂ incubator.
16) Fourteen days after transfection, PBMCs were suspended by pipetting the culture medium of each flask for FACS analysis, and cells were collected in an amount of 200 UI.

### 5-2. MaxCyte equipment conditions (PBMC not activated; feeder cells added)

1) PBMC LK053 was taken out from the nitrogen tank and quickly thawed in a 37 °C water bath, then 30 mL of culture medium was prepared in a 50 mL tube and PBMCs were added.
2) Centrifugation was performed at 1,500 rpm for five minutes, and the supernatant was removed.
3) The cell pellet was suspended in 20 mL of medium (ALYS505N-O + 3% HS), and cell counting was performed.
4) Centrifugation was performed at 1,500 rpm for five minutes at room temperature.
5) The supernatant was removed, and the cell pellet was suspended in 20 mL of Dulbecco's phosphate buffered saline (DPBS).
6) Centrifugation was performed at 1,500 rpm for five minutes at room temperature, the supernatant was completely removed, and the cell pellet was suspended in 5 mL of Opti-MEM medium.
7) Centrifugation was performed at 1,500 rpm for five minutes at room temperature, and the supernatant was completely removed.
8) The cell pellet was suspended in 50 µL of warm Opti-MEM medium to make 5.0 x 10⁶/50 µL.
9) The transposon vector and transposase vector were added each in an amount of 5 µg per well to the tubes of 8) as shown in Table 7 below (no vector was added to the control group).

**[Table 7]**

| .No. | Group | Cell No. | Transposon | Optimized Transposase type |
|---|---|---|---|---|
| 1 | No Electroporation | 5.0 x 10⁶ cells | - | - |
| 2 | pBat-1G4 TCR | 5.0 x 10⁶ cells | pBat 3M3-5M3 | Plasmid DNA type |
| 3 | pBat-1G4 TCR | 5.0 x 10⁶ cells | pBat 3M3-5M3 | mRNA type |

10) The cell suspension from 9) of 5.0 x 10⁶ cells/50 µL was carefully added to each OC100X2 assembly to avoid creating bubbles.
11) The resting T cell 14-3 protocol was selected in MaxCyte STx for electroporation.
12) The OC100x2 assembly of 10) was inserted into the chamber in the STx, and the protocol for electroporation was performed.
13) After electroporation, the cell suspension from the OC100x2 assembly was transferred to a T25 flask (5.0 x 10⁶ cells/50 µL/well).
14) The OC100X2 wells with 50 µL of Opti-MEM medium were washed, and the cell suspension of 13) was added to each well of the plate.
15) A recovery time of 20 minutes in a 37 °C, 5% CO₂ incubator was provided.
16) The A375 cell stock irradiated with 100 Gy was rapidly thawed in a 37 °C water bath and slowly added to 10 mL of medium, and centrifuged at 1,500 rpm for five minutes.
17) The supernatant was removed, cells were suspended in 10 mL of medium, and cell counting was performed.
18) Centrifugation was performed at room temperature for five minutes at 1,500 rpm, and the supernatant was completely removed.
19) Cells were resuspended in a complete medium (ALYS505N-O + 3% HS + 200 IU/mL IL-2) to 2.0 x 10⁶ cells/3 mL.
20) After the process of 15) was completed, 3 mL of the cell suspension of 19) was added to each flask, and then cultured in a 37 °C, 5% CO₂ incubator for two days.
21) Two days after electroporation, 2 mL of medium and IL-2 were added to each flask to a final concentration of 200 IU/mL, and culture was performed in a 37 °C, 5% CO₂ incubator.
22) Five days after electroporation, 10 mL of medium and IL-2 were added to a final concentration of 200 IU/mL, and culture was performed in a 37 °C, 5% CO₂ incubator.
23) Seven days after electroporation, the cultured cells were suspended, 500 µL of the cell suspension was transferred to a new tube for FACS analysis, and the remaining cell suspension was adjusted to a total of 15 mL, new medium (ALYS505N-O + 3% HS) and IL-2 were added to a final concentration of 200 IU/mL, and culture was performed in a 37 °C, 5% CO₂ incubator.
24) Nine days after electroporation, 5 mL of medium (ALYS505N-O + 3% HS) was added to make a total of 20 mL, IL-2 was added to a final concentration of 200 IU/mL, and culture was performed in a 37 °C, 5% CO₂ incubator.
25) Twelve days after electroporation, 5 mL of medium (ALYS505N-O + 3% HS) was added to make a total of 25 mL, IL-2 was added to a final concentration of 200 IU/mL, and culture was performed in a 37 °C, 5% CO₂ incubator.
26) Fourteen days after electroporation, the cultured cells were suspended, and 500 µL of the cell suspension was transferred to a new tube for FACS analysis, and 2 x 10⁷ cells per group were harvested for *in vitro* killing analysis and cultured in 20 mL of Resting medium (ALYS505N-O + 3% HS) for 24 hours. The remaining suspension was prepared into a stock by removing the supernatant, adding CS10 medium, storing the resulting mixture at -80 °C for one day, and then moving it to a nitrogen tank.

### 6. FACS analysis

FACS analysis was performed 7 and 14 days after electroporation.
1) After suspending cells in each group's flask, 500 µL was taken and transferred to a FACS tube.
2) Centrifugation was performed at 4 °C and 1,500 rpm for five minutes, the supernatant was removed, and cells were suspended with 1 mL of washing buffer (PBS + 2% FBS).
3) Step 2) was repeated once more for washing.
4) Centrifugation was performed at 4 °C and 1,500 rpm for five minutes, and the supernatant was removed.
5) 5 µL of human TruStain FcX + 95 µL of FACS buffer was added to each tube and incubated for five minutes at room temperature.
6) Each antibody (anti-CD3, anti-CD4, anti-CD8, anti-CD45RO, anti-CD62L) was added in an amount of 2 µL per tube, an anti-mTCRβ antibody was added in an amount of 0.5 µL per tube, and the resulting mixtures were allowed to react for 30 minutes at room temperature in a dark place.
7) 1 mL of FACS buffer (PBS + 2% FBS) was added, and centrifugation was performed at 4°C and 1,500 rpm for five minutes.
8) The supernatant was removed, and cells were suspended with 1 mL of FACS buffer (PBS + 2% FBS).
9) Centrifugation was performed at 4 °C and 1,500 rpm for five minutes, and the supernatant was removed.
10) Cells were suspended with 200 µL of FACS buffer (PBS + 2% FBS) containing 1x DAPI, and FACS analysis was performed.

### 7. Peptide pulsing in T2-Luc cells

1) T2-Luc cells cultured in Iscove's modified Dulbecco's medium (IMDM) (IMDM + 10% FBS + 1X P/S) were collected in 15 mL conical tubes.
2) Centrifugation was performed at 1,500 rpm for five minutes at room temperature.
3) The supernatant was removed, the cell pellet was suspended in 5 mL of IMDM, and cell counting was performed.
4) IMDM was added to make 1.0 x 10⁶/mL, and the resulting mixture was placed in a polypropylene (PP) tube according to the conditions shown in Table 8 below.

**[Table 8]**

| Tube | Condition | T2 cell count | β2m treatment concentration | Peptide treatment concentration |
|---|---|---|---|---|
| 1 | No pulsing | 1.0 x 10⁶ | - | - |
| 2 | NY-ESO-1 pulsing | 1.0 x 10⁶ | 3 µg/mL | 100 µg/mL |

5) 100 mg/mL NY-ESO-1 peptide and 0.5 mg/mL β2m were added to the T2-Luc cell pulsing group to achieve the same treatment concentration as the peptide 'pulsing' conditions in Table 8 above. The same volume of dimethyl sulfoxide (DMSO) as the added peptide volume was added to the 'no pulsing' group.
6) The reaction was performed for two hours while performing tapping every 30 minutes at room temperature.
7) Centrifugation was performed at 1,500 rpm for five minutes at room temperature, and the supernatant was removed.
8) The cell pellet was suspended in 10 mL of PBS, and centrifugation was performed at 1,500 rpm for five minutes at room temperature.
9) The supernatant was removed, the cell pellet was suspended in 1 mL of ALyS medium (ALYS505N-O + 3% HS), and then cell counting was performed.
10) Target cells were prepared by adding ALyS medium (ALYS505N-O + 3% HS) to T2-Luc cells at a concentration of 2.0 x 10⁴/50 µL.

### 8. Luciferase-based cytotoxicity assay after co-culture of 1G4 TCR-T cells and pulsed T2-Luc cells

1) 1G4 TCR-T cells rested for 24 hours under the '5-2. MaxCyte equipment conditions' were collected in 50 mL conical tubes.
2) Centrifugation was performed at 1,500 rpm for five minutes at room temperature.
3) The supernatant was removed, the cell pellet was suspended in 10 mL of ALyS medium (ALYS505N-O + 3% HS), and cell counting was performed.
4) ALyS medium (ALYS505N-O + 3% HS) was added to make the 1G4 TCR-T cell concentration of 6.0 x 10⁵/50 µL in order to prepare effector cells.
5) To achieve the ratios of effector cells (E) and target cells (T) of 30:1, 10:1, 3:1, and 1:1 in two 96-well white plates, first, 50 µL of medium was plated to lanes B to D and F to H according to the conditions described in the table below, and 9.0 x 10⁵/75 µL of 1G4 TCR-T cells, which are effector cells (E), were added to lanes A and E. Serial dilutions were performed in 25 µL from lanes A to D and E to H using a multi-channel pipette.
6) 2.0 x 10⁴ (50 µL) of the T2-Luc cells prepared in '7.' above were added according to the conditions in the table below and mixed well.
7) Only T2 cells were added according to the conditions in Table 9 and Table 10, and 20 µL of 10% Triton-X was added to some 'only T2 (Lysis)' wells and mixed well.
8) The total volume of all wells was adjusted to 100 µL with ALyS medium.

**[Table 9]**

| Plate 1 | | No EP | | Plasmid DNA type | | mRNA type | | No EP | | Plasmid DNA type | | mRNA type | |
|---|---|---|---|---|---|---|---|---|---|---|---|---|---|
| | | 1 | 2 | 3 | 4 | 5 | 6 | 7 | 8 | 9 | 10 | 11 | 12 |
| 30:1 | A | T cell + T2 cell (NYSEO-1 pulsing) | | | | | | Only T cell | | | | | |
| 10:1 | B | | | | | | | | | | | | |
| 3:1 | C | | | | | | | | | | | | |
| 1:1 | D | | | | | | | | | | | | |
| 30:1 | E | T cell + T2 cell | | | | | | Only T2 | | | | | |
| 10:1 | F | | | | | | | Only T2 (Lysis) | | | | | |
| 3:1 | G | | | | | | | | | | | | |
| 1:1 | H | | | | | | | | | | | | |

**[Table 10]**

| Plate 2 | | No EP-2 | | Plasmid DNA type-2 | | mRNA type-2 | | No EP-2 | | Plasmid DNA type-2 | | mRNA type-2 | |
|---|---|---|---|---|---|---|---|---|---|---|---|---|---|
| | | 1 | 2 | 3 | 4 | 5 | 6 | 7 | 8 | 9 | 10 | 11 | 12 |
| 30:1 | A | T cell + T2 cell (NYSEO-1 pulsing) | | | | | | Only T cell | | | | | |
| 10:1 | B | | | | | | | | | | | | |
| 3:1 | C | | | | | | | | | | | | |
| 1:1 | D | | | | | | | | | | | | |
| 30:1 | E | T cell + T2 Cell | | | | | | Only T2 | | | | | |
| 10:1 | F | | | | | | | Only T2 (Lysis) | | | | | |
| 3:1 | G | | | | | | | | | | | | |
| 1:1 | H | | | | | | | | | | | | |

9) Co-culture was performed for four hours in a 37 °C, 5% CO₂ incubator.
10) Luciferase assay reagent was added to each well in an amount of 100 µL, the well was covered with aluminum foil, and the mixture was allowed to react at room temperature for two minutes.
11) The 96-well plate was placed in a spectrophotometer, and luminescence was measured.

### [Results]

### 1. mRNA transposase cloning

For infusion cloning, PCR was performed for insert amplification using the DNA sequence (SEQ ID NO: 3) optimized for the mRNA form synthesized by Bionics CO., LTD. as a template. After loading on an agarose gel, the bands of the desired sizes were confirmed as shown in FIG. 11, and then gel extraction was performed.

Thereafter, infusion cloning was performed between the cloning kit for mRNA template (vector) included in the Takara IVTpro^{™}T7 mRNA synthesis system and the DNA sequence (Insert, SEQ ID NO. 3) optimized for the mRNA form. After extracting the cloned plasmid DNA, the final candidate clones were selected through enzyme mapping by treating with Cla I and Nde I restriction enzymes, and the structure is as shown in FIG. 12.

### 2. Confirmation of the total number of cultured cells

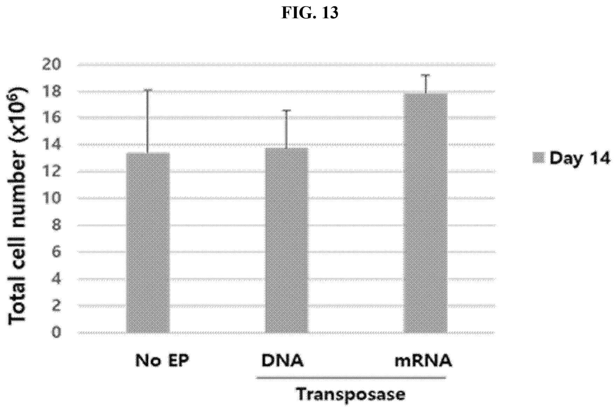

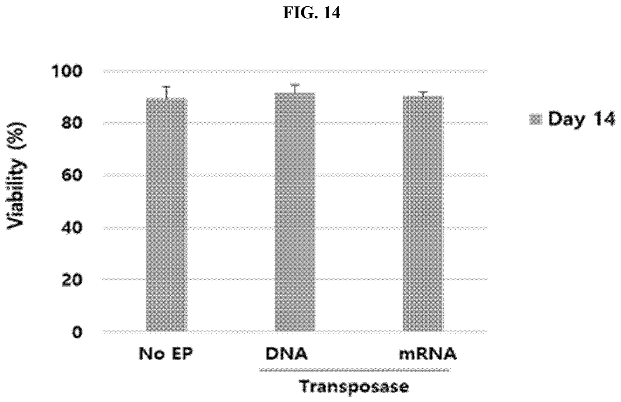

After delivering the 1G4 TCR gene to PBMCs by MaxCyte electroporation, the total number of cultured cells and cell viability were confirmed by culturing them together with feeder cells for 14 days. Regarding the total number of cells, as shown in FIG. 13, it was confirmed that the proliferation rate slightly differed according to the form of delivery of the transposase gene. The initial cell number at the time of electroporation was 5.0 x 10⁶ cells, but after 14 days of culture, the group that did not undergo electroporation proliferated to 1.3 x 10⁷ cells, the group that used the DNA type optimized transposase exhibited the highest proliferation rate of 1.4 x 10⁷ cells, and the group that used the mRNA type optimized transposase exhibited the highest proliferation rate of 1.8 x 10⁷ cells. In addition, as shown in FIG. 14, it was confirmed that the cell viability was 89.4% in the group that did not undergo electroporation, 91.7% in the group that used the DNA type optimized transposase, and 90.3% in the group that used the mRNA type optimized transposase.

### 3. FACS analysis according to the delivery type of optimized transposase gene after Neon electroporation (CD3/CD28 bead activation; No feeder cells added)

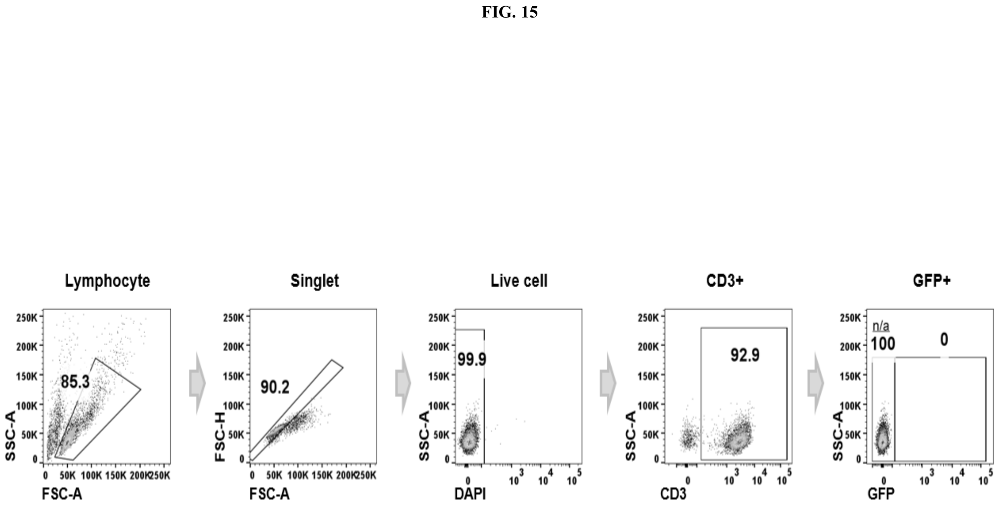

To confirm the expression of the GFP gene according to the mRNA and DNA forms of the transposase gene in PBMC cells, gene expression was confirmed through FACS analysis on days 7 and 14 of culture after transformation by Neon electroporation. In this experiment, PBMCs were activated with CD3/CD28 beads before electroporation, and culture was performed without feeder cells. As shown in FIG. 15, the efficiency of GFP gene delivery into CD3+ T cells and expression was confirmed by gating in the order of lymphocytes, singlets, live cells, CD3+ T cells, and GFP+ T cells.

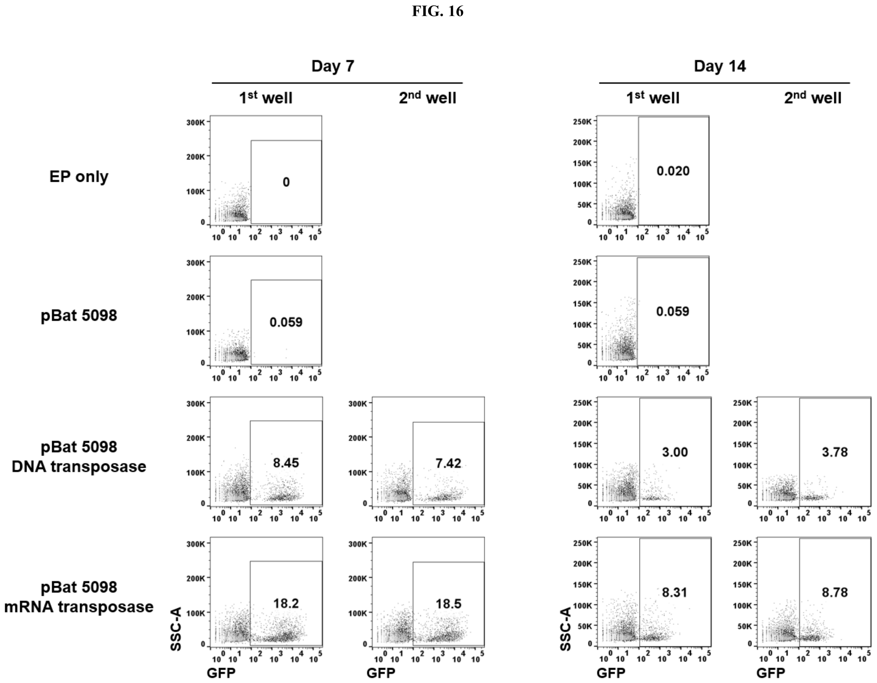

To confirm the expression of the GFP gene according to the mRNA type optimized transposase and the DNA type optimized transposase in PBMC cells, the proportion of cells expressing GFP among CD3+ T cells was confirmed on day 7 of culture. As a result, as shown in FIG. 16, after 7 days of culture, it was confirmed that GFP was not expressed in the negative control group "EP only group" where only electroporation was performed, and the proportion was 0.06%, 0.02% in the "pBat 5098 (pBat 3M3-5M3) group" where only a transposon was used, indicating that almost no GFP expression occurred. On the other hand, the GFP expression rate was 8.5% and 7.4% in the DNA type optimized transposase group and 18.2% and 18.5% in the mRNA type optimized transposase group, confirming that the GFP expression rate in the mRNA type optimized transposase group was higher by about 10% or more. In addition, after 14 days of culture, the GFP expression rate was 3.0% and 3.8% in the DNA type optimized transposase group, while the GFP expression rate was 8.3% and 8.8% in the mRNA type optimized transposase group, confirming that, similar to the results obtained on day 7, the expression rate in the mRNA type optimized transposase group was higher by about 5% or more.

### 4. FACS analysis according to delivery type of optimized transposase gene after MaxCyte electroporation (without activation; feeder cells added)

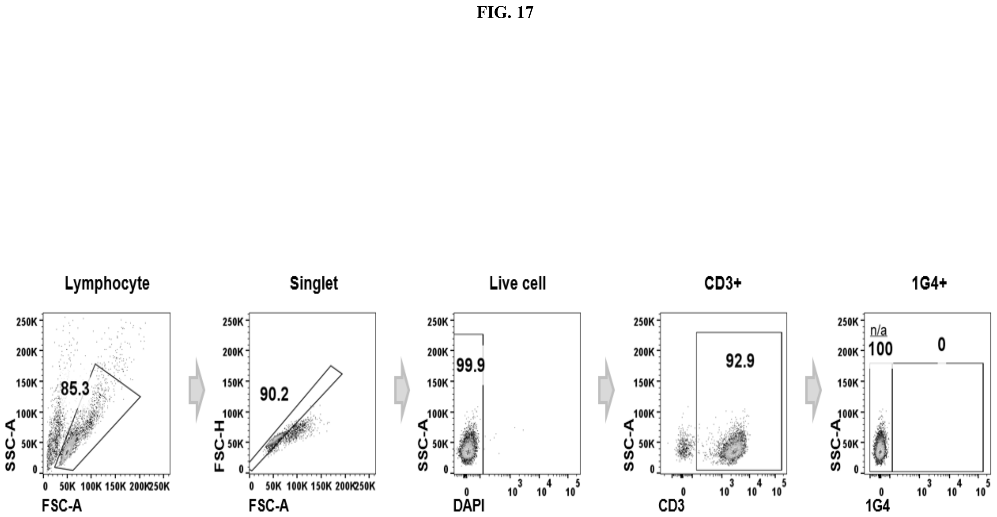

To confirm the expression of the 1G4 TCR gene according to the mRNA form and DNA form of the transposase gene in PBMC cells, gene expression was confirmed through FACS analysis on days 7 and 14 of culture after transformation by MaxCyte electroporation. In this experiment, PBMCs were not activated with CD3/CD28 beads before electroporation, but culture was performed by adding feeder cells. Regarding the analytical method, as shown in FIG. 17, the efficiency of 1G4 TCR gene delivery into CD3+ T cells and expression was confirmed by gating in the order of lymphocytes, singlets, live cells, CD3+ T cells, and 1G4+ T cells.

### 4-1. Comparison of 1G4 TCR gene expression according to delivery type of optimized transposase gene

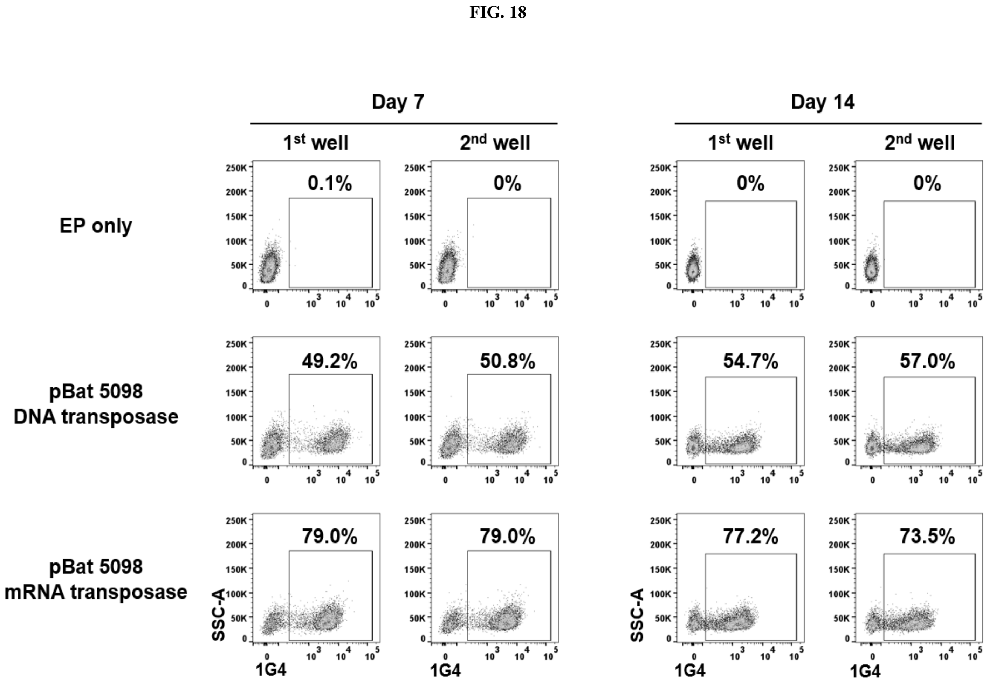

To confirm the expression of the 1G4 TCR gene according to the mRNA type optimized transposase and the DNA type of optimized transposase in PBMC cells, the proportion of cells expressing 1G4 TCR among CD3+ T cells was confirmed on day 7 of culture. As a result, as shown in FIG. 18, after 7 days of culture, it was confirmed that 1G4 TCR was not expressed in the negative control group "EP only group" where only electroporation was performed. On the other hand, the 1G4 TCR expression rate was 49.2% and 50.8% in the DNA type optimized transposase group and 79.0% and 79.0% in the mRNA type optimized transposase group, confirming that the 1G4 TCR expression rate in the mRNA type optimized transposase group was higher by about 29% or more. In addition, after 14 days of culture, the 1G4 TCR expression rate was 54.7% and 57.0% in the DNA type optimized transposase group, while the 1G4 TCR expression rate was 77.2% and 73.5% in the mRNA type optimized transposase group, confirming that, similar to the results obtained on day 7, the expression rate in the mRNA type optimized transposase group was higher by about 20% or more.

### 4-2. Confirmation of the proportion of memory type T cell in 1G4 TCR-T cells according to the delivery type of optimized transposase gene

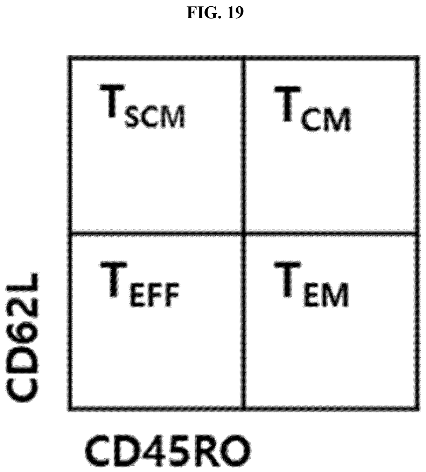

The proportion of memory type T cells among T cells expressing 1G4 TCR (CD3+1G4+ T cells) was analyzed using CD45RO and CD62L markers according to the delivery type of the optimized transposase gene. When memory type T cells are distinguished using CD45RO and CD62L markers, CD45RO-CD62L- T cells are classified as T_{EFF} (effector T cells), CD45RO-CD62L+ T cells are classified as T_{SCM} (stem cell like memory T cells), CD45RO+CD62L- T cells are classified as T_{EM} (effect memory T cells), and CD45RO+CD62L+ T cells are classified as T_{CM} (central memory T cells). The position of each memory type T cell in the quadrant plot is as shown in FIG 19.

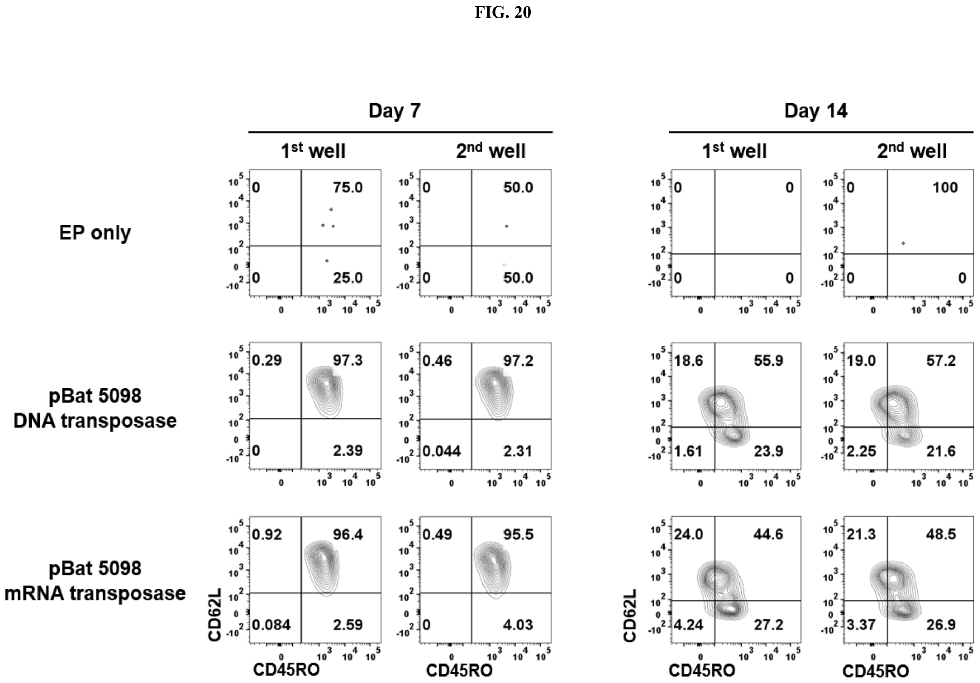

In order to compare the proportion of memory T cells according to the delivery type of the transposase gene, the proportion of memory type T cells was confirmed on days 7 and 14 after transformation with the 1G4 TCR gene using a transposon vector. As a result, as shown in FIG. 20 and Table 11, it was confirmed that on day 7 after transformation, the proportion of T_{SCM} and T_{CM} was approximately 97.0% in the case of the DNA type optimized transposase, and it was approximately 96.0% in the case of the mRNA type optimized transposase, which was similar to the DNA type. In addition, on day 14, it was confirmed that the proportion of T_{SCM} and T_{CM} was approximately 75.0% in the case of the DNA type optimized transposase, and it was approximately 69.0% in the case of the mRNA type optimized transposase, indicating that the proportion of T_{SCM} and T_{CM} memory type T cells was high in both delivery types.

**[Table 11]**

| | Well | Day 7 | | | | Day 14 | | | |
|---|---|---|---|---|---|---|---|---|---|
| | | T_{SCM} | T_{CM} | T_{EM} | T_{EFF} | T_{SCM} | T_{CM} | T_{EM} | T_{EFF} |
| DNA Transposase | 1 | 0.29 | 97.3 | 2.39 | 0 | 18.6 | 55.9 | 23.9 | 1.61 |
| | 2 | 0.46 | 97.2 | 2.31 | 0.044 | 19.0 | 57.2 | 21.6 | 2.25 |
| mRNA | 1 | 0.92 | 96.4 | 2.59 | 0.084 | 24.0 | 44.6 | 27.2 | 4.24 |
| Transposase | 2 | 0.49 | 95.5 | 4.03 | 0 | 21.3 | 48.5 | 26.9 | 3.37 |

### 4-3. Confirmation of proportions of CD4 and CD8 in T cells expressing 1G4 TCR

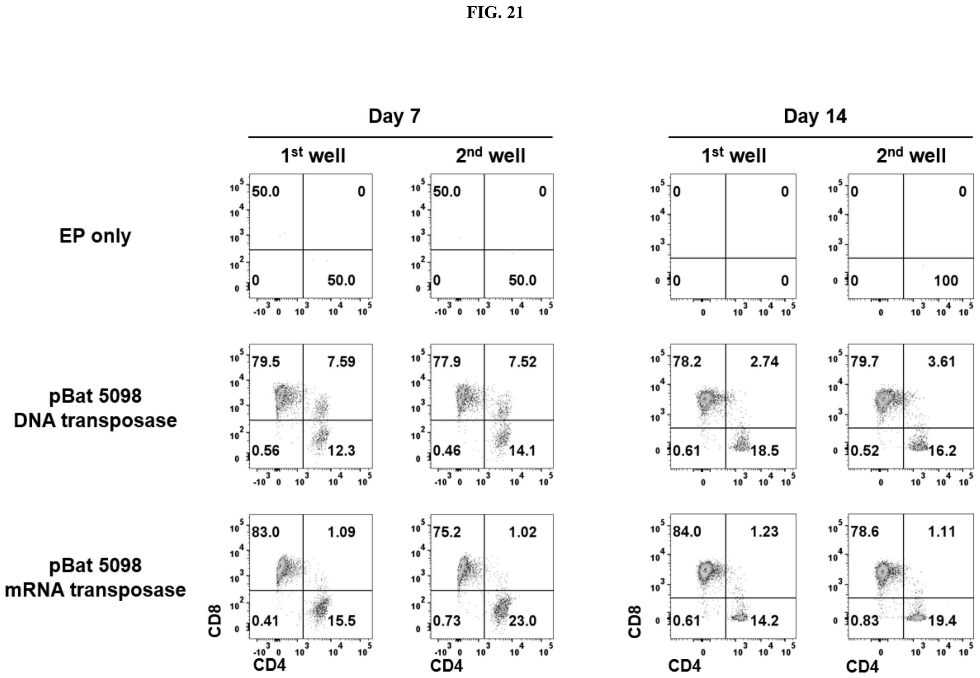

In order to confirm the proportions of CD4 and CD8 in T cells expressing 1G4 TCR according to the delivery type of the transposase gene, the proportions were confirmed on days 7 and 14 after transformation. As a result, as shown in FIG. 21 and Table 12, it was confirmed that on day 7 after transformation, the proportions of CD8+ and CD4+ in the case of the DNA type optimized transposase were 86.3% and 20.8%, respectively, and the proportions of CD8+ and CD4+ in the case of the mRNA type optimized transposase were 82.1% and 20.5%, respectively. In addition, on day 14, the proportions of CD8+ and CD4+ in the case of the DNA type optimized transposase were about 80.2% and 20.3%, respectively, and the proportions of CD8+ and CD4+ in the case of the mRNA type optimized transposase were about 82.5% and 17.8%, respectively, indicating that that the proportion of CD8+ cells was high in both delivery types without any difference.

**[Table 12]**

| Test group | Day 7 | | Day 14 | |
|---|---|---|---|---|
| | CD8+ | CD4+ | CD8+ | CD4+ |
| DNA Transposase | 87.1% | 19.9% | 80.9% | 21.2% |
| | 85.4% | 21.6% | 83.3% | 19.8% |
| mRNA Transposase | 84.1% | 16.6% | 85.2% | 15.4% |
| | 76.2% | 24.0% | 79.7% | 20.5% |

### 4-4. Results of luciferase-based cytotoxicity assay

For the *in vitro* killing test of 1G4 TCR-T cells cultured for 14 days, T2-Luc cells pulsed with 100 µg/mL of peptide were co-cultured at ratios of 30:1, 10:1, 3:1, and 1:1 (effector cells: target cells), and the luciferase detected when the target cells were killed was converted into percentage (%) values to calculate the luciferase assay expression rate. As a result, as shown in FIG. 22 and Table 13, in the "No E/P (negative control) group" in which 1G4 TCR was not expressed, the luciferase assay expression rate (%) was less than 7.0% under the 30:1 condition, and it was not detected under the 10:1, 3:1, and 1:1 conditions. In the group in which the mRNA type optimized transposase was used when introducing 1G4 TCR using the transposon system, the luciferase assay expression rate (%) was 67.3%, 48.1%, and 13.4% under the 30:1, 10:1, and 3:1 conditions, respectively, and it was not detected under the 1:1 condition. In the group in which the DNA type optimized transposase was used, the luciferase assay expression rate (%) was 54.2% and 32.2% under the 30:1 and 10:1 conditions, respectively, and it was not were not detected under the 3:1 and 1:1 conditions. As a result, it was confirmed that the cytotoxicity activity was more than 10% higher in the 1G4 TCR-T cells delivered with the mRNA type optimized transposase.

**[Table 13]**

| Effector : Target ratio | mRNA Transposase | | DNA Transposase | | No E/P | |
|---|---|---|---|---|---|---|
| | Plate 1 | Plate 2 | Plate 1 | Plate 2 | Plate 1 | Plate 2 |
| 30:1 | 73.6 | 60.9 | 63.8 | 44.6 | 7.5 | -0.91 |
| 10:1 | 50.0 | 46.2 | 34.0 | 30.3 | -1.2 | 1.63 |
| 3:1 | 12.4 | 14.3 | -0.24 | -3.3 | -13.2 | 11.6 |
| 1:1 | -21.7 | -5.4 | -25.8 | -24.7 | -18.1 | -22.1 |

As described above, in order to confirm GFP gene expression according to the delivery type (mRNA and DNA) of transposase when delivering genes using the transposon system to PBMC cells, GFP gene expression was confirmed through FACS analysis on days 7 and 14 of culture after transformation by Neon electroporation. As a result, it was confirmed that on day 7, the GFP expression rate of the mRNA type optimized transposase group was about 10% higher than that of the DNA type optimized transposase group, and on day 14, the expression rate of the mRNA type optimized transposase group was about 5% higher. In addition, after transformation with the 1G4 TCR gene using MaxCyte electroporation, which is a different type of electroporator equipment, the expression of the 1G4 TCR gene was confirmed through FACS analysis on days 7 and 14 of culture, and the results confirmed that the 1G4 TCR gene was not expressed in the negative control group "EP only group" that underwent only electroporation. On the other hand, it was confirmed that the expression of the 1G4 TCR gene in the mRNA type optimized transposase group on day 7 after transformation was about 29% higher than that in the DNA type optimized transposase group. In addition, it was also confirmed that after 14 days of culture, the expression rate in the mRNA type optimized transposase group was about 20% higher than that in the DNA type optimized transposase group. In addition, it was confirmed that there was no difference in the proportions of the memory types of T_{SCM} and T_{CM} and the proportions of CD4 and CD8 regardless of the gene delivery type. In addition, for the *in vitro* killing test of 1G4 TCR-T cells cultured for 14 days, T2-Luc cells pulsed with 100 µg/mL of peptide were co-cultured at ratios of 30:1, 10:1, 3:1, and 1:1 (effector cells: target cells), and the luciferase detected when the target cells were killed was converted into percentage (%) values to calculate the luciferase assay expression rate. As a result, it was confirmed that in the "No E/P (negative control) group" exhibiting no 1G4 TCR expression, no luciferase was detected under any conditions, and the result values of the cytotoxicity assay were higher by 10% or more in the mRNA type optimized transposase group than in the DNA type optimized transposase group under all ratio conditions. From the above-described results, it was confirmed that the gene expression rate and cytotoxicity effect were higher when the transposase gene was delivered in the mRNA form of the transposon system than in the DNA form.

### Example E. Confirmation of gene delivery efficiency of pBat transposase after adding a nuclear localization sequence (NLS) gene

A transposase moves into the nucleus of the cell after translation into protein, and thus an NLS gene was added to the 5' end or 3' end of the optimized transposase to increase the efficiency of transposase movement into the nucleus, and then the gene delivery efficiency was confirmed.

SV40 and C-myc were selected as an NLS to be added to the 5' end or 3' end of the optimized transposase, and the DNA sequences of SV40 and C-myc were also optimized for good expression in T cells.

**[Table 14]**

| NLS | Sequence (5'→3') | |
|---|---|---|
| SV40 | Addgene #115136 | cccaagaagaagaggaaagtc (SEQ ID NO: 4) |
| | Addgene #117811 | ccaaagaagaagcgtaaggta (SEQ ID NO: 5) |
| | Addgene #114086 | ccaaagaaaaagaggaaagtc (SEQ ID NO: 6) |
| | Addgene #114086 | cctaaaaagaaacgaaaggtt (SEQ ID NO: 7) |
| | Creative Biogene | CCAAAAAAGAAGAGAAAGGTA (SEQ ID NO: 8) |
| | Optimization | CCCAAAAAGAAGCGCAAGGTG (SEQ ID NO: 9) |
| C-myc | Creative Biogene | CCTGCTGCCAAGAGGGTCAAGTTGGAC (SEQ ID NO: 10) |
| | Optimization | CCCGCGGCTAAGCGCGTGAAGCTGGAC (SEQ ID NO: 11) |

As shown in FIGs. 22 and 23, optimized SV40 or C-myc was added to the 5' end or 3' end of optimized transposase (SEQ ID NO: 2) to optimize the optimized transposase.

### 1. Confirmation of GFP gene delivery efficiency

The GFP gene delivery efficiency of the optimized transposase was confirmed by adding an NLS to the 5' or 3' end. To confirm the GFP gene delivery efficiency, Jurkat cells (1x10⁵/well, 24 well plate), a T cell line, were used, and Neon transfection (electroporation) was performed under the conditions of 1,600 V, 10 ms, and 3 pulses. The transposon and the DNA type optimized transposase shown in Table 15 below were used at a ratio of transposon: transposase = 1 µg: 1 µg. The GFP expression level in the cells was confirmed using a fluorescence microscope and FACS 1, 7, and 14 days after transfection. Jurkat cells cultured in a 24-well plate were transferred to a T25 flask one day after neon transfection.

**[Table 15]**

| **transposon vector No.** | **transposase** |
|---|---|
| 5096_GFP | 5088 ver1.1 |
| | + 5' SV40 |
| | + 5' cmyc |
| | + 3' SV40 |
| | + 3' cmyc |

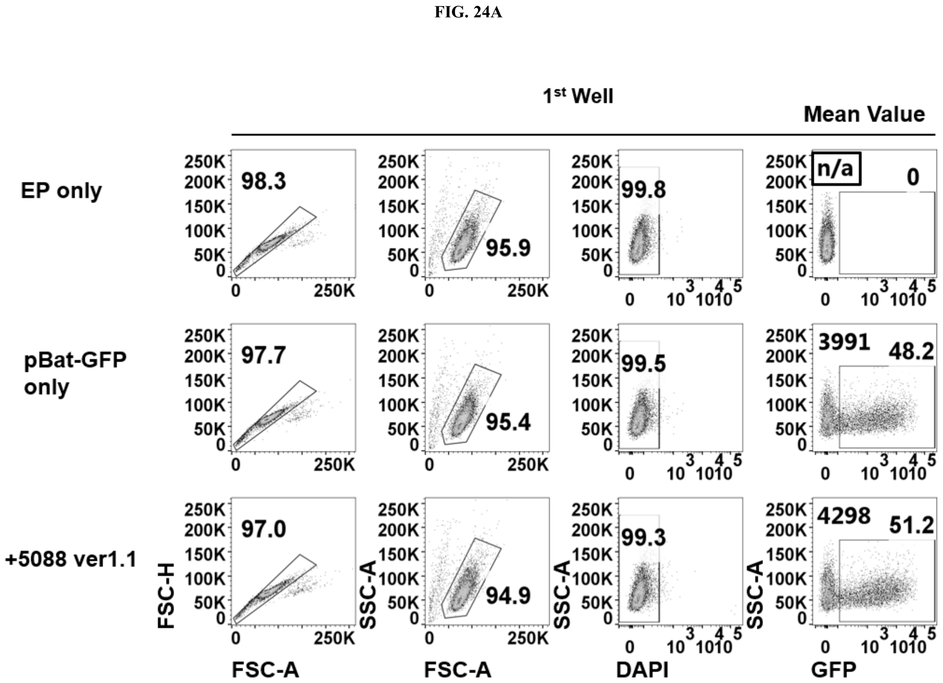

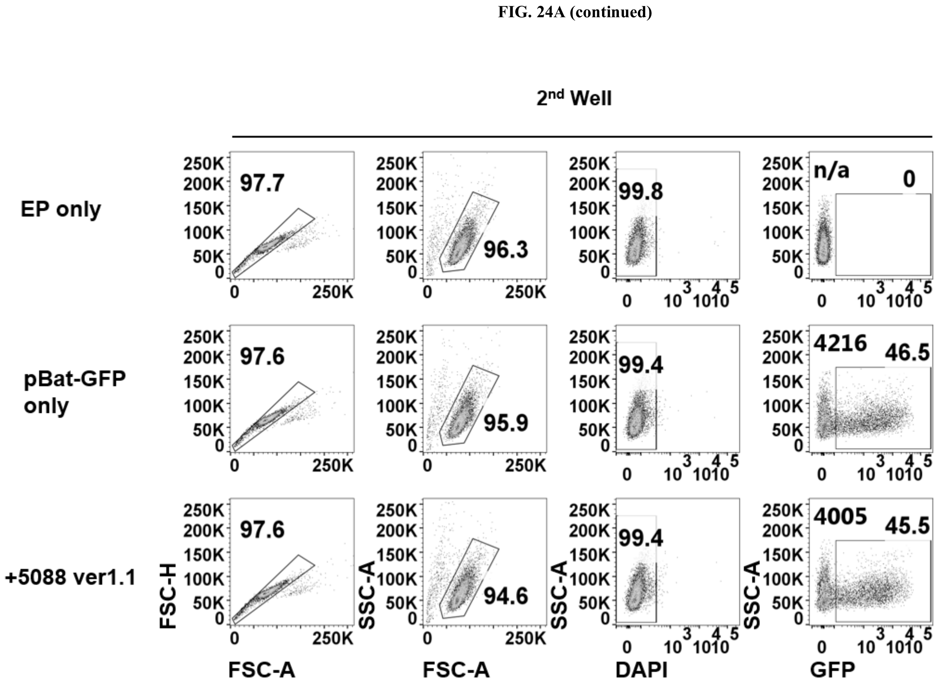

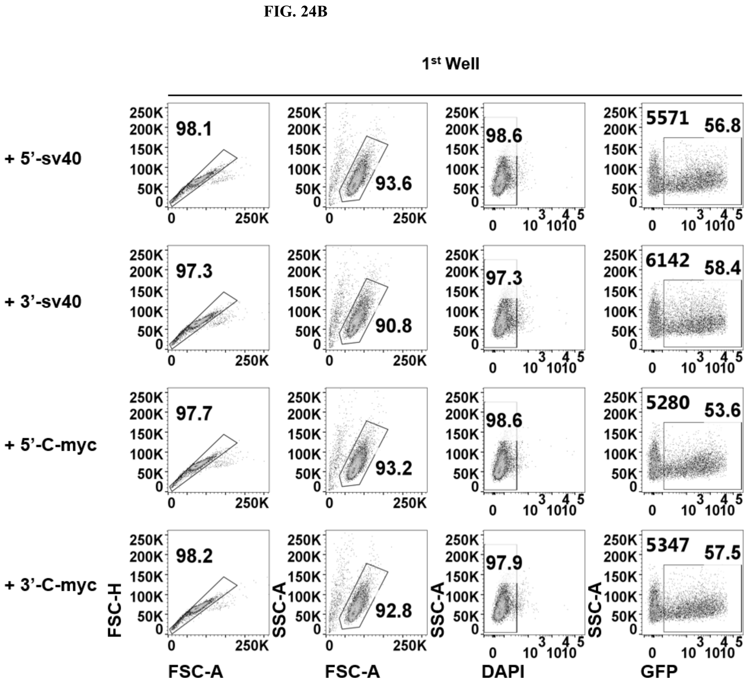

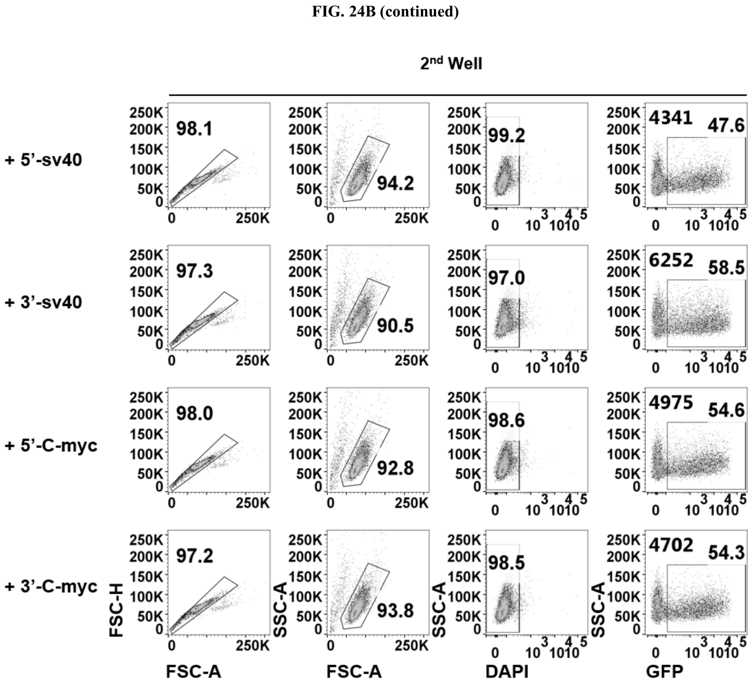

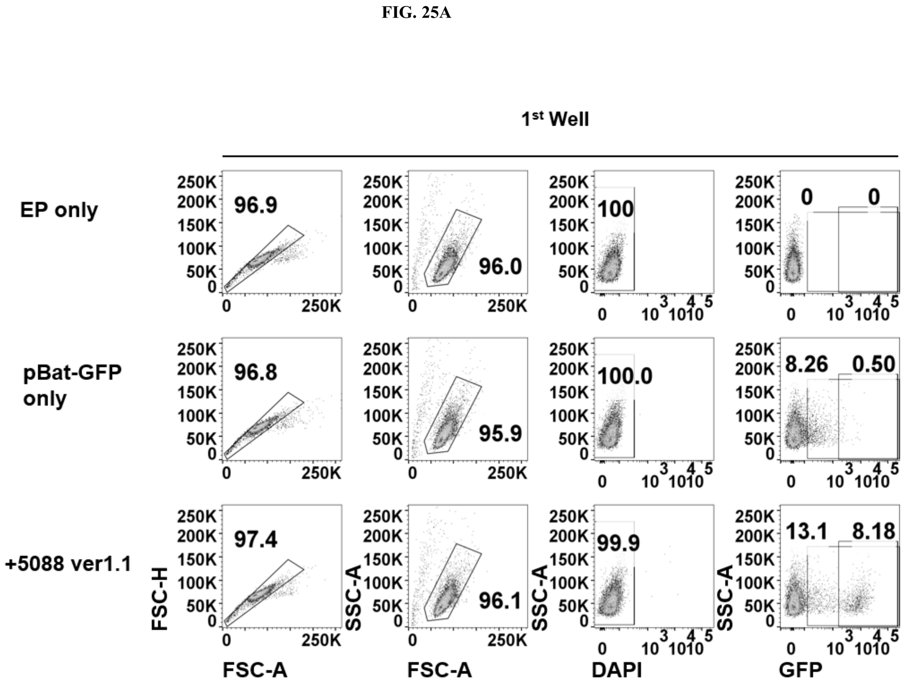

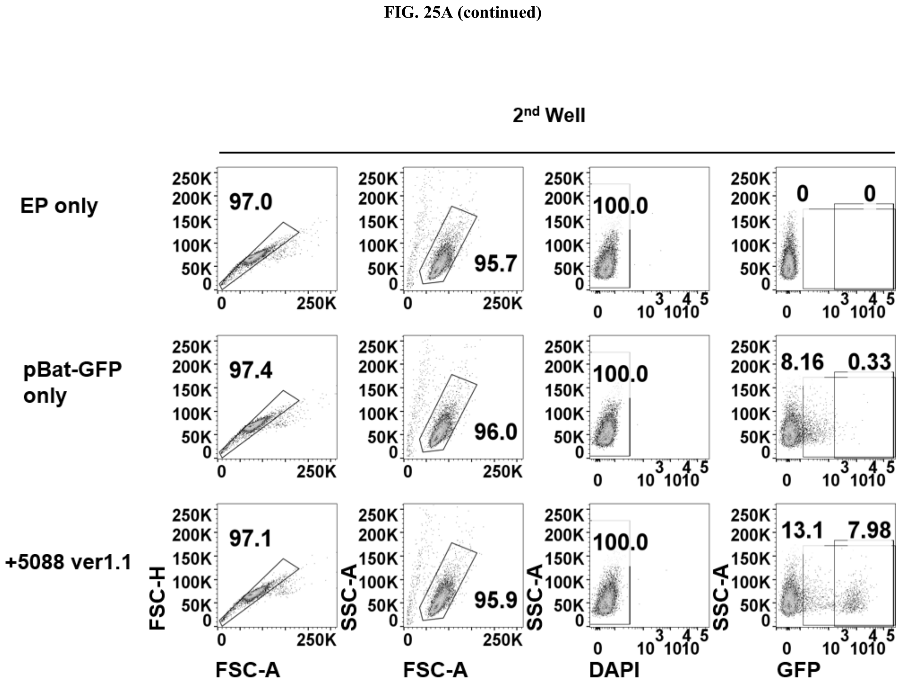

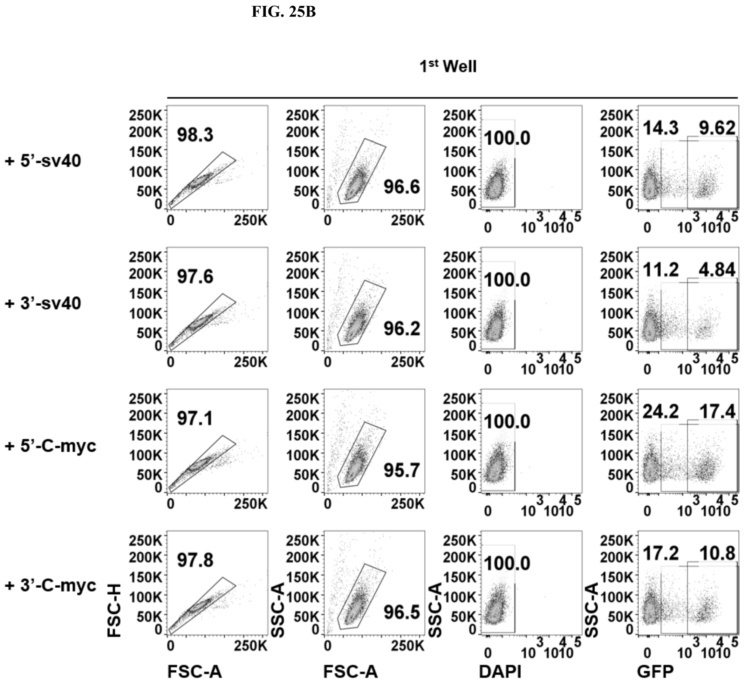

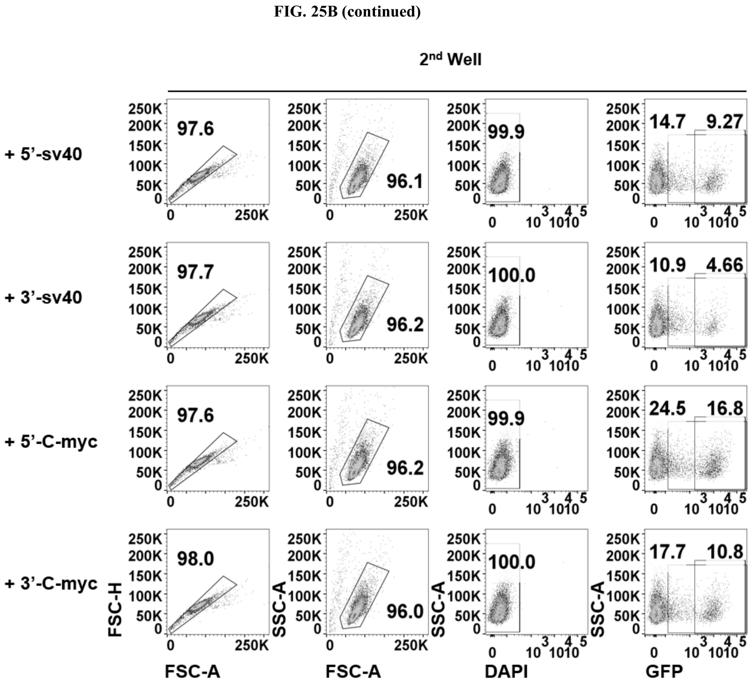

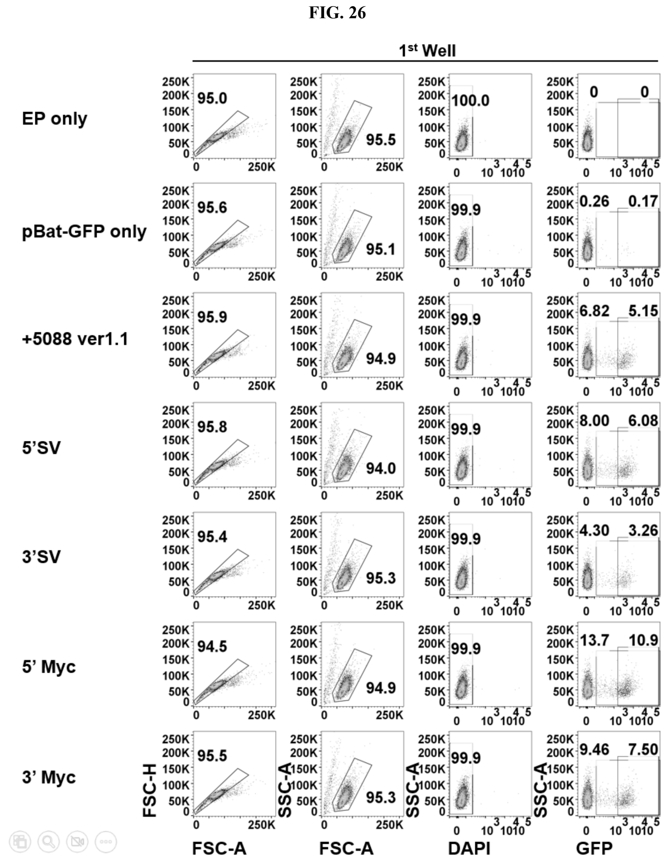

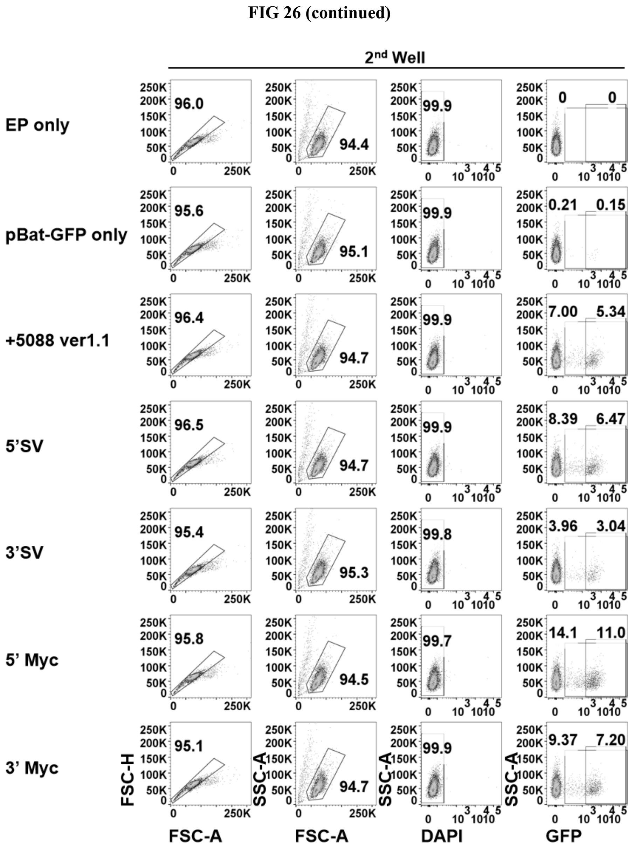

As shown in FIG. 24A and FIG. 24B, one day after electroporation, there was no difference according to the type and location (5' or 3') of the NLS. As shown in FIG. 25A and FIG. 25B, it was confirmed that seven days after electroporation, when C-myc was located at the 5' end, the overall GFP expression rate and the high intensity GFP expression rate were high. The GFP expression rate 14 days after electroporation was the same as that shown in FIG. 26.

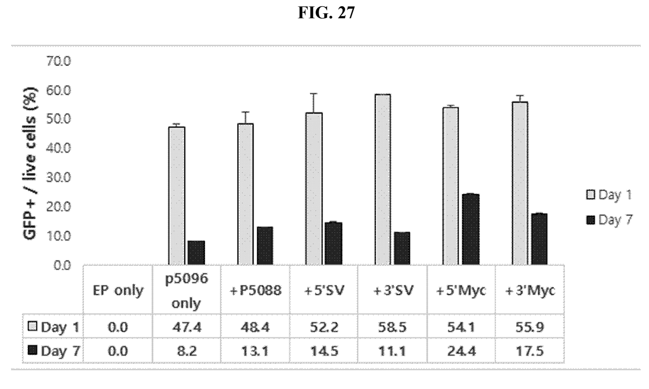

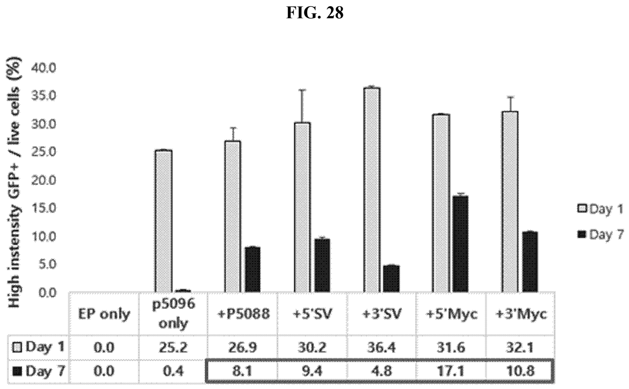

To confirm the efficiency of the NLS transposase in Jurkat cells, FACS analysis was performed on GFP expression one and seven days after electroporation. As a result, as shown in FIG. 27, it was confirmed that one day after electroporation, 3' SV exhibited the highest GFP expression, but seven days after electroporation, 5' Myc exhibited the highest, followed by 3' Myc and the optimized transposase without an NLS. In addition, as shown in FIG. 28, the high intensity GFP expression rate was 8% in the optimized transposase without an NLS, 17% in 5' Myc, and 11% in 3' Myc, and the transposase with 5' Myc insertion exhibited the best stable transfection efficiency in Jurkat cells.

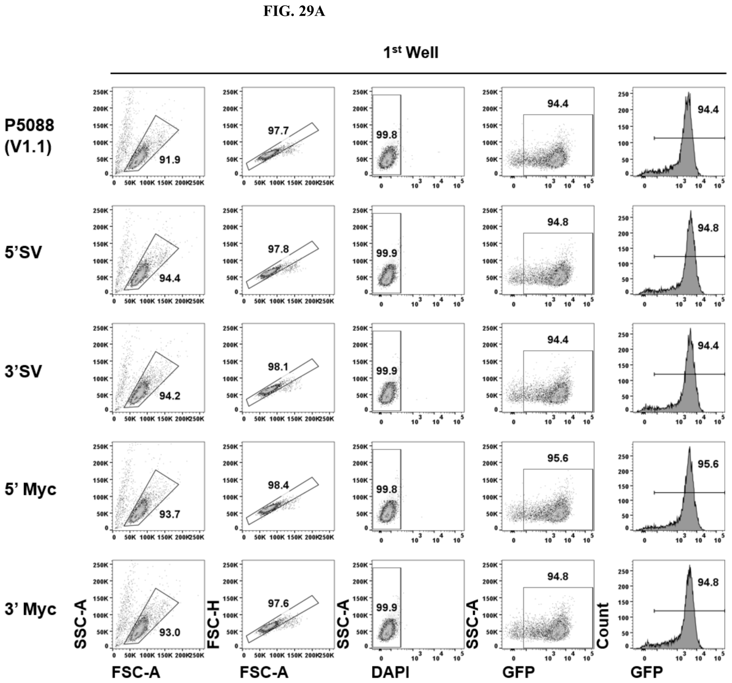

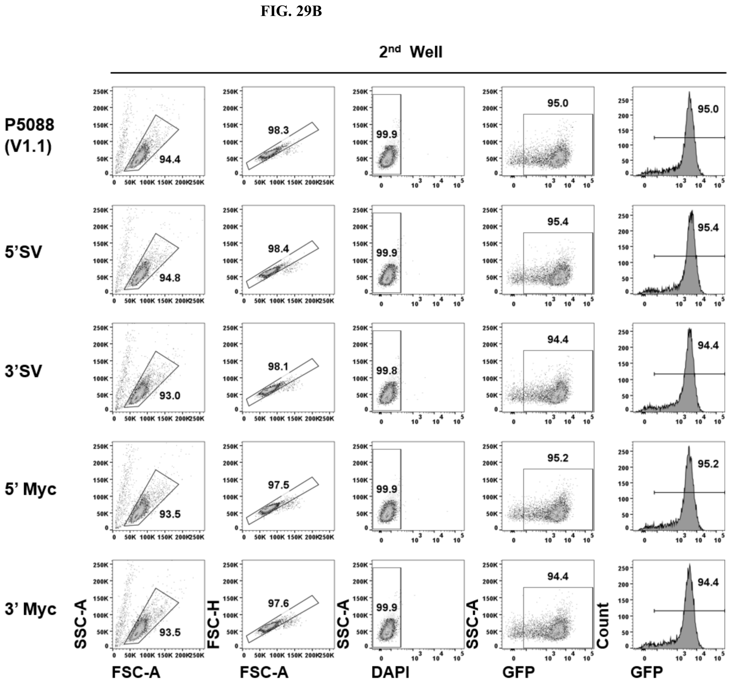

In addition, Jurkat cells expressing GFP on day 14 of electroporation were sorted on day 15 and additionally cultured for nine days. As a result, as shown in FIG. 29A and FIG. 29B, it was confirmed that the GFP expressing cells were stably maintained at a GFP expression rate of 95%.

### 2. Confirmation of 1G4 TCR gene delivery efficiency

The 1G4 TCR gene delivery efficiency of the optimized transposase which was optimized by adding an NLS to the 5' or 3' end in PBMCs was confirmed. Specifically, PBMCs (LK048, 17011) stored in a liquid nitrogen (LN₂) tank were used, and transfection (electroporation) using MaxCyte was performed using OC100X2, the Resting T cell 14-3 protocol with 5x10⁶ PBMC/50 µL opti-MEM buffer. As a plasmid, the transposon vector and the DNA type optimized transposase shown in Table 16 below were used at a ratio of transposon: transposase = 8 µg: 2 µg. FACS analysis was performed 7 and 14 days after transfection.

**[Table 16]**

| **Transposon** | **transposase** |
|---|---|
| p8at-1G4 TCR | Optimized transposase plasmid DNA |
| | + 5' SV40 |
| | + 5' cmyc |
| | + 3' SV40 |
| | + 3' cmyc |

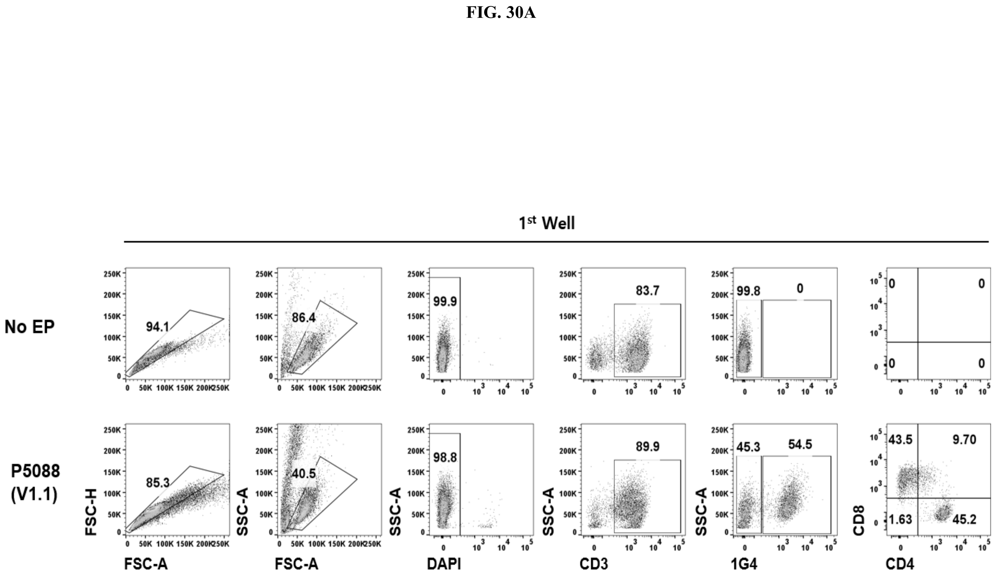

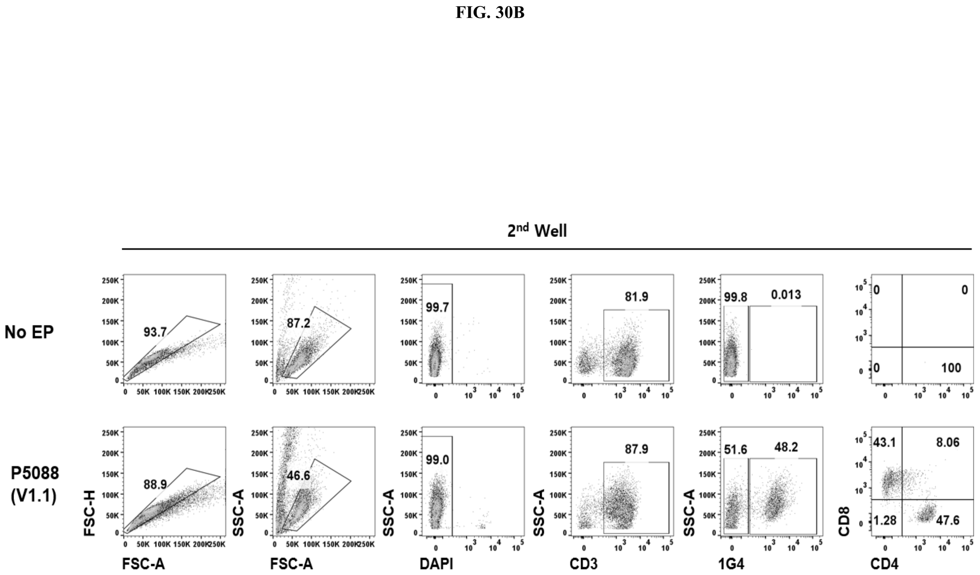

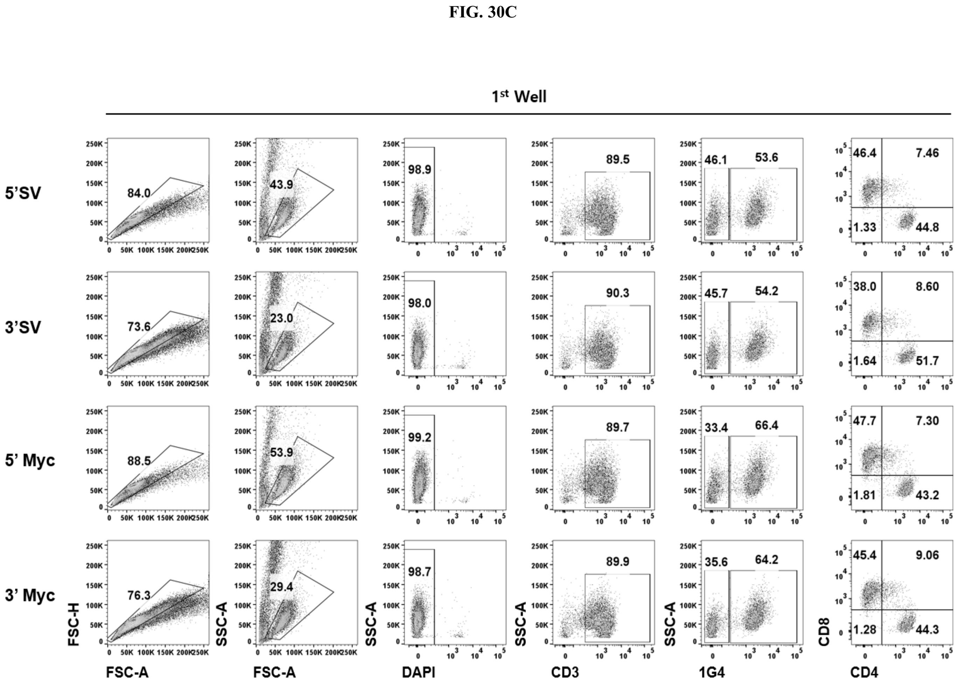

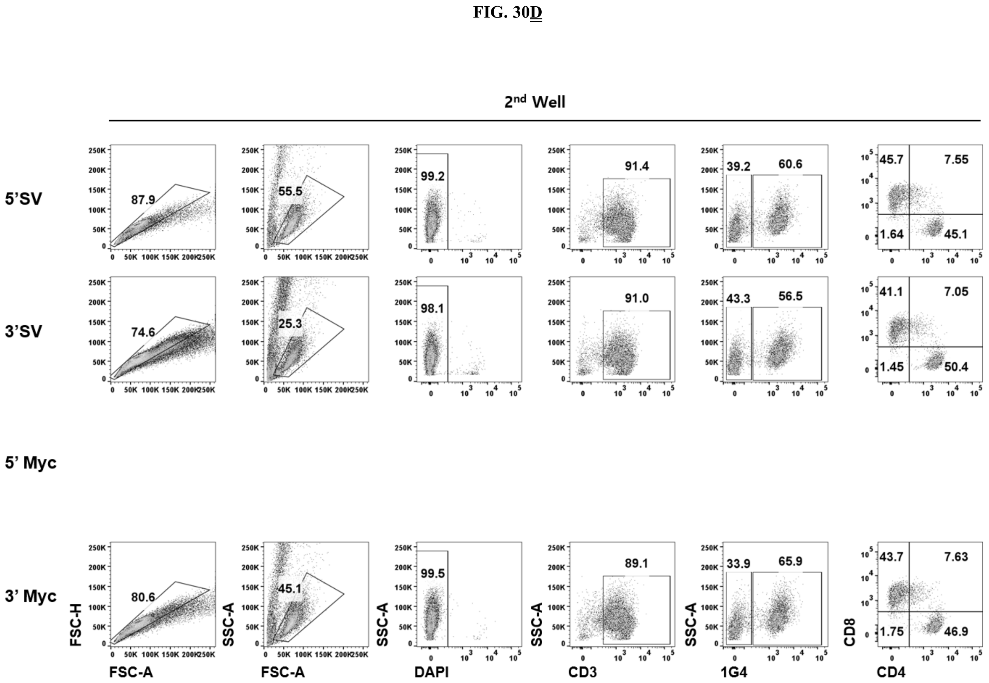

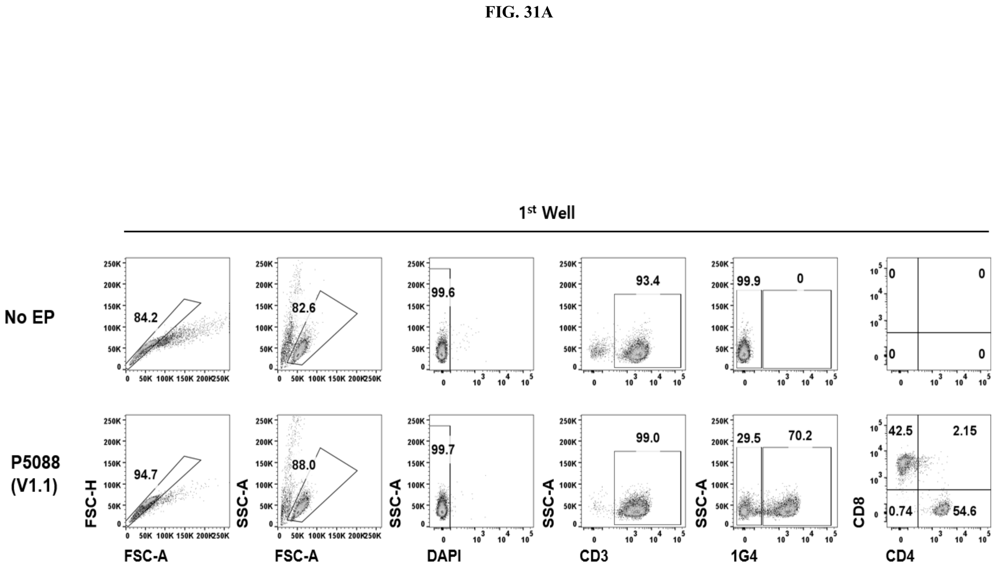

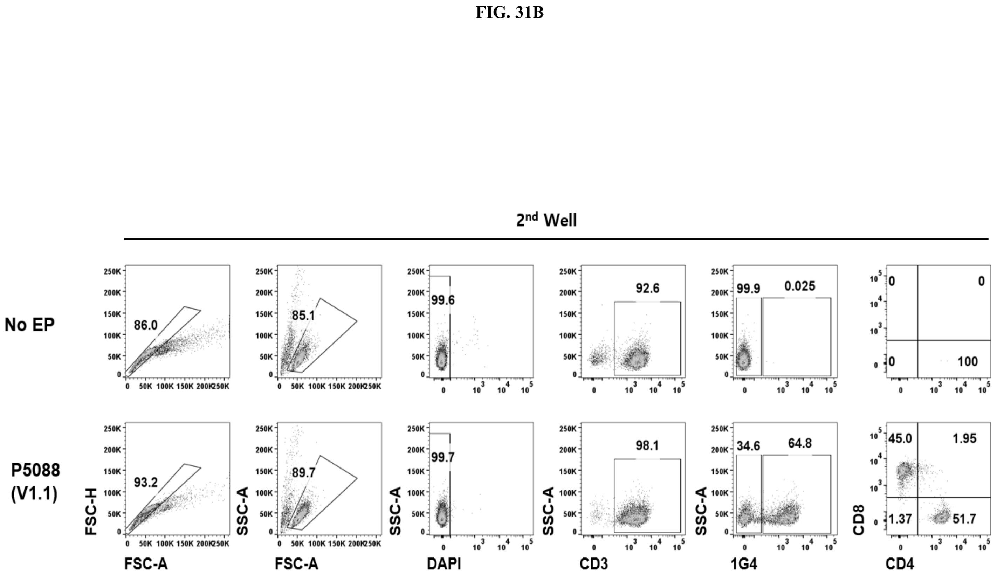

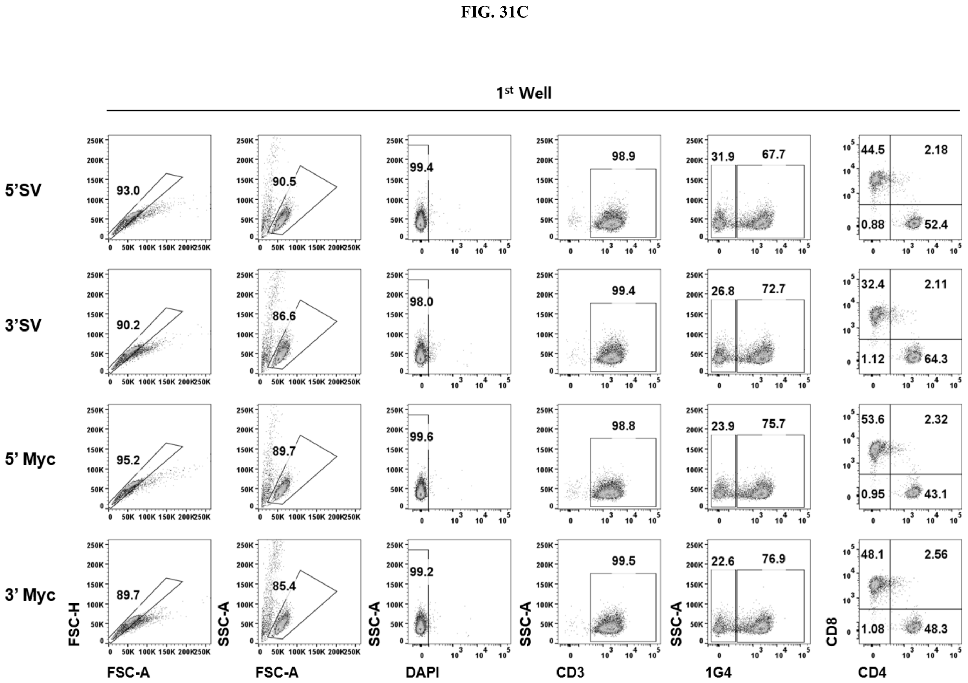

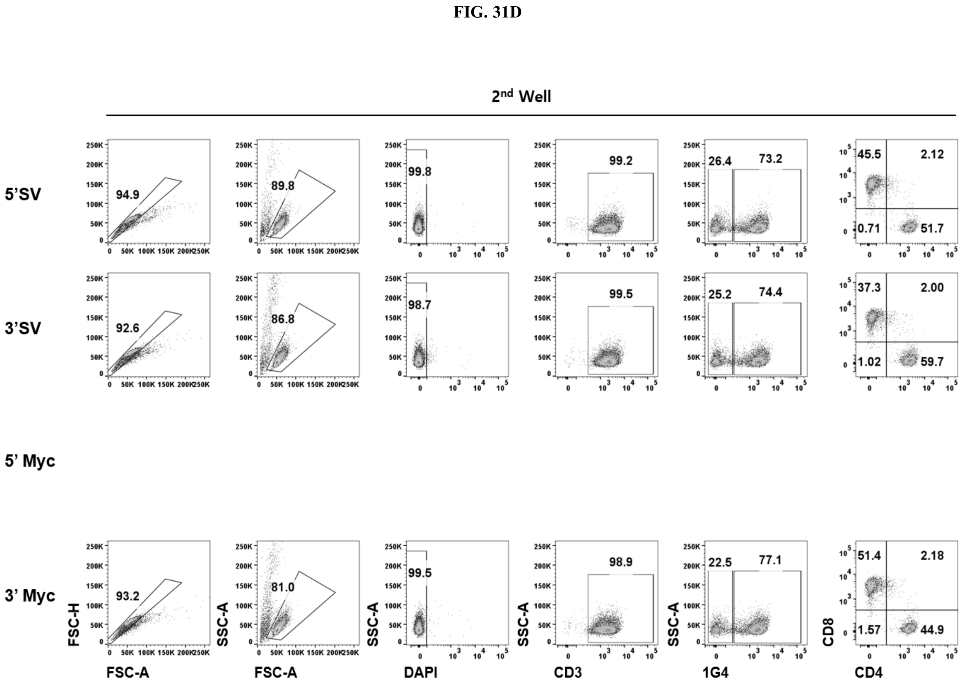

The 1G4 TCR gene delivery efficiency in PBMCs was confirmed with 8 µg/well of transposon and 2 µg/well of transposase. As a result of FACS analysis performed seven days after transfection, as shown in FIGs. 30A to 30D, it was confirmed that the 1G4 TCR expression rate was high in the C-myc group. As a result of FACS analysis performed 14 days after transfection, as shown in FIGs. 31A to 31D, it was confirmed that, similar to day 7, the 1G4 TCR expression rate was high in the C-myc group.

### Example F. Comparison of gene delivery efficiency between pBat transposon and lentivirus using CD19 CAR

The gene delivery efficiency of the nonviral vector pBat transposon and the viral vector lentivirus was compared.

### [Method]

### 1. DNA and cells

To compare the nonviral vector pBat transposon and the viral vector lentivirus, the pBat transposon plasmid vector and the pBat optimized transposase plasmid vector (SEQ ID NO: 14) were used as the nonviral vector pBat transposon, and the lentivirus CD19 CAR transfer plasmid vector and the lentivirus packaging vector were used for the production of lentivirus.

As target cells, PBMCs (LK032/LK053), Lenti-X 293T (Takara, Cat no. 632180, Lot no. AIY0002S), Jurkat, Clone E6-1 (ATCC, Cat No. TIB-152, Lot no. 70017560), NALM6, and a GFP-Luciferase reporter cell line were used.

### 2. Production of pBat CD19 CAR plasmid vector

In order to compare the gene delivery efficiency in CAR-T production with the lentiviral transfer vector, the following cloning method was performed to replace the EGFP gene portion in the pBat transposon plasmid vector containing EGFP with CD19 CAR to produce the pBat CD19 CAR plasmid vector.
1) The CD19 CAR gene to be used as an insert was subjected to PCR using primers with the plasmid containing the CD19 CAR gene as a template, and then the PCR product was loaded onto an agarose gel to elute the DNA of the band of the desired size.
2) The DNA of the band of the desired size was eluted by treating it with the restriction enzymes BstB I and EcoR I in the pBat transposon plasmid vector containing EGFP.
3) Gibson assembly cloning was performed using the DNA obtained in 1) and 2).
4) To confirm pBat CD19 CAR among the clones for which cloning was completed, enzyme mapping was performed using the restriction enzyme EcoR V, and candidate clones were selected and finally sequenced.

### 3. PBMC activation

1) RPMI medium (RPMI + 10% FBS + 1X P/S) was prepared by prewarming in a 37 °C water bath.
2) PBMC LK032 and LK053 were taken out from a nitrogen tank and quickly thawed in a 37 °C water bath.
3) Two 50 mL tubes were prepared, 30 mL of culture medium was added to each, and the cells from the thawed PBMC LK032 and PBMC LK053 vials were placed in each of the 50 mL tubes.
4) Centrifugation was performed at 1,500 rpm for five minutes, the supernatant was removed, cells were suspended in 40 mL of RMPI medium, and cell counting was performed.
5) 1.2 x 10⁸ PBMCs were added to a T175 flask, and RPMI medium was added to make 1.2 x 10⁸/120 mL.
6) IL-2 was added to a final concentration of 20 IU/mL, and 100 µL of TransAct^{™} was added per 2.0 x 10⁷ (IL-2 was diluted 1/10 with medium from a concentration of 1.0 x 10⁶ IU/mL to 1.0 x 10⁵ IU/mL.).
7) Culture was performed in a 37 °C, 5% CO₂incubator for two days.

### 4. PBMC electroporation

The nonviral transposon system containing the CD19 CAR gene was delivered into the activated PBMCs as described below.
1) The activated PBMCs were collected in a conical tube, centrifuged at 300 x g for ten minutes, and the supernatant was removed.
2) The cell pellet was suspended in 20 mL of medium (ALYS505N-O + 3% HS), and cell counting was performed.
3) After centrifugation at 1,500 rpm for five minutes at room temperature, the supernatant was removed, and the cell pellet was suspended in 20 mL of DPBS.
4) After centrifugation at 1,500 rpm for five minutes at room temperature, the supernatant was completely removed.
5) The supernatant was removed, and the cell pellet was suspended in 5 mL of Opti-MEM medium.
6) After centrifugation at 1,500 rpm for five minutes at room temperature, the supernatant was completely removed.
7) The cell pellet was suspended in 50 µL of warm Opti-MEM medium to 5.0 x 10⁶/50 µL.
8) Each of the transposon vector and the transposase vector was added in an amount of 5 µg per well to the tube in 7) as shown in Table 17 below. However, no vector was added to the control group.

**[Table 17]**

| No. | Group | Cell No. | pBat 3M3-5M3# transposon | Optimized transposase | IL-2 |
|---|---|---|---|---|---|
| 1 | Electroporation only | 5.0 x 10⁶ cells | | - | 200 IU/mL |
| 2 | Electroporation only | 5.0 x 10⁶ cells | | - | 400 IU/mL |
| 3 | Transposon _CD 19 CAR | 5.0 x 10⁶ cells | pBat 3M3-5M3 CD19-CAR | DNA type optimized transposase | 200 IU/mL |
| 4 | Transposon _CD19 CAR | 5.0 x 10⁶ cells | pBat 3M3-5M3 CD19-CAR | DNA type optimized transposase | 400 IU/mL |

| | | | | | |
|---|---|---|---|---|---|
| **The experiment was performed with n=2 in each group, and in the case of LK053, the experiment was performed only under the condition of 200 IU/mL IL-2. | | | | | |

9) 5 x 10⁶ cells/50 µL of the cell suspension from 7) was carefully added to the OC100X2 assembly to avoid creating bubbles.
10) For electroporation, the Resting T cell 14-3 protocol was selected in MaxCyte STx.
11) The OC100X2 assembly from 9) was inserted into the chamber in the STx, and electroporation was carried out by performing the protocol.
12) After electroporation, the cell suspension from the OC100x2 assembly was transferred to a T25 flask (5 x 10⁶ cells/50 µL/well).
13) The OC100X2 well was washed with 50 µL of Opti-MEM medium and added to each well of the plate from 11).
14) A recovery time of 20 minutes in a 37 °C, 5% CO₂ incubator was provided.
15) After carefully adding 10 mL of complete medium (ALYS505N-O + 3% HS + 200 IU/mL IL-2) to the T25 flask containing PBMCs, the resulting mixture was placed back into the 37 °C, 5% CO₂ incubator and cultured for two days.
16) Two days after electroporation, 20 mL of medium (ALYS505N-O + 3% HS + 1x P/S) was added to the control group, and 10 mL of medium was added to the CAR group, and then IL-2 was added to a final concentration of 200 IU/mL or 400 IU/mL, and culture was performed in a 37 °C, 5% CO₂ incubator.
17) Five days after electroporation, IL-2 was added to a final concentration of 200 IU/mL or 400 IU/mL, and culture was performed in a 37 °C, 5% CO₂ incubator.
18) Seven days after electroporation, the cultured cells were suspended, and 500 µL of the cell suspension was transferred to a new tube for FACS analysis. The remaining cell suspension was added to new medium (ALYS505N-O + 3% HS + 1x P/S) to a concentration of 5.0 x 10⁶/mL, IL-2 was added to a final concentration of 200 IU/mL or 400 IU/mL, and culture was performed in a 37 °C, 5% CO₂ incubator.
19) Nine days after electroporation, the cultured cells were suspended, and to store half of the suspension as a stock, the supernatant was removed, and CS10 medium was added. The resulting mixture was stored in a deep freezer for one day and then transferred to a nitrogen tank.
20) The remaining half of the suspension was added to new medium (ALYS505N-O + 3% HS + 1x P/S) to a concentration of 5.0 x 10⁶ cells/mL, IL-2 was added to the final concentration of 200 IU/mL or 400 IU/mL, and culture was performed in a 37 °C, 5% CO₂ incubator.
21) Twelve days after electroporation, the cultured cells were suspended, and new medium (ALYS505N-O + 3% HS + 1x P/S) was added at a concentration of 5.0 x 10⁶ cells/mL. IL-2 was added to a final concentration of 200 IU/mL or 400 IU/mL, and culture was performed in a 37 °C, 5% CO₂ incubator.
22) Fourteen days after electroporation, the cultured cells were suspended, and 500 µL of the cell suspension was transferred to a new tube for FACS analysis. 2.0x 10⁷ cells per group were harvested for an *in vitro* killing analysis and cultured in 20 mL of Resting medium (RPMI1640 + 10% FBS + 1x P/S). To prepare the remaining suspension into a stock, the supernatant was removed, and CS10 medium was added. The resulting mixture was stored in a deep freezer for one day and then transferred to a nitrogen tank.

### 5. Lentiviral transduction

The lentivirus containing the CD19 CAR gene was delivered into activated PBMCs as described below.
1) The activated PBMCs were collected in a 50 mL conical tube and centrifuged at 300 x g for ten minutes at room temperature.
2) The supernatant was removed, the cell pellet was suspended in 20 mL of culture medium (RPMI1640 + 10% FBS + 1x P/S), and cell counting was performed.
3) The PBMCs were suspended in culture medium to 5.0 x 10⁶/2 mL.
4) The required number of wells were seeded in a 12-well plate at 5.0 x 10⁶/2 mL/well.
5) Each well was treated with 10 µg/mL protamine and lentivirus with a multiplicity of infection (MOI) of 4.
6) Spinoculation was performed at 1,200 x g for 90 minutes.
7) After centrifugation, PBMCs from each well were transferred to a T75 flask.
8) Culture medium was added to the T75 flask so that the total volume was 10 mL, and the test groups are as shown in Table 18 below.

**[Table 18]**

| No. | Group | Cell No. | Activation | Lentivirus | IL-2 |
|---|---|---|---|---|---|
| 1 | Activation only | 5.0 x 10⁶ cells | TransAct^{™} | - | 200 IU/mL |
| 2 | Activation only | 5.0 x 10⁶ cells | TransAct^{™} | - | 400 IU/mL |
| 3 | Lentivirus_CD19 CAR | 5.0 x 10⁶ cells | TransAct^{™} | 4 MOI | 200 IU/mL |
| 4 | Lentivirus_CD19 CAR | 5.0 x 10⁶ cells | TransAct^{™} | 4 MOI | 400 IU/mL |

9) IL-2 was added to a final concentration of 200 IU/mL or 400 IU/mL, and culture was performed in an incubator at 37 °C, 5% CO₂.
10) Two days after transduction, the cultured cells were suspended, and new medium (RPMI1640 + 10% FBS + 1x P/S) was added to the cell suspension to a concentration of 5.0 x 10⁶/mL. IL-2 was added to a final concentration of 200 IU/mL or 400 IU/mL, and culture was performed in an incubator at 37 °C, 5% CO₂.
11) Five days after transduction, the same amount of new medium (RPMI1640 + 10% FBS + 1x P/S) was added, IL-2 was added to a final concentration of 200 IU/mL or 400 IU/mL, and culture was performed in an incubator at 37 °C, 5% CO₂.
12) Seven days after transduction, the cultured cells were suspended, and 500 µL of the cell suspension was transferred to a new tube for FACS analysis. New medium (RPMI1640 + 10% FBS + 1x P/S) was added to the remaining cell suspension to a concentration of 5.0 x 10⁶/mL, IL-2 was added to a final concentration of 200 IU/mL or 400 IU/mL, and culture was performed in an incubator at 37 °C, 5% CO₂.
13) Nine days after transduction, the cultured cells were suspended, and to prepare half of the suspension as a stock, the supernatant was removed, and CS10 medium was added. The resulting mixture was stored in a deep freezer for one day and then transferred to a nitrogen tank. New medium (RPMI1640 + 10% FBS + 1x P/S) was added to the remaining half of the suspension to a concentration of 5.0 x 10⁶ cells/mL, IL-2 was added to a final concentration of 200 IU/mL or 400 IU/mL, and culture was performed in an incubator at 37 °C, 5% CO₂.
14) Twelve days after transduction, the cultured cells were suspended, new medium (RPMI1640 + 10% FBS + 1x P/S) was added to a concentration of 5.0 x 10⁶ cells/mL, IL-2 was added to a final concentration of 200 IU/mL or 400 IU/mL, and culture was performed in an incubator at 37 °C, 5% CO₂.
15) Fourteen days after transduction, the cultured cells were suspended, and 500 µL of the cell suspension was transferred to a new tube for FACS analysis. 2.0 x 10⁷ cells per group were harvested for an *in vitro* killing analysis and cultured in 20 mL of Resting medium (RPMI1640 + 10% FBS + 1x P/S). To prepare the remaining suspension into a stock, the supernatant was removed, and CS10 medium was added. The resulting mixture was stored in a deep freezer for one day and then transferred to a nitrogen tank.

### 6. FACS analysis

FACS analysis was performed 7, 10, and 14 days after electroporation.
1) Cells were suspended in each group's flask and transferred to 3 mL each FACS tube.
2) Centrifugation was performed at 4 °C and 1,500 rpm for five minutes, the supernatant was removed, and cells were suspended with 1 mL of washing buffer (PBS + 2% FBS).
3) Step 2) was repeated once more for washing.
4) Centrifugation was performed at 4 °C and 1,500 rpm for five minutes, and the supernatant was removed.
5) 5 µL of human TruStain FcX + 95 µL of FACS buffer were added to each tube, and the resulting mixture was incubated for five minutes at room temperature.
6) Each antibody (anti-CD3, anti-CD4, anti-CD8, anti-CD45RA, anti-CCR7, anti-CD62L, anti-FLAG TAG) was added in an amount of 2 µL per tube, and the resulting mixture was incubated for 30 minutes at room temperature in a dark place.
7) 1 mL of FACS buffer (PBS + 2% FBS) was added, and centrifugation was performed at 4 °C and 1,500 rpm for five minutes.
8) The supernatant was removed, and cells were suspended with 1 mL of FACS buffer (PBS + 2% FBS).
9) Centrifugation was performed at 4 °C and 1,500 rpm for five minutes, and the supernatant was removed.
10) Cells were suspended with 200 µL of FACS buffer (PBS + 2% FBS) containing 1x DAPI, and FACS analysis was performed.

### 7. Luciferase-based cytotoxicity assay after co-culture of CD19 CAR-T cells and GFP-luciferase reporter-NALM6 cells

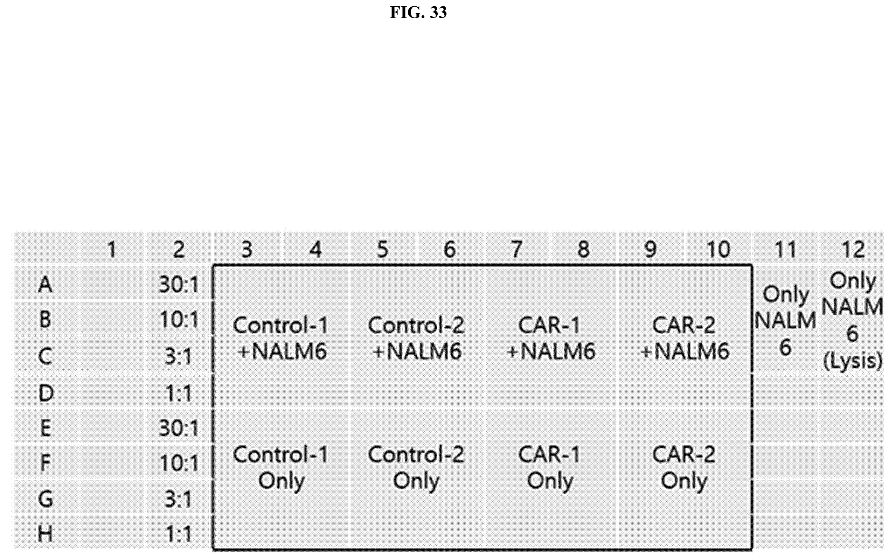

1) GFP-luciferase reporter-NALM6 cells cultured in a T75 flask were collected in a 50 mL tube.
2) Centrifugation was performed at room temperature and 1,500 rpm for five minutes.
3) The supernatant was removed, the cell pellet was suspended in 5 mL of RPMI medium, and cell counting was performed.
4) GFP-luciferase reporter-NALM6 cells were prepared by adding RPMI medium (10% FBS + 1% P/S) to 2.0 x 10⁴ cells/100 µL.
5) After counting the cells of the two types of rested CD19 CAR-T cells (cells prepared in 6. and cells prepared in 7.), the cells were prepared to have a concentration of 6.0 x 10⁵/100 µL.
6) As shown in FIG. 33, 100 µL of RPMI medium (10% FBS + 1% P/S) was placed in lanes B to D and F to H of two white 96-well flat plates in advance, and 150 µL (9.0 x 10⁵) of the CD19 CAR-T cells of 5) were placed in lanes A and E. Then, the PBMCs were serially diluted 3-fold using a multi-channel pipette so that the NALM6/PBMC ratios were 30:1, 10:1, 3:1, and 1:1 (plate 1: for transposon system; plate 2: for lentivirus system).
7) NALM6 cells prepared in 4) were added to lanes A to D at 100 µL each as shown in FIG. 33 and co-cultured at 200 µL/well.
8) 100 µL complete RPMI was added to the 'Only PBMC' group (lanes E to H) to adjust to 200 µL/well.
9) 100 µL of the 'Only NALM6' cells of 4) and 100 µL of complete RPMI were added to lanes 11 and 12 as shown in FIG. 33 to adjust to 200 µL/well.
10) For lysis, 20 µL of 10% Triton-X was added to lane 12 wells and mixed well.
11) Incubation was performed for four hours in a 37°C, 5% CO₂ incubator for reaction.
12) Bright-Glo reagent mixture (100 mL of Bright-Glo luciferase assay buffer + substrate) was dissolved in a required amount in a 37°C, 5% CO₂ incubator.
13) After suspending the mixture well in each co-culture well of the 96-well plate, 100 µL was removed.
14) Bright-Glo reagent was added to each well at 100 µL, each well was covered with aluminum foil, and the mixture was allowed to stand at room temperature for two minutes.
15) The 96-well plate was mounted on a spectrophotometer, and luminescence was measured.

### [Results]

### 1. pBat CD19 CAR plasmid cloning results

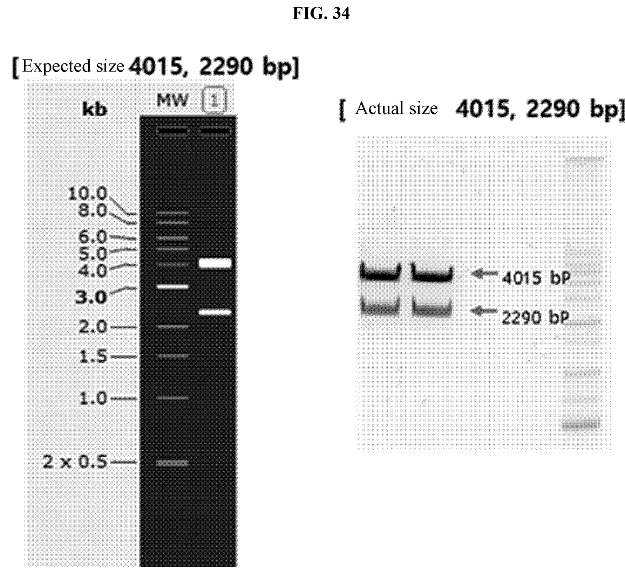

To clone the pBat CD19 CAR plasmid vector, BstBI and EcoR I were treated, and then the CD19 CAR gene to be used as an insert was cloned using Gibson assembly. In addition, to confirm whether the cloning was successful, enzyme mapping was performed using the restriction enzyme EcoR V as shown in FIG. 34, and then candidate clones were selected and finally identified through sequencing.

### 2. Confirmation of total number of cultured cells

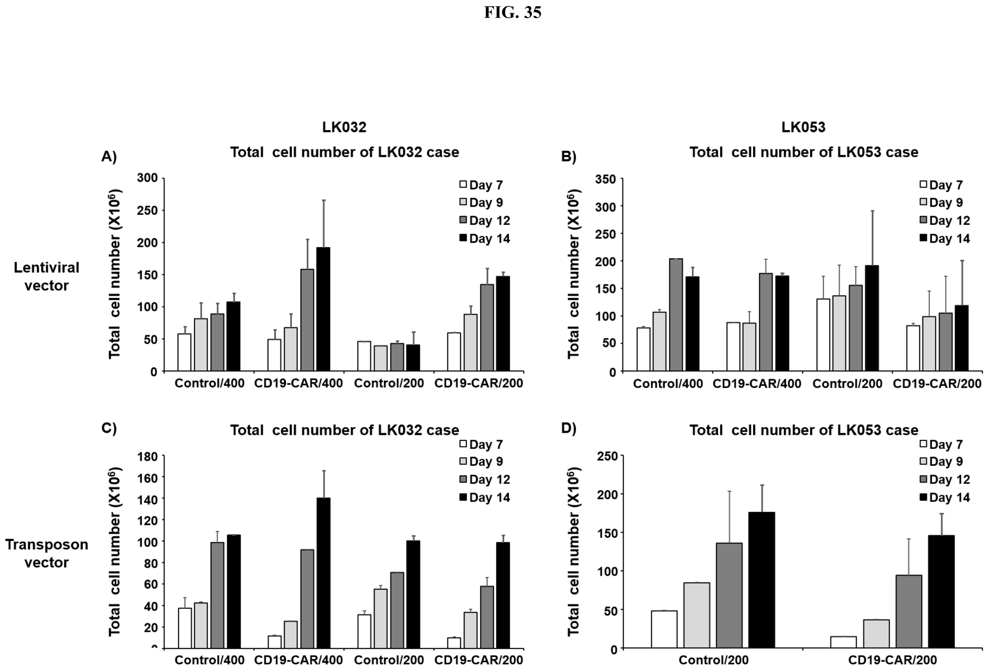

To produce CD19 CAR-T cells, genes were delivered to PBMCs by transduction and electroporation methods using a lentivirus and transposon, respectively, and cells were cultured for 7 and 14 days. Thereafter, the total number and the cell viability of cultured T cells were confirmed. Regarding the total number of cells, as shown in FIG. 35 and Table 19, it was confirmed that the viability due to electroporation on day 7 tended to decrease in the group treated with the transposon vector compared to the control group that was not treated with the vector in this experiment, but the cell proliferation rate increased over time.

**[Table 19]**

| Test group | LK032 (x 10⁶ cells) | | | | LK053 (x 10⁶ cells) | | | |
|---|---|---|---|---|---|---|---|---|
| | Day 7 | Day 9 | Day 12 | Day 14 | Day 7 | Day 9 | Day 12 | Day 14 |
| Activation only (200 IU/mL of IL-2) | 46.09 | 39.18 | 42.68 | 40.54 | 131.16 | 137.13 | 155.55 | 191.65 |
| Lentivirus_CD19-CAR (200 IU/mL of IL-2) | 59.63 | 88.35 | 134.66 | 147.52 | 82.45 | 99.17 | 105.02 | 118.96 |
| Activation only (400 IU/mL of IL-2) | 57.98 | 81.47 | 88.85 | 107.67 | 78.40 | 107.31 | 203.68 | 171.09 |
| Lentivirus_CD19-CAR (400 IU/mL of IL-2) | 49.32 | 67.68 | 158.27 | 192.57 | 88.23 | 87.29 | 177.04 | 172.71 |
| Electroporation only (200 IU/mL of IL-2) | 31.35 | 55.24 | 70.57 | 100.22 | 48.75 | 84.75 | 136.54 | 176.48 |
| pBat CD19-CAR(200 IU/mL of IL-2) | 9.90 | 33.62 | 57.68 | 98.59 | 14.90 | 36.63 | 94.63 | 146.23 |
| Electroporation only (400 IU/mL of IL-2) | 37.50 | 42.29 | 98.42 | 105.68 | - | - | - | - |
| pBat CD19-CAR (400 IU/mL of IL-2) | 11.60 | 25.25 | 91.64 | 140.05 | - | - | - | - |

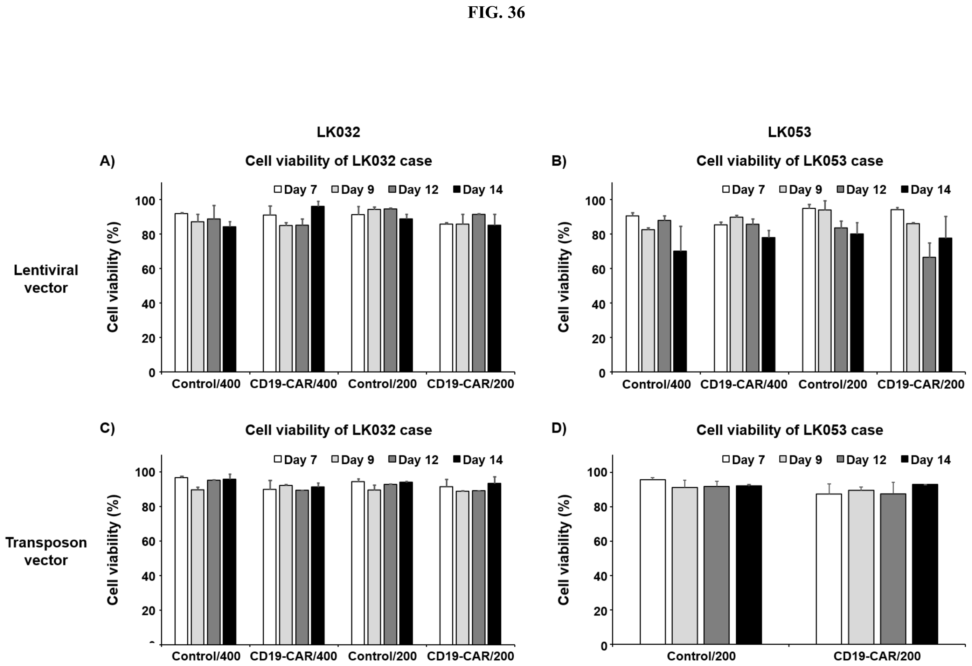

As shown in FIG. 36 and Table 20, on day 7, the cell viability of the lentivirus was 85.8% to 94.4%, and that of the transposon was 87.3% to 94.4%, and on day 14, the cell viability of the lentivirus was 77.7% to 96.2%, and that of the transposon was 91.4% to 93.4%, confirming that the viability of T cells using a lentivirus vector was slightly lower than that of T cells produced using a transposon vector.

**[Table 20]**

| Test group | LK032 (%) | | | | LK053 (%) | | | |
|---|---|---|---|---|---|---|---|---|
| | Day 7 | Day 9 | Day 12 | Day 14 | Day 7 | Day 9 | Day 12 | Day 14 |
| Activation only (200 IU/mL of IL-2) | 91.34 | 94.44 | 94.75 | 88.88 | 95.12 | 94.06 | 83.58 | 80.15 |
| Lentivirus_CD19-CAR (200 IU/mL of IL-2) | 85.80 | 85.85 | 91.56 | 85.18 | 94.38 | 86.12 | 66.53 | 77.70 |
| Activation only (400 IU/mL of IL-2) | 91.94 | 87.29 | 88.89 | 84.31 | 90.74 | 82.58 | 87.94 | 70.18 |
| Lentivirus_CD19-CAR (400 IU/mL of IL-2) | 91.10 | 85.10 | 85.27 | 96.18 | 85.55 | 89.88 | 85.69 | 78.02 |
| Electroporation only (200 IU/mL of IL-2) | 94.48 | 89.69 | 92.74 | 94.06 | 95.60 | 90396 | 91.85 | 92.02 |
| pBat CD19-CAR(200 IU/mL of IL-2) | 91.55 | 89.00 | 89.16 | 93.35 | 87.26 | 89.33 | 87.53 | 92.82 |
| Electroporation only (400 IU/mL of IL-2) | 96.80 | 89.79 | 95.14 | 95.79 | - | - | - | - |
| pBat CD19-CAR (400 IU/mL of IL-2) | 89.95 | 92.36 | 89.41 | 91.35 | - | - | - | - |

### 4. Comparison of CD19 CAR protein expression according to gene delivery vector

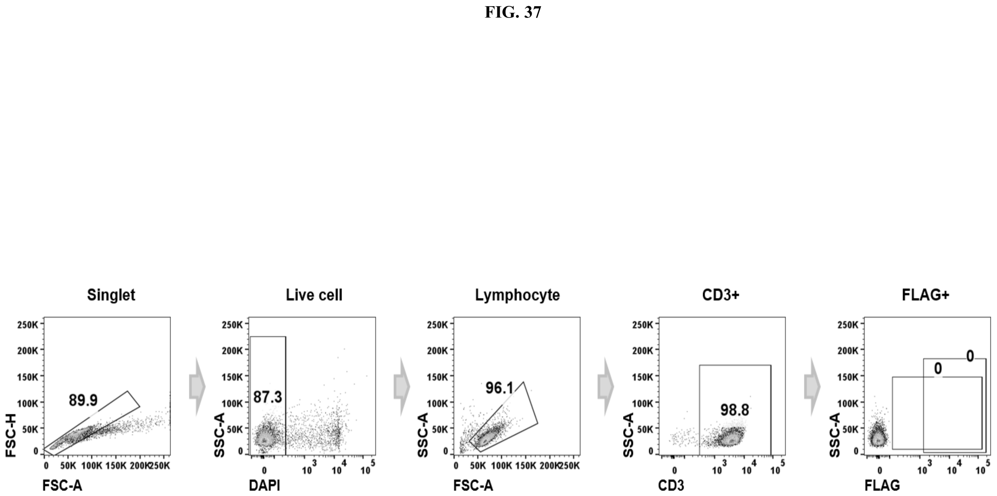

Expression of CD19 CAR was confirmed by FACS using an anti-FLAG tag antibody with the FLAG tag protein expressed by the FLAG tag gene located between a leader sequence and CD19scFv in the CD19 CAR gene. As shown in FIG. 37, the analysis method was performed by gating in the order of singlets, live cells, lymphocytes, CD3+ T cells, and FLAG+ T cells to confirm CD19 CAR gene delivery into CD3+ T cells and expression efficiency.

### 4-1. LK032 PBMCs

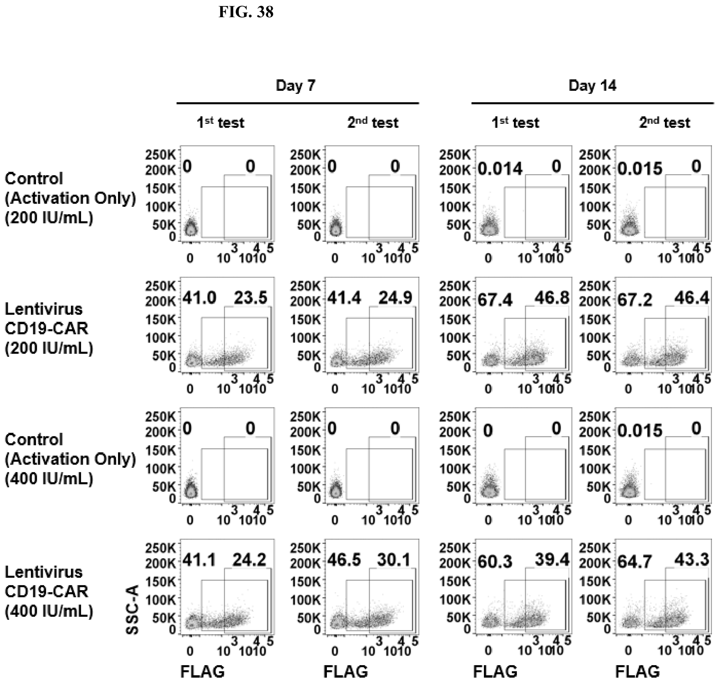

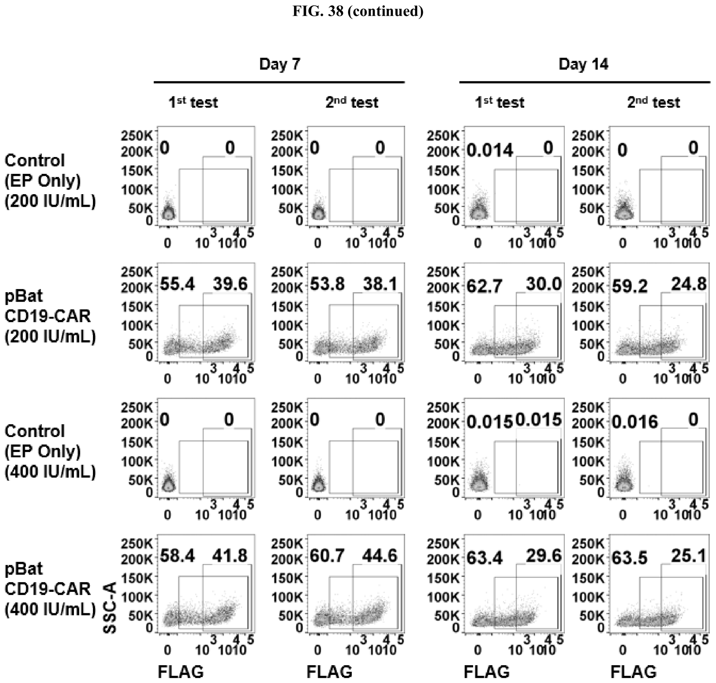

After transformation of LK032 PBMCs with the CD19-CAR gene using lentiviral and transposon vectors, the proportion of cells expressing FLAG (CD19-CAR) among CD3+ T cells was confirmed on day 7 of culture. As a result, as shown in FIG. 38 and Table 21, it was confirmed that FLAG (CD19-CAR) was not expressed in the negative control group "activation only group" in which only activation was performed with each vector and in the negative control group "EP only group" in which only electroporation was performed. In the groups cultured for seven days by adding 200 IU/mL IL-2 after transformation with each vector, the proportion of cells expressing FLAG (CD19-CAR) was 41.0% and 54.6% for the lentivirus and transposon vectors, respectively, indicating that the proportion was about 14% higher in the cells using the transposon vector. In the groups cultured by adding 400 IU/mL IL-2, the proportion was 43.4% and 59.6%, indicating that the proportion was about 15% higher in the cells using the transposon vector. In addition, after 14 days of culture, the proportion of cells expressing FLAG (CD19-CAR) in the groups cultured by adding 200 IU/mL IL-2 was 67.30% and 61.0%, indicating that the proportion was slightly lower for the transposon vector, but in the group cultured by adding 400 IU/mL IL-2, the proportion was 62.5% and 63.5%, which were almost similar to each other. On the other hand, it was confirmed that the proportion of cells expressing FLAG at high intensity among cells expressing FLAG was high for the transposon vector on day 7, whereas it was high for the lentiviral vector on day 14.

**[Table 21]**

| Test group | LK032 | | | |
|---|---|---|---|---|
| | Day 7 | | Day 14 | |
| | Total FLAG | High intensity FLAG | Total FLAG | High intensity FLAG |
| Activation only (200 IU/mL of IL-2) | 0% | 0% | 0% | 0% |
| Lentivirus _CD 19-CAR (200 IU/mL of IL-2) | 41.0% | 24.2% | 67.3% | 46.6% |
| Activation only (400 IU/mL of IL-2) | 0% | 0% | 0% | 0% |
| Lentivirus _CD19-CAR (400 IU/mL of IL-2) | 43.8% | 28.3% | 62.5% | 41.4% |
| Electroporation only (200 IU/mL of IL-2) | 0% | 0% | 0% | 0% |
| pBat CD19-CAR (200 IU/mL of IL-2) | 54.6% | 38.9% | 61.0% | 27.4% |
| Electroporation only (400 IU/mL of IL-2) | 0% | 0% | 0% | 0% |
| pBat CD19-CAR (400 IU/mL of IL-2) | 59.6% | 43.2% | 63.5% | 27.4% |

### 4-2. LK053 PBMCs

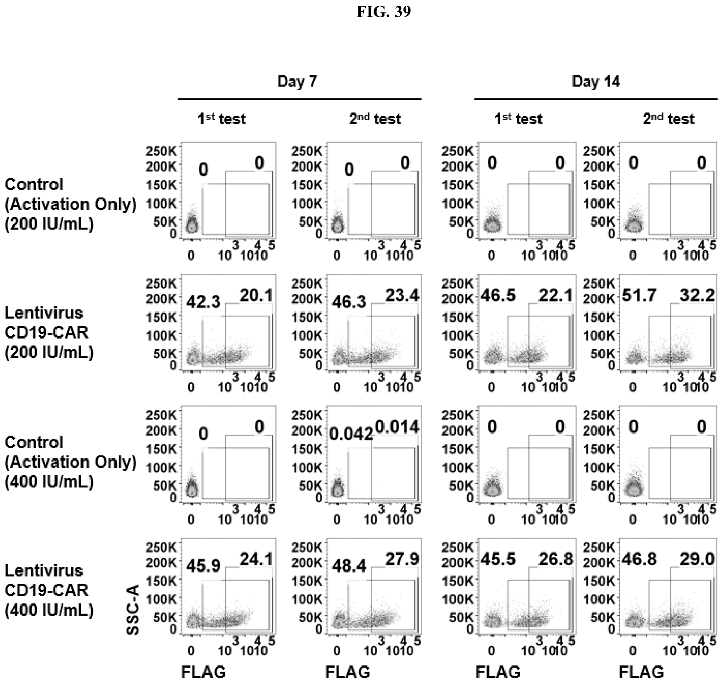

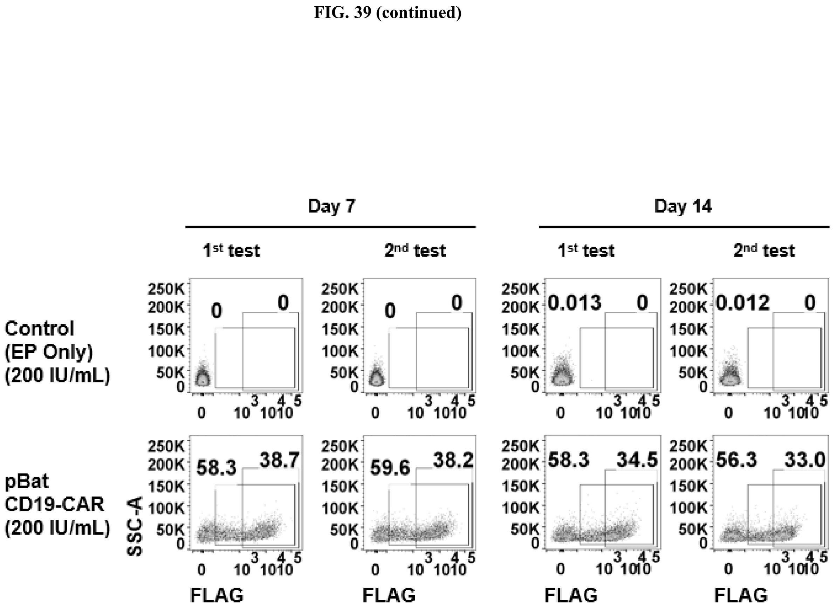

After transformation of LK053 PBMCs with the CD19 CAR gene using lentivirus and transposon vectors, the proportion of cells expressing FLAG (CD19 CAR) among CD3+ T cells was confirmed on day 7 of culture. As a result, as shown in FIG. 39 and Table 22, it was confirmed that FLAG (CD19 CAR) was not expressed in the negative control group "activation only group" in which only activation was performed with each vector and the negative control group "EP only group" in which only electroporation was performed. In the groups cultured for seven days by adding 200 IU/mL IL-2 after transformation, the proportion of cells expressing FLAG (CD19 CAR) was 44.3% and 59.0% for the lentivirus and transposon vectors, respectively, indicating that the proportion was about 15% higher in the cells using the transposon vector. In addition, after 14 days of culture, the proportion of cells expressing FLAG (CD19 CAR) in the groups cultured by adding 200 IU/mL IL-2 was 49.1% and 57.3%, indicating that the proportion was about 8% higher in the cells using the transposon vector. In addition, it was confirmed that unlike the LK032 case, the proportion of cells expressing FLAG at high intensity among the cells expressing FLAG was high for the transposon vector on both days 7 and 14.

**[Table 22]**

| Test group | LK053 | | | |
|---|---|---|---|---|
| | Day 7 | | Day 14 | |
| | Total FLAG | High intensity FLAG | Total FLAG | High intensity FLAG |
| Activation only (200 IU/mL of IL-2) | 0% | 0% | 0% | 0% |
| Lentivirus _CD19-CAR (200 IU/mL of IL-2) | 44.3% | 21.8% | 49.1% | 27.2% |
| Activation only (400 IU/mL of IL-2) | 0% | 0% | 0% | 0% |
| Lentivirus _CD 19-CAR (400 IU/mL of IL-2) | 47.2% | 26.0% | 46.2% | 27.9% |
| Electroporation only (200 IU/mL of IL-2) | 0% | 0% | 0% | 0% |
| pBat CD19-CAR (200 IU/mL of IL-2) | 59.0% | 38.5% | 57.3% | 33.8% |
| Electroporation only (400 IU/mL of IL-2) | - | - | - | - |
| pBat CD19-CAR (400 IU/mL of IL-2) | - | - | - | - |

### 5. Confirmation of memory T cell proportion according to gene delivery vector

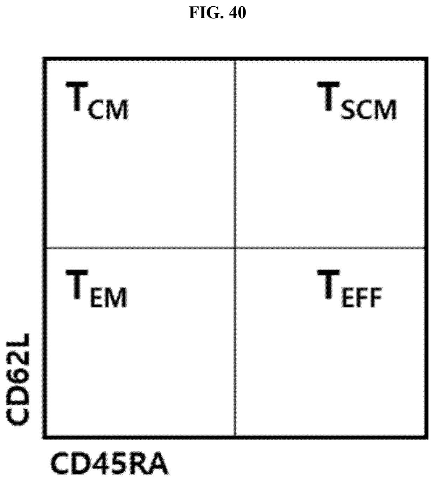

The proportion of T cell memory types among the T cells (CD3+ cells) transformed with the two vectors was analyzed using CD45RA and CD62L markers. When memory type T cells are distinguished using CD45RA and CD62L markers, CD45RA+CD62L- T cells are classified as T_{EFF} (effector T cells), CD45RA-CD62L+ T cells are classified as T_{EM} (effect memory T cells), CD45RA-CD62L+ T cells are classified as T_{CM} (central memory T cells), and CD45RA+CD62L+ T cells are classified as T_{SCM} (stem cell like memory T cells). The position of each memory type T cell in the quadrant plot is as shown in FIG 40.

### 5-1. LK032 PBMCs

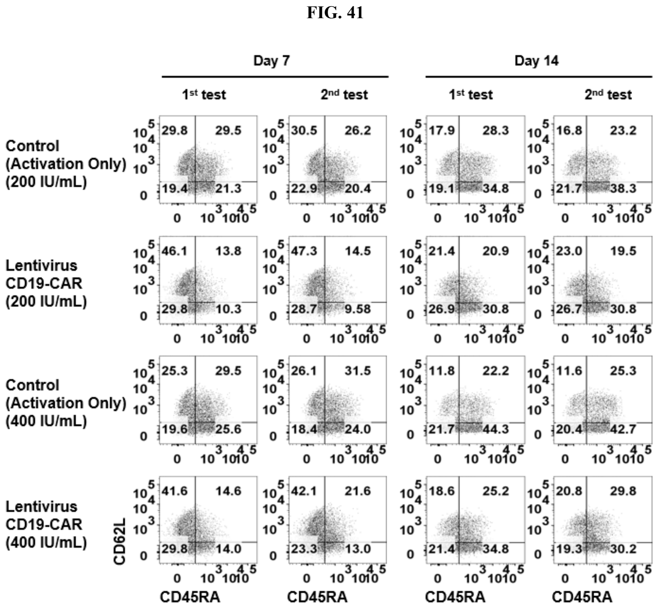

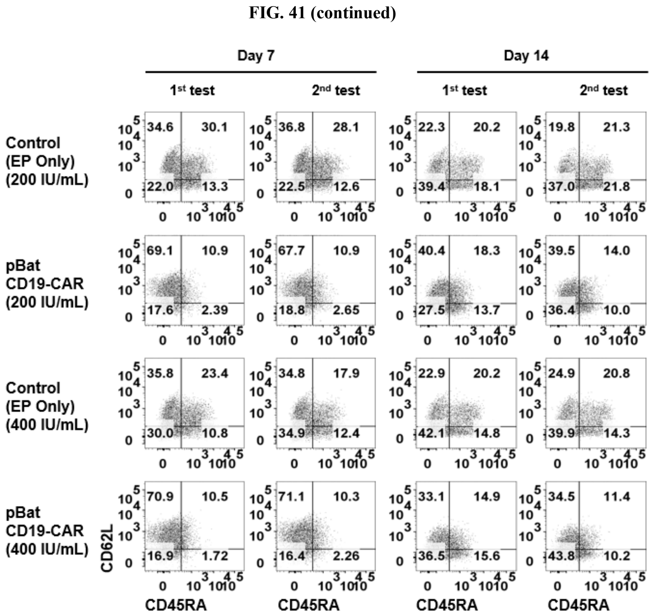

After transformation of LK032 PBMCs with the CD19 CAR gene using the lentivirus vector and transposon vector, the proportion of T cell memory types among the total cultured cells was confirmed on days 7 and 14. As a result, as shown in FIG. 41 and Table 23, the sum of the proportions of T_{SCM} and T_{CM} was similarly confirmed to be 56% to 65% on average in the negative control group "activation only group" in which only activation was performed with each vector and the negative control group "EP only group" in which only electroporation was performed. In the case of the lentivirus vector, the sum of the proportions of T_{SCM} and T_{CM} was confirmed to be approximately 61% in all groups cultured by adding 200 IU/mL and 400 IU/mL IL-2 on day 7 after transformation, whereas in the case of the transposon vector, the sum of the proportions of T_{SCM} and T_{CM} was confirmed to be approximately 80% to 82% in all groups cultured by adding 200 IU/mL and 400 IU/mL IL-2, which was about 20% higher than that of the groups using lentivirus vectors. In addition, on day 14, in the case of the lentivirus vector, the sum of the proportions of T_{SCM} and T_{CM} was confirmed to be approximately 43% to 48%, while in the case of the transposon vector, the sum of the proportions of T_{SCM} and T_{CM} was confirmed to be slightly higher as 47% to 56%, indicating that the overall proportion of T cells of T_{SCM} and T_{CM} memory types was high in the groups using the transposon vector.

**[Table 23]**

| Test group | LK032 | | | | | | | |
|---|---|---|---|---|---|---|---|---|
| | Day 7 | | | | Day 14 | | | |
| | T_{SCM} | T_{CM} | T_{EM} | T_{EFF} | T_{SCM} | T_{CM} | T_{EM} | T_{EFF} |
| Activation only (200 IU/mL of IL-2) | 28% | 31% | 21% | 21% | 26% | 18% | 21% | 37% |
| Lentivirus _CD 19-CAR (200 IU/mL of IL-2) | 15% | 47% | 30% | 10% | 21% | 22% | 27% | 31% |
| Activation only (400 IU/mL of IL-2) | 31% | 26% | 19% | 25% | 24% | 12% | 21% | 44% |
| Lentivirus_CD19-CAR(400 IU/mL of IL-2) | 19% | 42% | 27% | 14% | 28% | 20% | 20% | 33% |
| Electroporation only (200 IU/mL of IL-2) | 29% | 36% | 23% | 13% | 21% | 21% | 38% | 20% |
| pBat CD19-CAR (200 IU/mL of IL-2) | 11% | 69% | 19% | 3% | 16% | 40% | 32% | 12% |
| Electroporation only(400 IU/mL of IL-2) | 21% | 36% | 33% | 12% | 21% | 24% | 41% | 15% |
| pBat CD19-CAR (400 IU/mL of IL-2) | 11% | 71% | 17% | 2% | 13% | 34% | 41% | 13% |

### 5-2. LK053 PBMCs

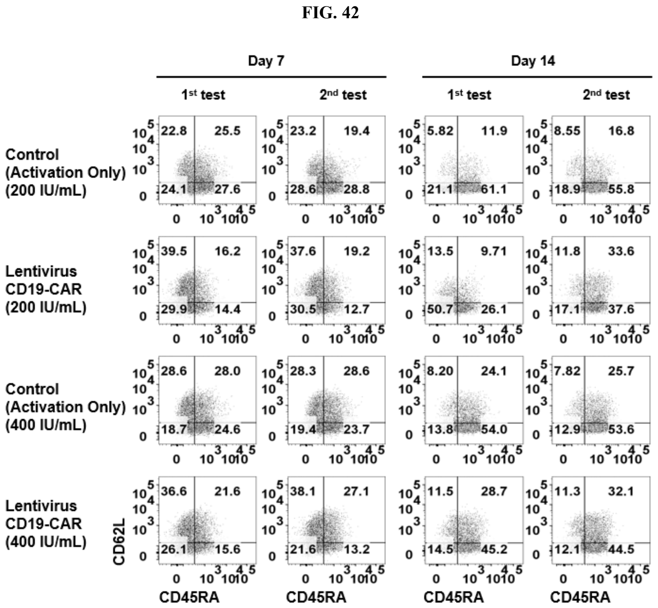

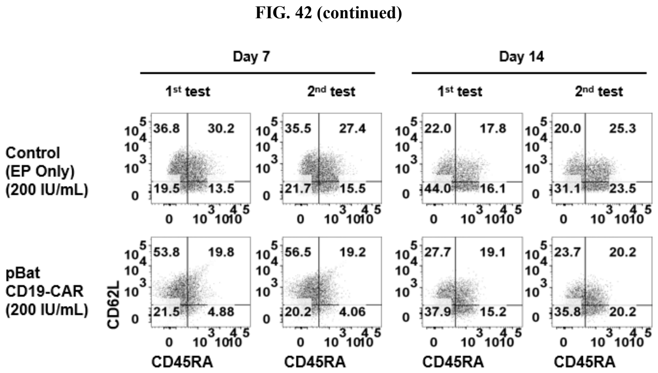

After the transformation of LK053 PBMCs with the CD19 CAR gene using the lentivirus vector and transposon vector, the proportion of T cell memory types among the total cultured cells was confirmed on days 7 and 14. As a result, as shown in FIG. 42 and Table 24, on day 7, the sum of the proportions of T_{SCM} and T_{CM} was approximately 46% to 57% in the negative control group of the lentivirus vector and approximately 65% in the negative control group of the transposon vector, confirming that confirming that the proportion of T cells of T_{SCM} and T_{CM} memory types was high for the transposon vector. In addition, the sum of the proportions of T_{SCM} and T_{CM} in the negative control groups on day 14 was approximately 22% to 33% in the case of the lentivirus vector and 43% in the case of the transposon vector, confirming that the proportion of T cells of T_{SCM} and T_{CM} memory types was high for the transposon vector. In addition, in the case of the lentivirus vector among the test groups, on day 7, the proportion of T_{SCM} and T_{CM} was confirmed to be about 57% to 62% in all groups cultured by adding 200 IU/mL and 400 IU/mL IL-2, whereas in the case of the transposon vector, the proportion was confirmed to be about 75% in the group cultured by adding 200 IU/mL IL-2, which was about 15% higher than the groups using the lentivirus vector. In addition, on day 14, the proportion was confirmed to be about 35% to 42% in the case of the lentivirus vector and 46% in the case of the transposon vector, which was slightly higher than that of the groups using the lentivirus vector, indicating that the overall proportion of T cells of T_{SCM} and T_{CM} memory types was high in the groups using the transposon vector.

**[Table 24]**

| Test group | LK053 | | | | | | | |
|---|---|---|---|---|---|---|---|---|
| | Day 7 | | | | Day 14 | | | |
| | T_{SCM} | T_{CM} | T_{EM} | T_{EFF} | T_{SCM} | T_{CM} | T_{EM} | T_{EFF} |
| Activation only (200 IU/mL of IL-2) | 23% | 23% | 27% | 29% | 15% | 8% | 20% | 58% |
| Lentivirus_CD19-CAR (200 IU/mL of IL-2) | 18% | 39% | 31% | 14% | 22% | 13% | 34% | 32% |
| Activation only (400 IU/mL of IL-2) | 29% | 29% | 19% | 25% | 25% | 8% | 14% | 54% |
| Lentivirus _CD19-CAR(400 IU/mL of IL-2) | 25% | 38% | 24% | 15% | 31% | 12% | 13% | 45% |
| Electroporation only (200 IU/mL of IL-2) | 29% | 36% | 21% | 15% | 22% | 21% | 38% | 20% |
| pBat CD19-CAR (200 IU/mL of IL-2) | 20% | 56% | 21% | 5% | 20% | 26% | 37% | 18% |

### 6. Confirmation of memory T cells among T cells expressing FLAG (CD19 CAR)

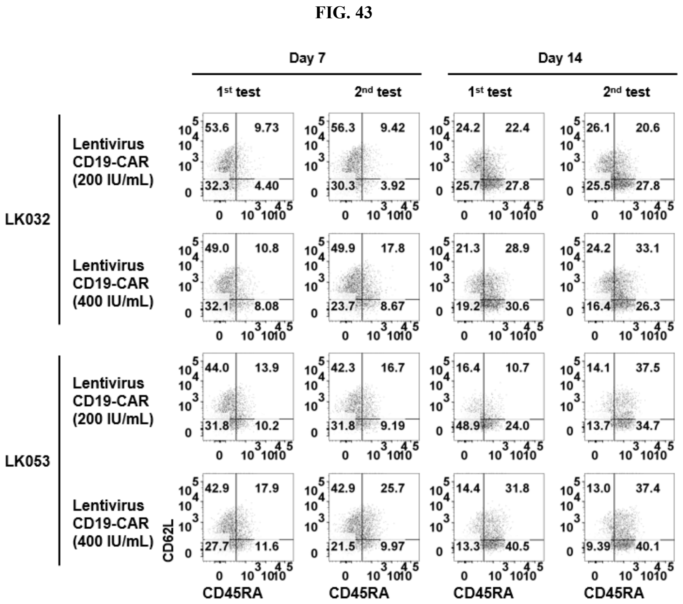

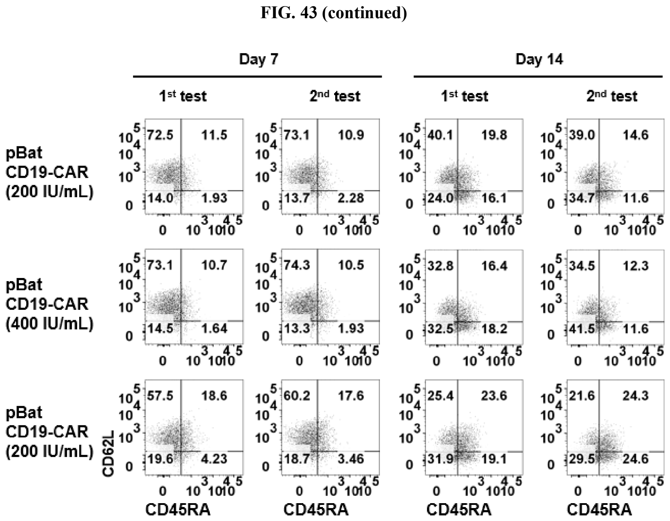

The memory type of T cells transformed with the two delivery systems and expressing FLAG (CD19 CAR) was confirmed. As a result, as shown in FIG. 43 and Table 25, in the case of the lentivirus vector, the sum of the proportions of T_{SCM} and T_{CM} on day 7 was approximately 59% to 65% in both LK032 PBMCs and LK053 PBMCs, whereas in the case of the transposon vector, it was approximately 78% to 85%, which was approximately 20% higher than that of the lentivirus vector. However, on day 14, in the case of the lentivirus vector, the sum of the proportions of T_{SCM} and T_{CM} was approximately 40% to 54% in both LK053 and LK032, whereas in the case of the transposon vector, it was approximately 48% to 57%, confirming that the proportion was similar for the two vectors.

**[Table 25]**

| Case | Test group | Day 7 | | | | Day 14 | | | |
|---|---|---|---|---|---|---|---|---|---|
| | | T_{SCM} | T_{CM} | T_{EM} | T_{EFF} | T_{SCM} | T_{CM} | T_{EM} | T_{EFF} |
| LK032 | Lentivirus CD19-CAR (200 IU/mL of IL-2) | 10% | 55% | 31% | 4% | 22% | 25% | 26% | 28% |
| | Lentivirus CD19-CAR (400 IU/mL of IL-2) | 15% | 50% | 28% | 9% | 31% | 23% | 18% | 29% |
| | pBat CD19-CAR (200 IU/mL of IL-2) | 12% | 73% | 14% | 2% | 18% | 40% | 30% | 14% |
| | pBat CD19-CAR (400 IU/mL of IL-2) | 11% | 74% | 14% | 2% | 14% | 34% | 38% | 15% |
| LK053 | Lentivirus CD19-CAR (200 IU/mL of IL-2) | 16% | 43% | 32% | 10% | 25% | 15% | 32% | 30% |
| | Lentivirus CD19-CAR (400 IU/mL of IL-2) | 22% | 43% | 25% | 11% | 35% | 14% | 11% | 41% |
| | pBat CD19-CAR (200 IU/mL of IL-2) | 19% | 59% | 20% | 4% | 24% | 24% | 31% | 22% |

### 7. Confirmation of proportion of CD4 and CD8 in T cells expressing FLAG (CD19 CAR)

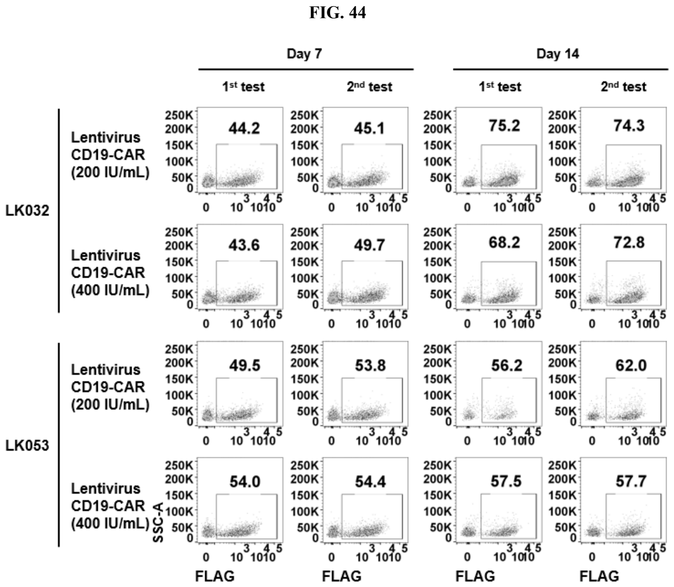

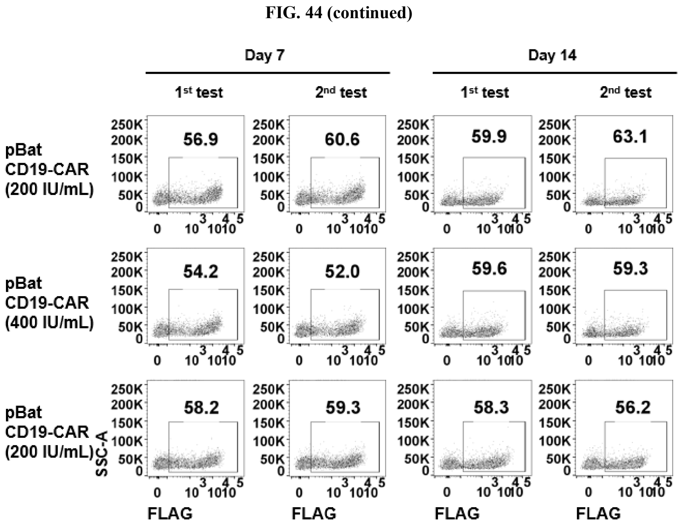

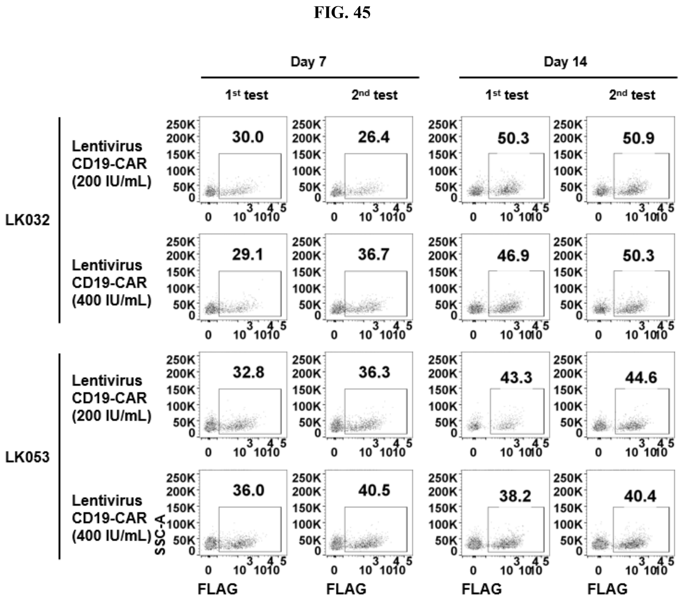

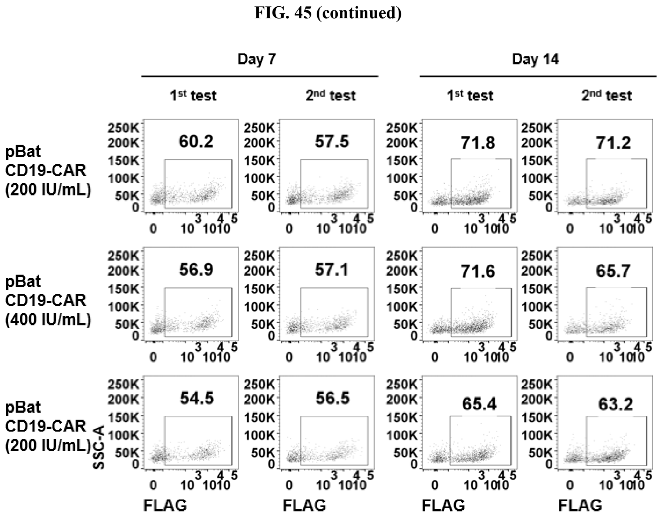

To confirm the proportion of CD4 and CD8 in T cells expressing FLAG (CD19-CAR) transformed with two delivery systems, the proportion was confirmed on days 7 and 14 after transformation. As a result, as shown in FIGs. 44 and 45, and Table 26, it was confirmed that in the case of the lentivirus vector, the proportions of CD8+ and CD4+ on day 7 after transformation were 28% to 38% and 45% to 54%, respectively, in all groups cultured by adding 200 IU/mL and 400 IU/mL IL-2, and in the case of the transposon vector, the proportions of CD8+ and CD4+ were 56% to 59% and 53% to 59%, respectively, confirming that the proportion of CD8 was 5% to 10% higher than that of the lentivirus. In addition, it was confirmed that in the case of the lentivirus vector, the proportions of CD8+ and CD4+ on day 14 were approximately 39% to 51% and 58% to 75%, respectively, and in the case of the transposon vector, the proportions of CD8+ and CD4+ were approximately 64% to 72% and 57% to 61%, respectively, indicating that the proportion of CD8+ was about 20% to 25% higher than that of the lentivirus.

**[Table 26]**

| Case | Test group | Day 7 | | Day14 | |
|---|---|---|---|---|---|
| | | CD4+ | CD8+⁺ | CD4+ | CD8+ |
| LK032 | Lentivirus_CD19-CAR (200 IU/mL of IL-2) | 45% | 28% | 75% | 51% |
| | Lentivirus_CD19-CAR (400 IU/mL of IL-2) | 47% | 33% | 71% | 49% |
| | pBat CD19-CAR (200 IU/mL of IL-2) | 59% | 59% | 61% | 72% |
| | pBat CD19-CAR (400 IU/mL of IL-2) | 53% | 57% | 59% | 69% |
| LK053 | Lentivirus_CD19-CAR (200 IU/mL of IL-2) | 52% | 35% | 59% | 44% |
| | Lentivirus_CD19-CAR (400 IU/mL of IL-2) | 54% | 38% | 58% | 39% |
| | pBat CD19-CAR (200 IU/mL of IL-2) | 59% | 56% | 57% | 64% |

### 8. Results of in vitro killing assay

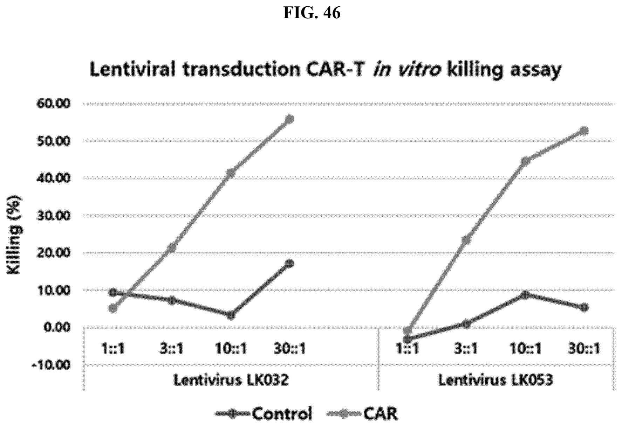

For an *in vitro* killing test of CAR-T cells cultured for 14 days, co-culture was performed with GFP-luciferase reporter-NALM6 cells at ratios of 30:1, 10:1, 3:1, and 1:1 (effector cells: target cells), and the luciferase detected when the target cells were killed was converted into percentage (%) values to calculate the luciferase assay expression rate. As a result, as shown in FIG. 46 and Table 27, in the case of the lentivirus vector, the luciferase assay expression rate (%) measured for the "No Transduction (negative control) group" that did not express CD19 CAR was approximately 5.3% to 17.2%, 3.4% to 8.9%, 1.0% to 7.4%, and 0% to 9.3% under the ratio conditions of 30:1, 10:1, 3:1, and 1:1, respectively, whereas the luciferase assay expression rate (%) of the group transduced with the lentivirus was 52.8% to 55.9%, 41.5% to 44.6%, 21.4% to 23.6%, and 0% to 5.2% under the ratio conditions of 30:1, 10:1, 3:1, and 1:1, respectively, confirming a dose-dependent increase in CD19 CAR-T cells.

**[Table 27]**

| Effector : Target ratio | Lentivirus | | Negative control | |
|---|---|---|---|---|
| | LK032 | LK053 | LK032 | LK053 |
| 30:1 | 55.9 | 52.8 | 17.2 | 5.3 |
| 10:1 | 41.5 | 44.6 | 3.4 | 8.9 |
| 3:1 | 21.4 | 23.6 | 7.4 | 1.0 |
| 1:1 | 5.2 | -1.0 | 9.3 | -3.2 |

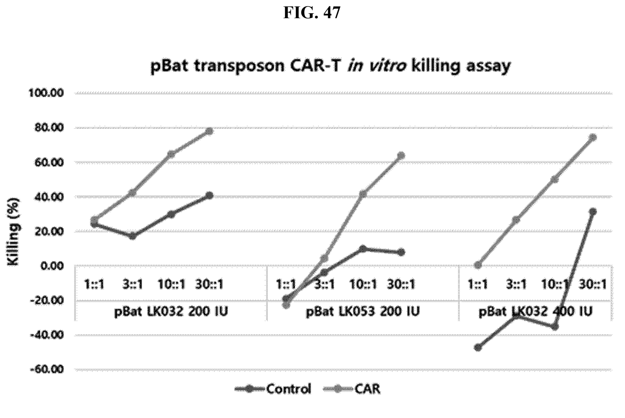

In addition, in the case of the transposon vector, as shown in FIG. 47 and Table 28, the luciferase assay expression rate (%) measured for the "No E/P (negative control) group" that did not express CD19 CAR was 7.8% to 40.8%, 9.9% to 29.9%, 0% to 17.3%, and 0% to 24.3% under the ratio conditions of 30:1, 10:1, 3:1, and 1:1, respectively, whereas in the group transfected with the transposon, the luciferase assay expression rates (%) was 63.8% to 78.1%, 41.5% to 64.7%, 4.5% to 42.5%, and 0% to 26.6% under the ratio conditions of 30:1, 10:1, 3:1, and 1:1, respectively, confirming a dose-dependent increase in CD19 CAR-T cells. In conclusion, it was confirmed that CD19 CAR-T cells produced with the transposon vector exhibited approximately 20% higher *in vitro* killing activity against actual target cells than CD19 CAR-T cells produced with the lentivirus, under all conditions.

**[Table 28]**

| Effector : Target ratio | Transposon | | Negative control | |
|---|---|---|---|---|
| | LK032 | LK053 | LK032 | LK053 |
| 30:1 | 78.1 | 63.8 | 40.8 | 7.8 |
| 10:1 | 64.7 | 41.5 | 29.9 | 9.9 |
| 3:1 | 42.5 | 4.5 | 17.3 | -3.6 |
| 1:1 | 26.6 | -22.6 | 24.3 | -18.9 |

As discussed above, by comparing the gene delivery efficiency and the proportion of CD19 CAR expressing cells according to the culture period between the third-generation vector lentivirus, which is a widely used viral vector with proven stability due to the application the self-inactivating (SIN) vector system, and the non-viral vector pBat transposon vector, the performance of the pBat transposon vector was confirmed. As a result, when PBMCs transformed with the CD19 CAR gene using the lentiviral vector and transposon vector were cultured for 14 days, it was confirmed that the cell viability was somewhat lower in T cells using the lentiviral vector than in T cells produced using the transposon vector. In addition, it was confirmed that the proportion of cells expressing FLAG (CD19 CAR) among CD3+ T cells on day 7 of culture was approximately 15% higher in cells using the transposon vector in both LK032 PBMCs and LK053 PBMCs, and on day 14, the FLAG (CD19-CAR) expression rate in the group using the transposon vector was similar to or slightly higher than that of the group using the lentivirus vector. In addition, as a result of analyzing the proportion of memory type T cells among T cells (CD3+ and FLAG+ T cells) transformed with the two vectors, using CD45RA and CD62L markers, it was confirmed that the proportion of T cells of T_{SCM} and T_{CM} memory types was higher in both LK032 PBMCs and LK053 PBMCs using the transposon vector. As a result of confirming the proportion of CD4 and CD8 T cells among T cells expressing FLAG (CD19 CAR), the proportion of CD8+ T cells was about 5% to 10% higher for the transposon vector than in the lentivirus on day 7 and about 20% to 25% higher on day 14. Finally, as a result of confirming the *in vitro* killing activity of CAR-T cells cultured for 14 days, it was confirmed that the T cells produced with the transposon vector exhibited about 20% higher *in vitro* killing activity against actual target cells under all conditions than the T cells produced with the lentivirus vector. From the above-described results, it was determined that the pBat transposon vector could be applied as a gene delivery vector that can replace the lentiviral vector.

The above description of the present invention is for illustrative purposes only, and those skilled in the art will understand that the present invention may be easily modified into other specific forms without changing the technical idea or essential characteristics of the present invention. Therefore, it should be understood that the above-described embodiments are exemplary in all respects and not restrictive.

In Table 29 below, the bold part in SEQ ID NO: 14 is the sequence of the SEQ ID NO: 2; the bold part in SEQ ID NO: 15 is the sequence of the SEQ ID NO: 3; the underlined part in SEQ ID NO: 16 is the sequence of the SEQ ID NO: 9, and the bold part is the sequence of SEQ ID NO: 2; the underlined part in SEQ ID NO: 17 is the sequence of the SEQ ID NO: 9, and the bold part is the sequence of SEQ ID NO: 2; the underlined part in SEQ ID NO: 18 is the sequence of the SEQ ID NO: 11, and the bold part is the sequence of SEQ ID NO: 2; the underlined part in SEQ ID NO: 19 is the sequence of the SEQ ID NO: 11, and the bold part is the sequence of SEQ ID NO: 2; the underlined part in SEQ ID NO: 20 is the sequence of the SEQ ID NO: 9, and the bold part is the sequence of SEQ ID NO: 2; the underlined part in SEQ ID NO: 21 is the sequence of the SEQ ID NO: 9, and the bold part is the sequence of SEQ ID NO: 2; the underlined part in SEQ ID NO: 22 is the sequence of the SEQ ID NO: 11, and the bold part is the sequence of SEQ ID NO: 2; and the underlined part in SEQ ID NO: 23 is the sequence of the SEQ ID NO: 11, and the bold part is the sequence of SEQ ID NO: 2.

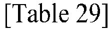

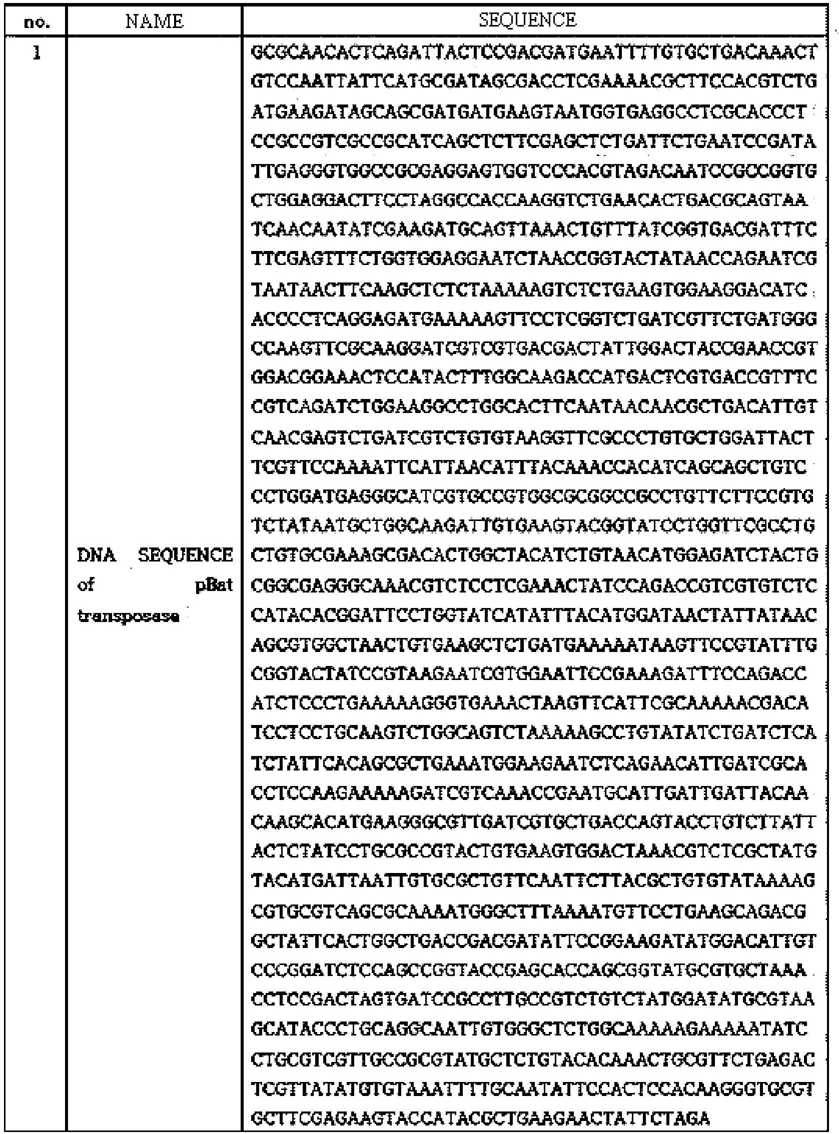

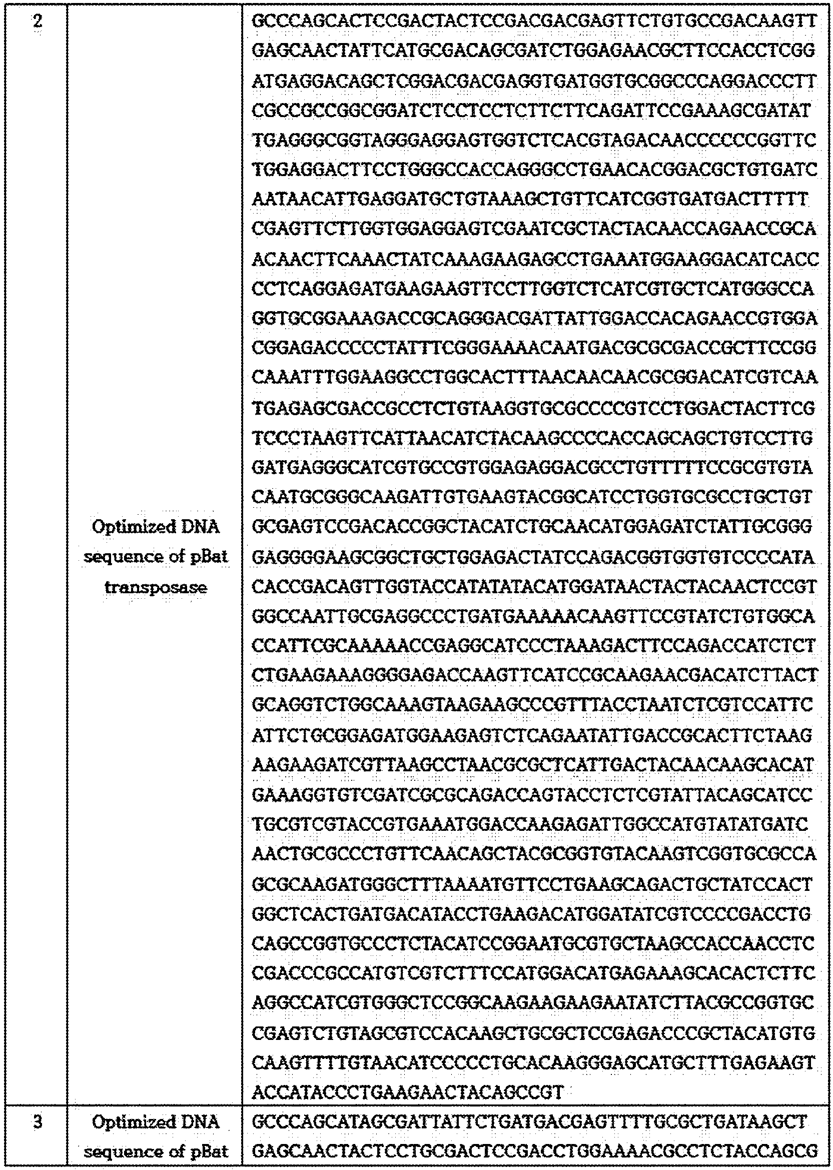

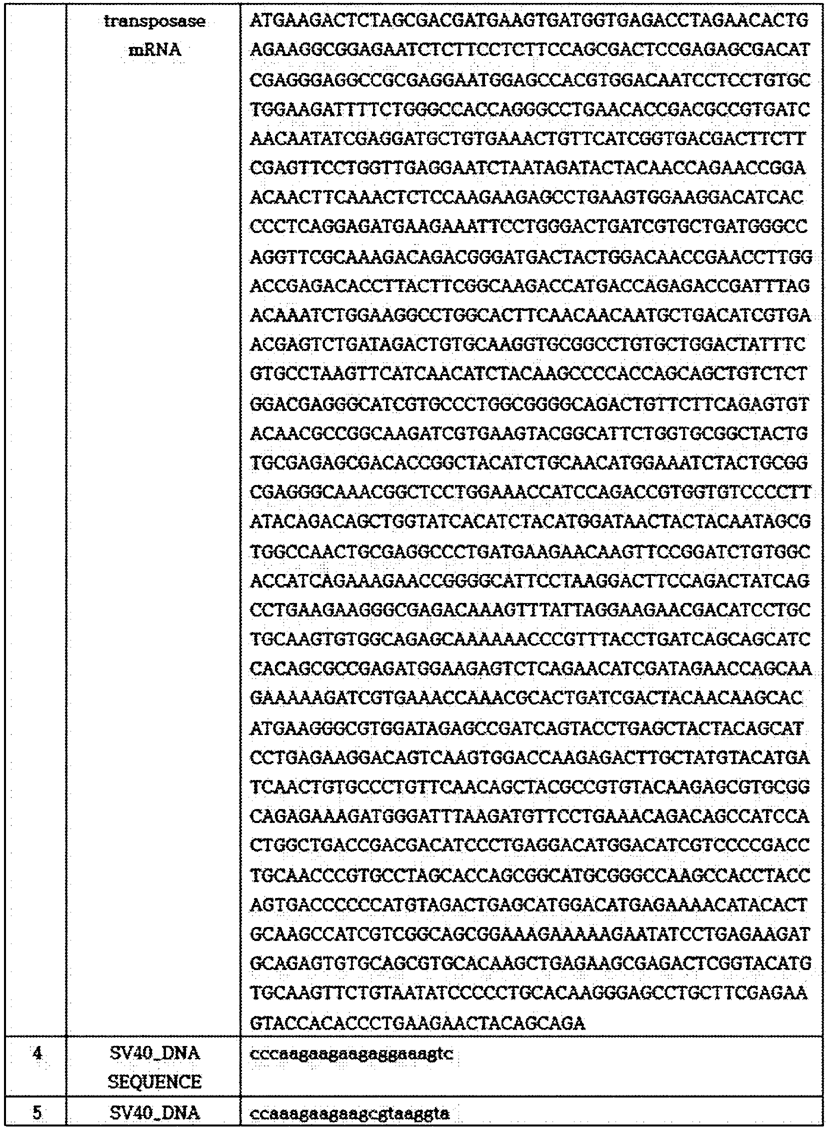

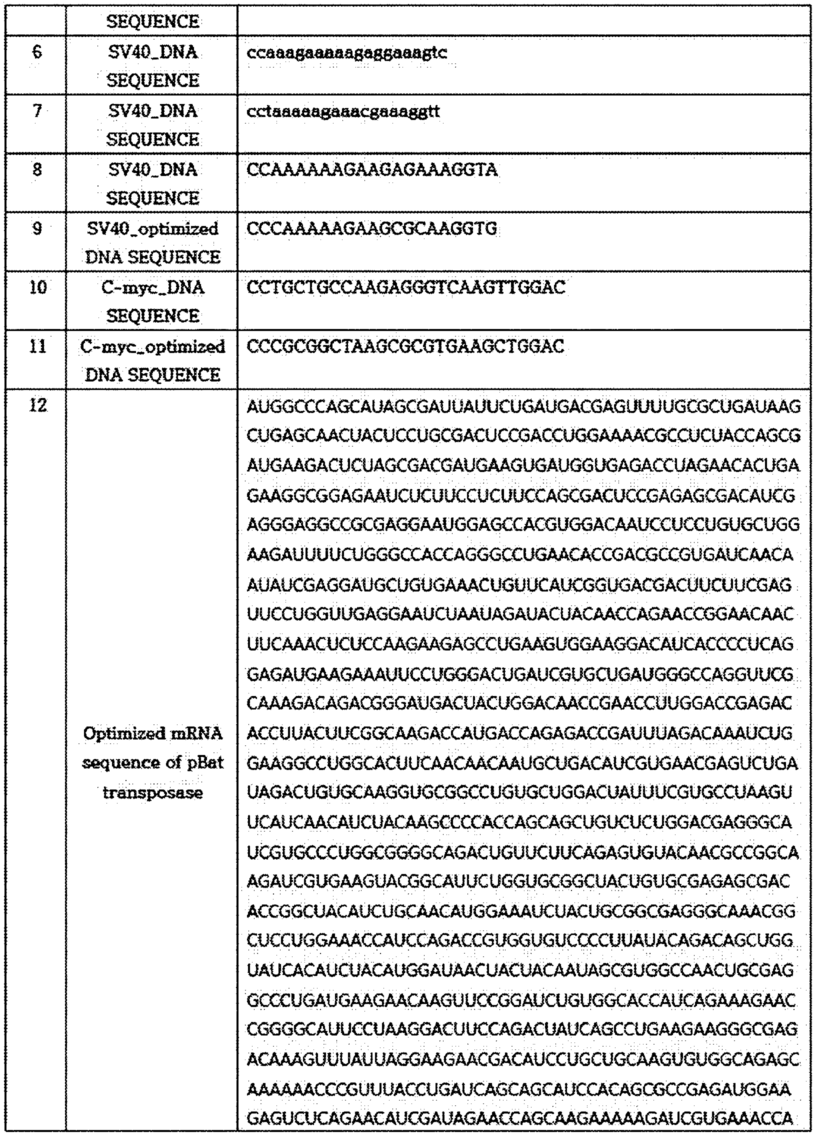

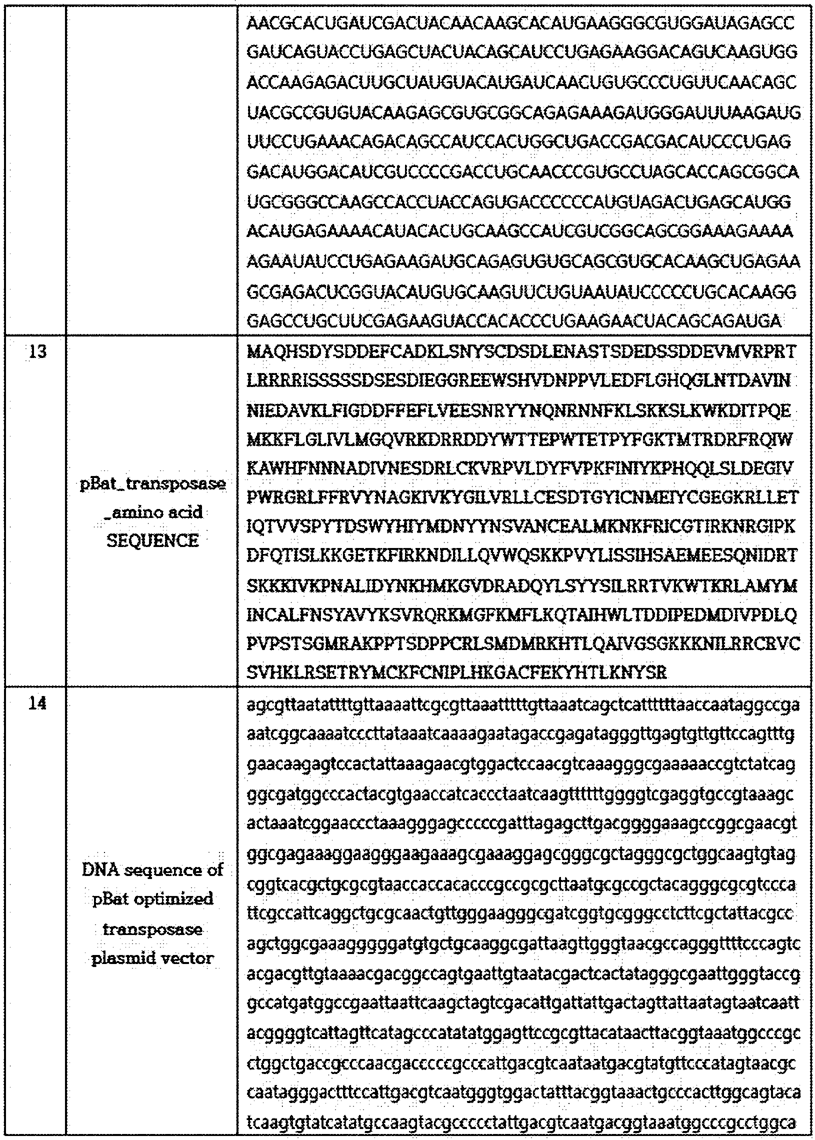

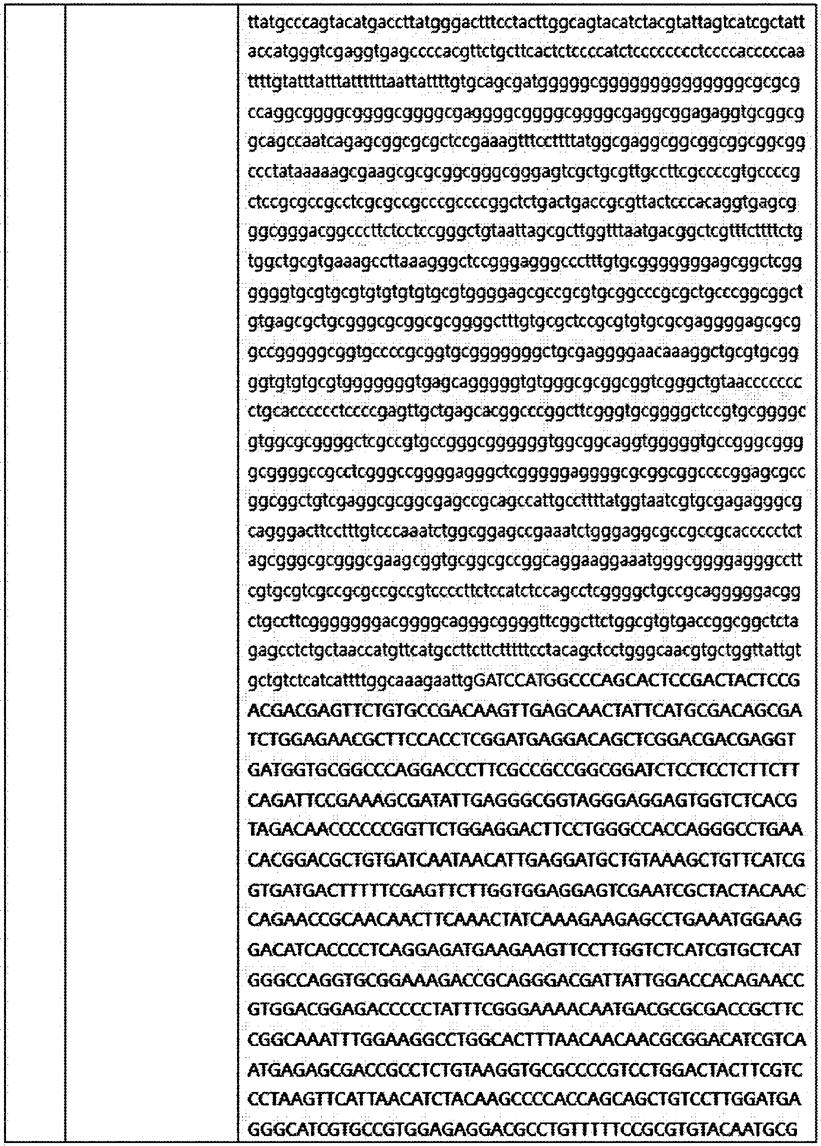

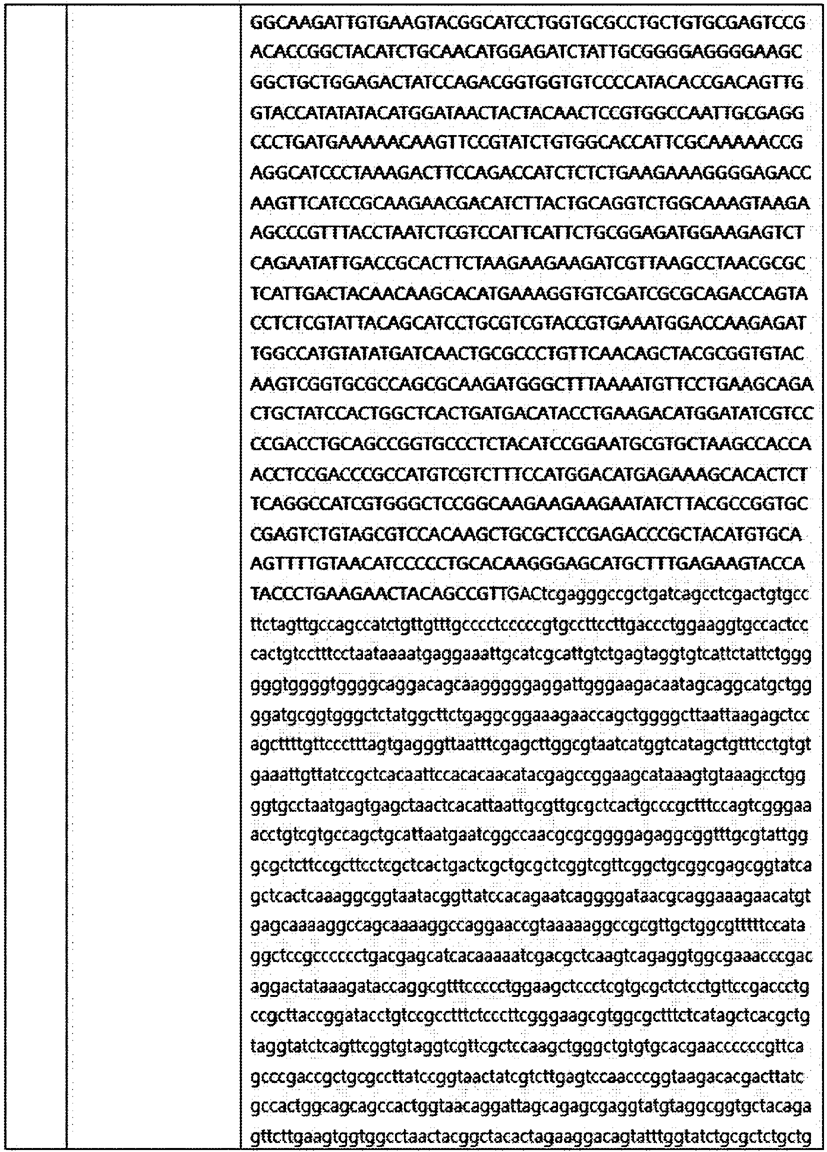

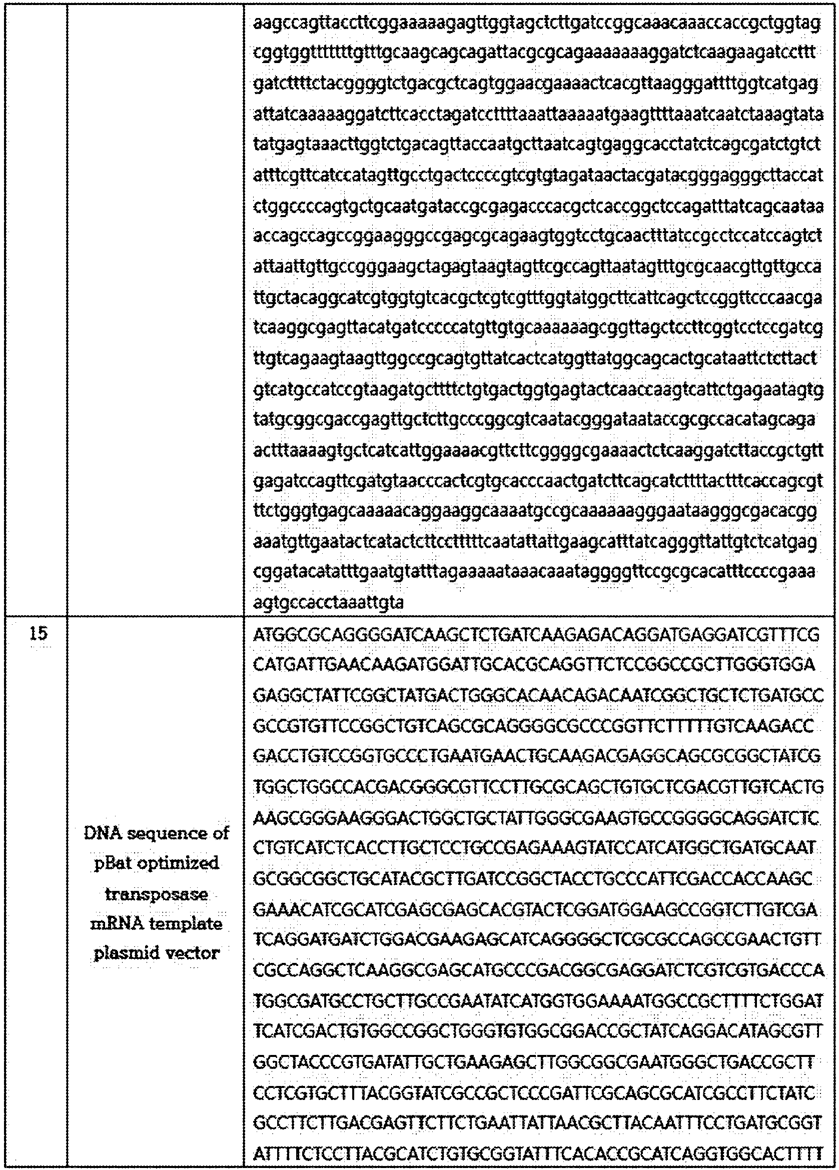

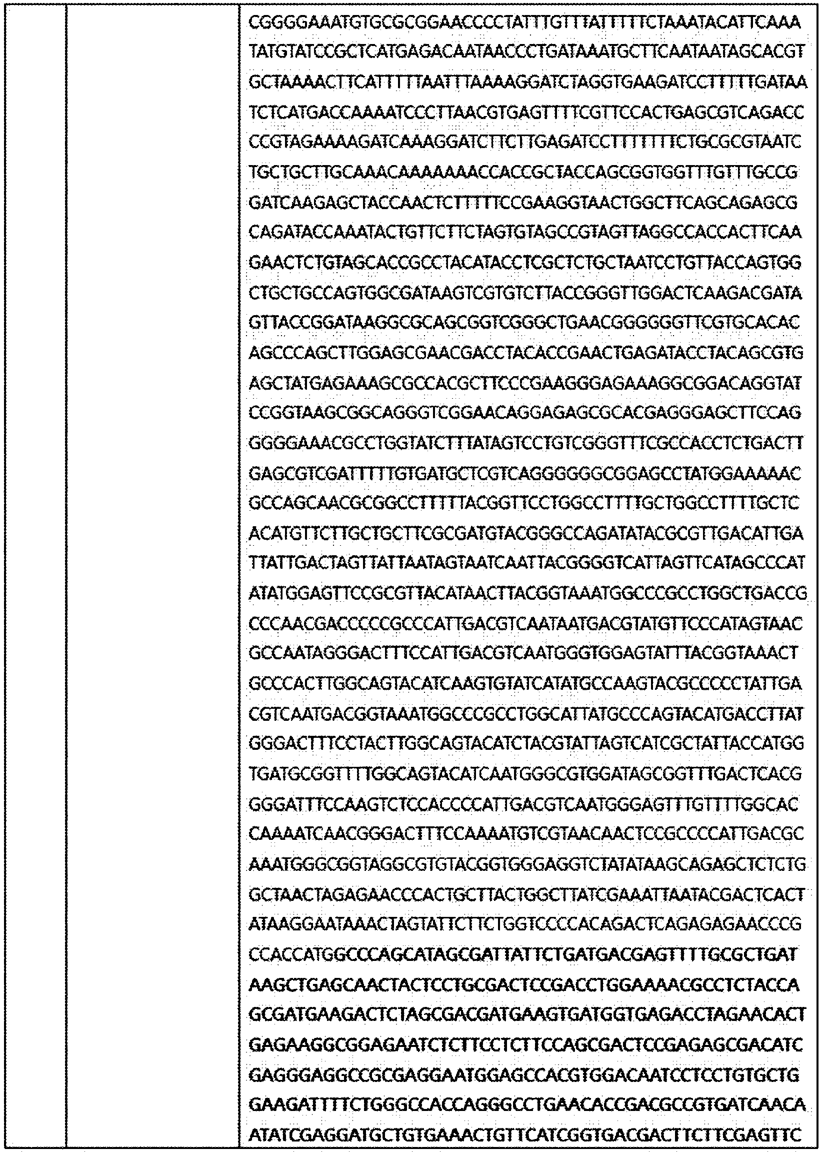

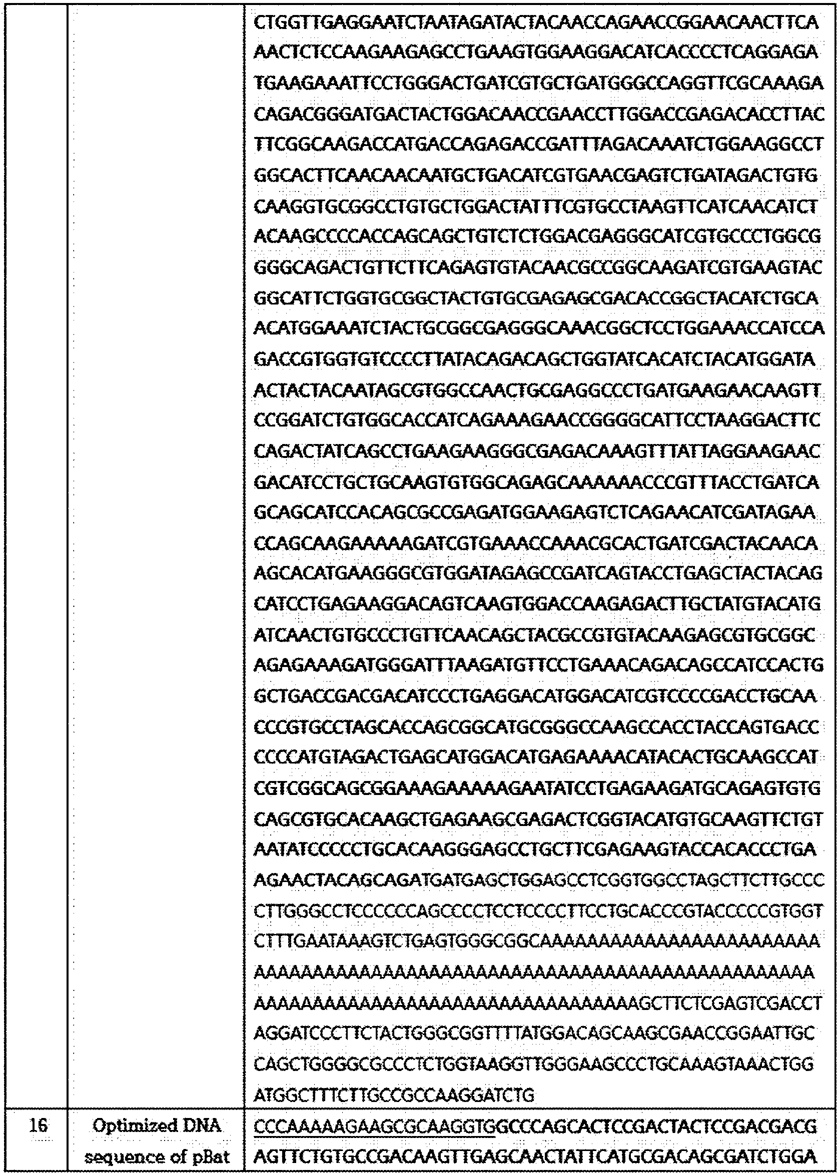

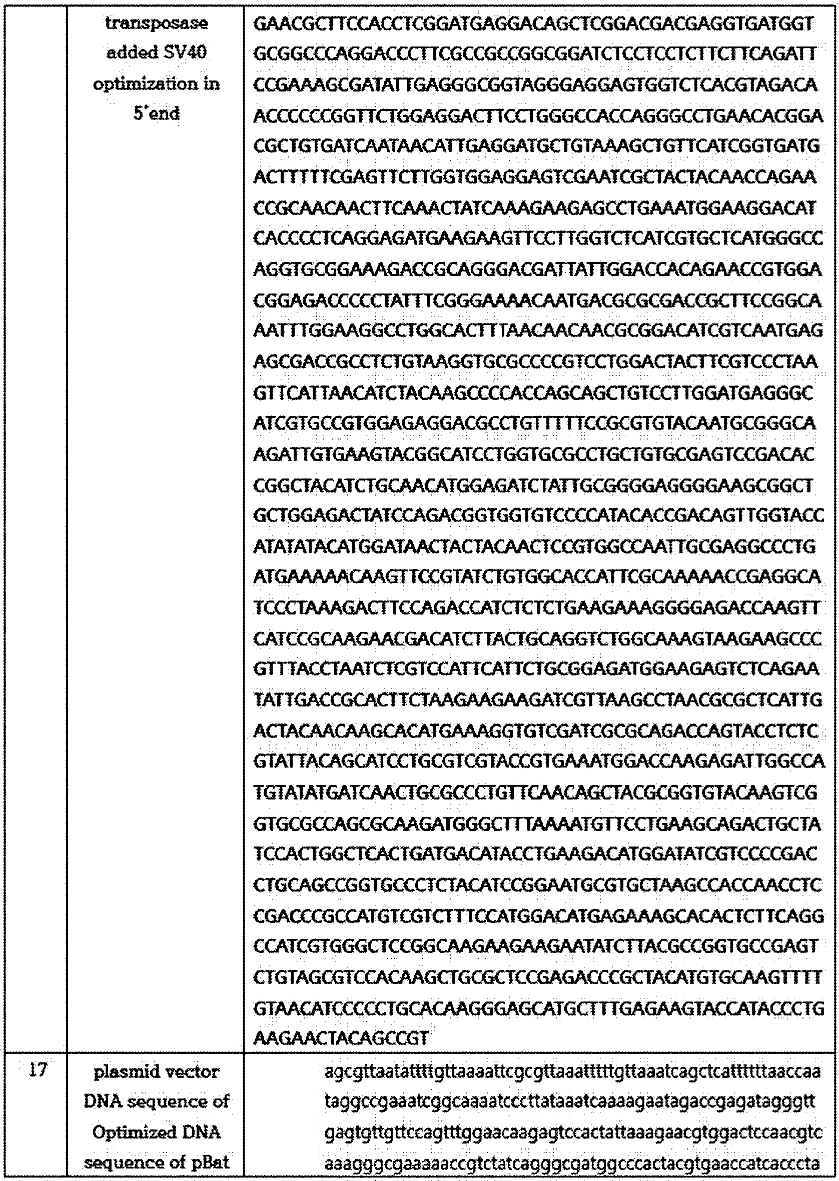

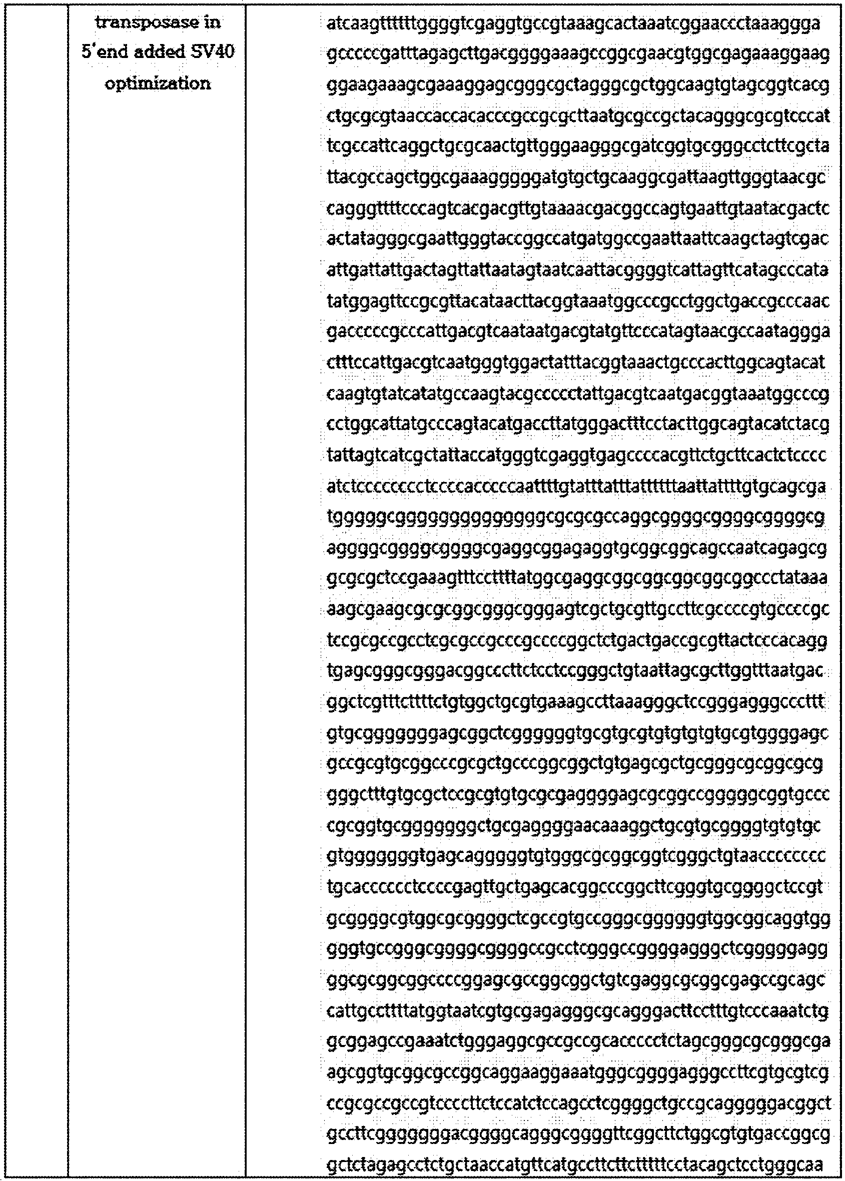

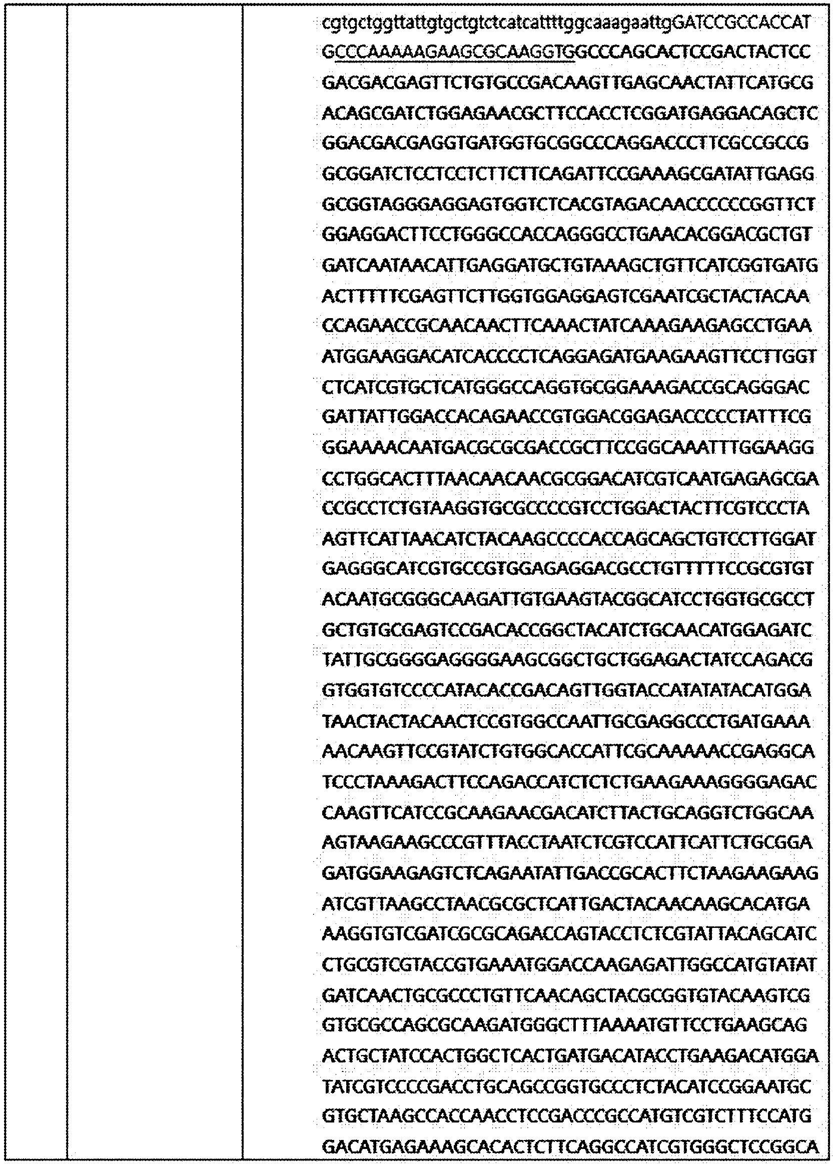

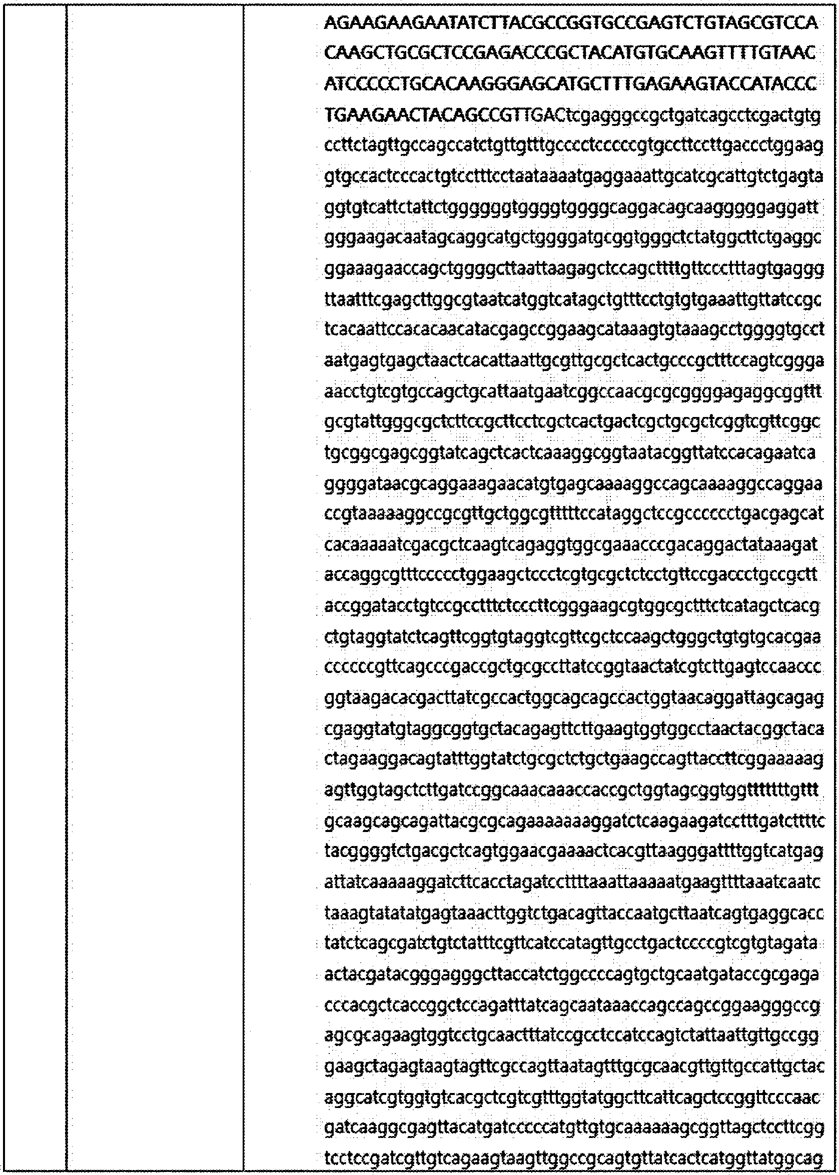

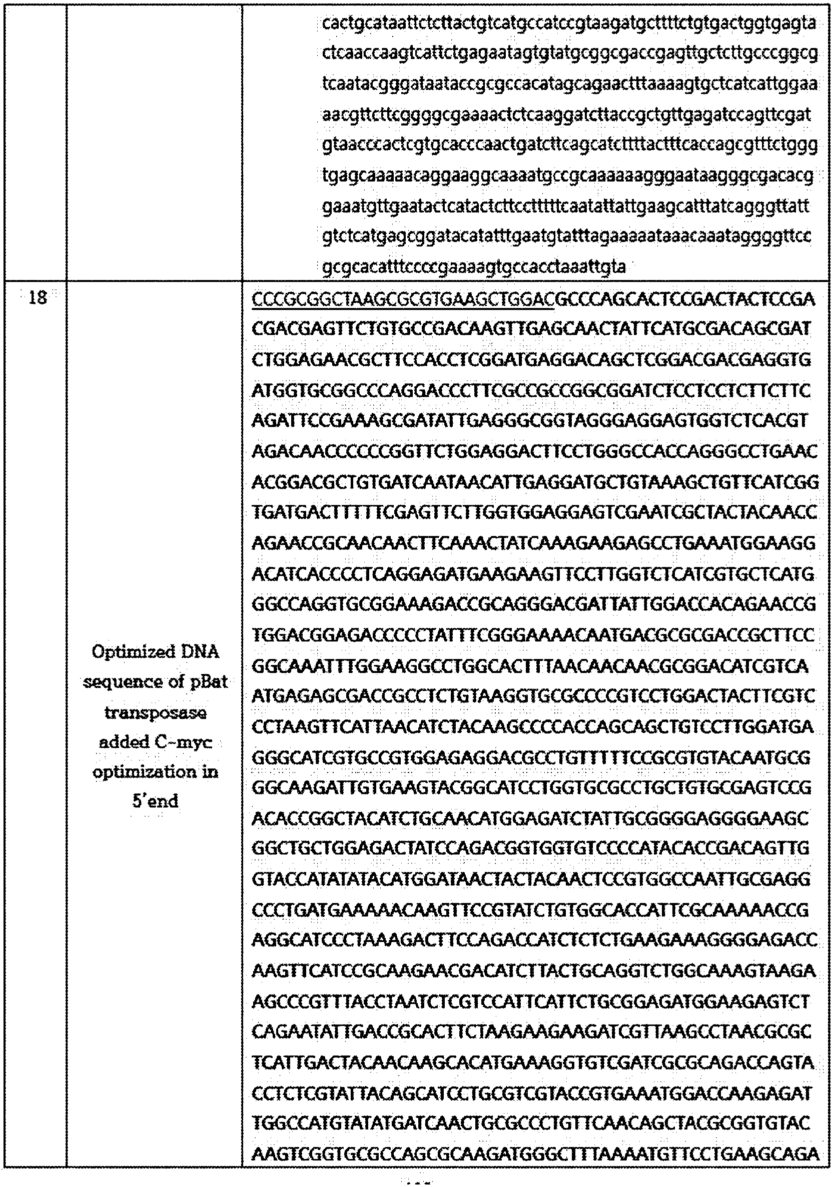

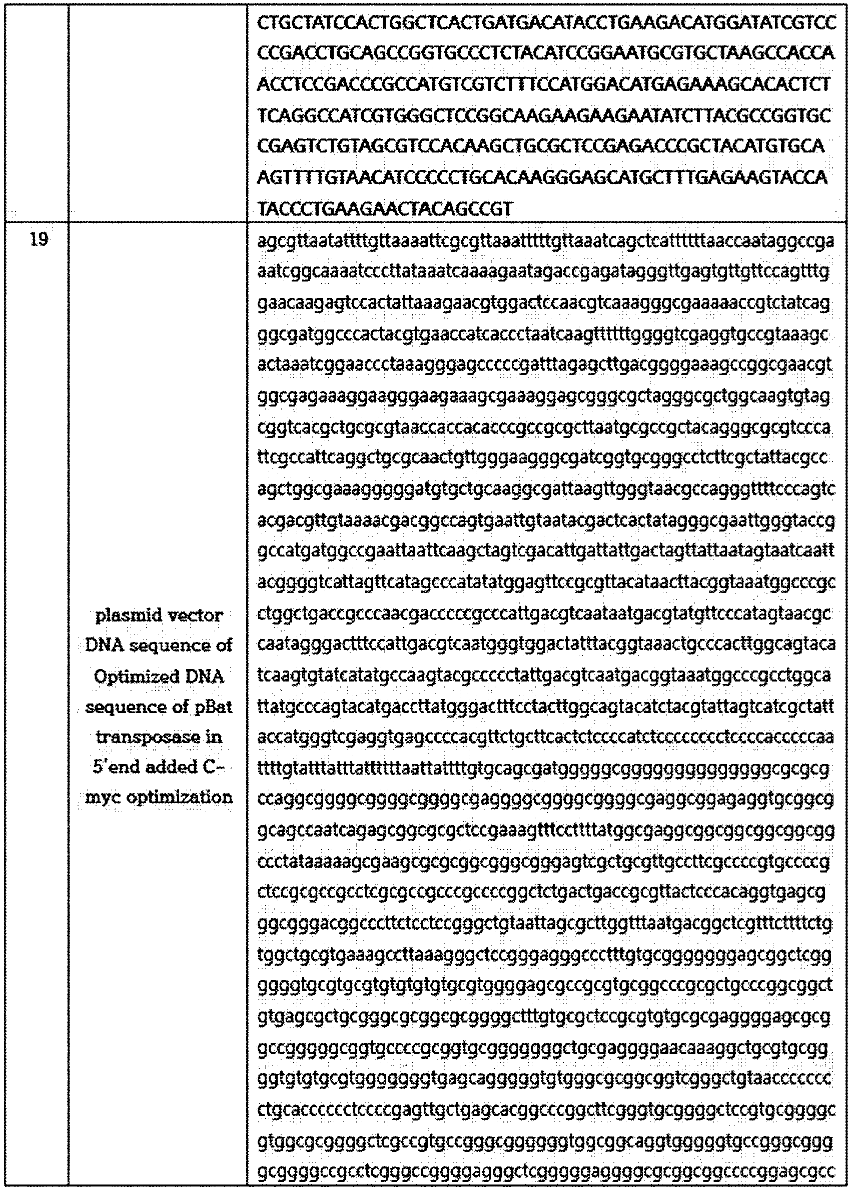

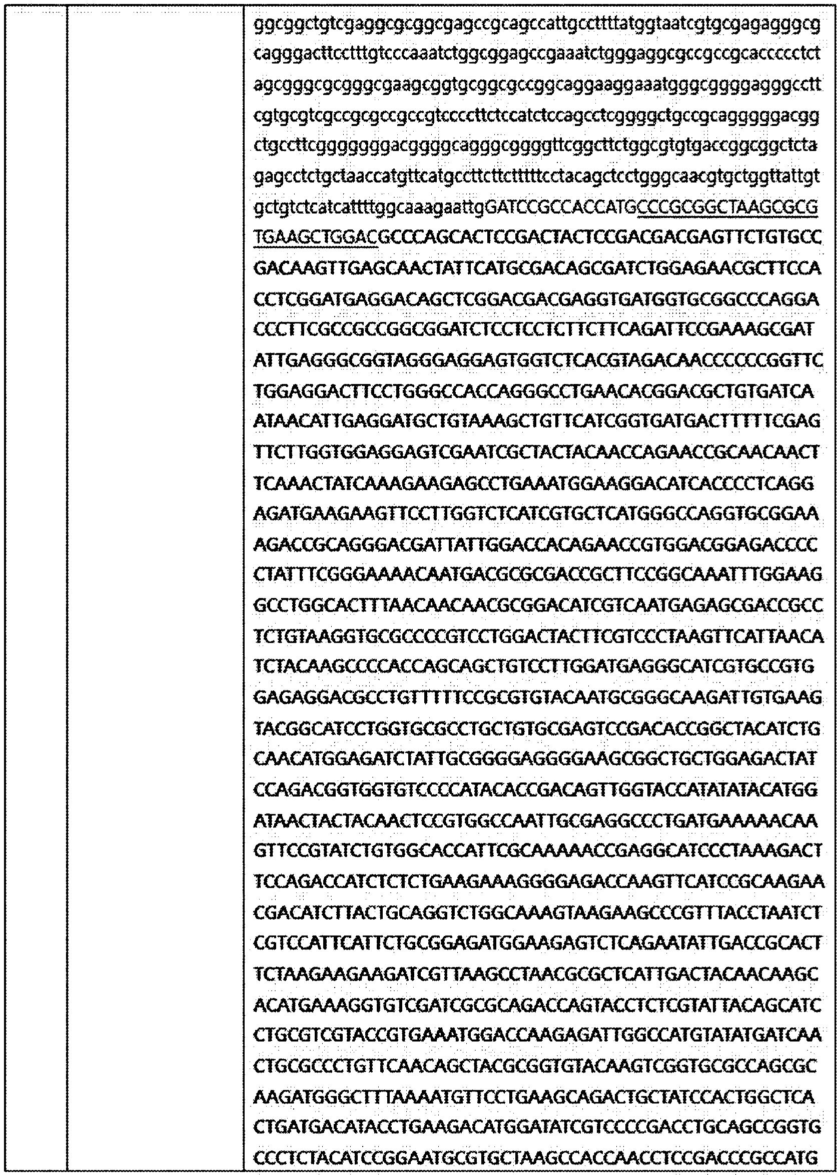

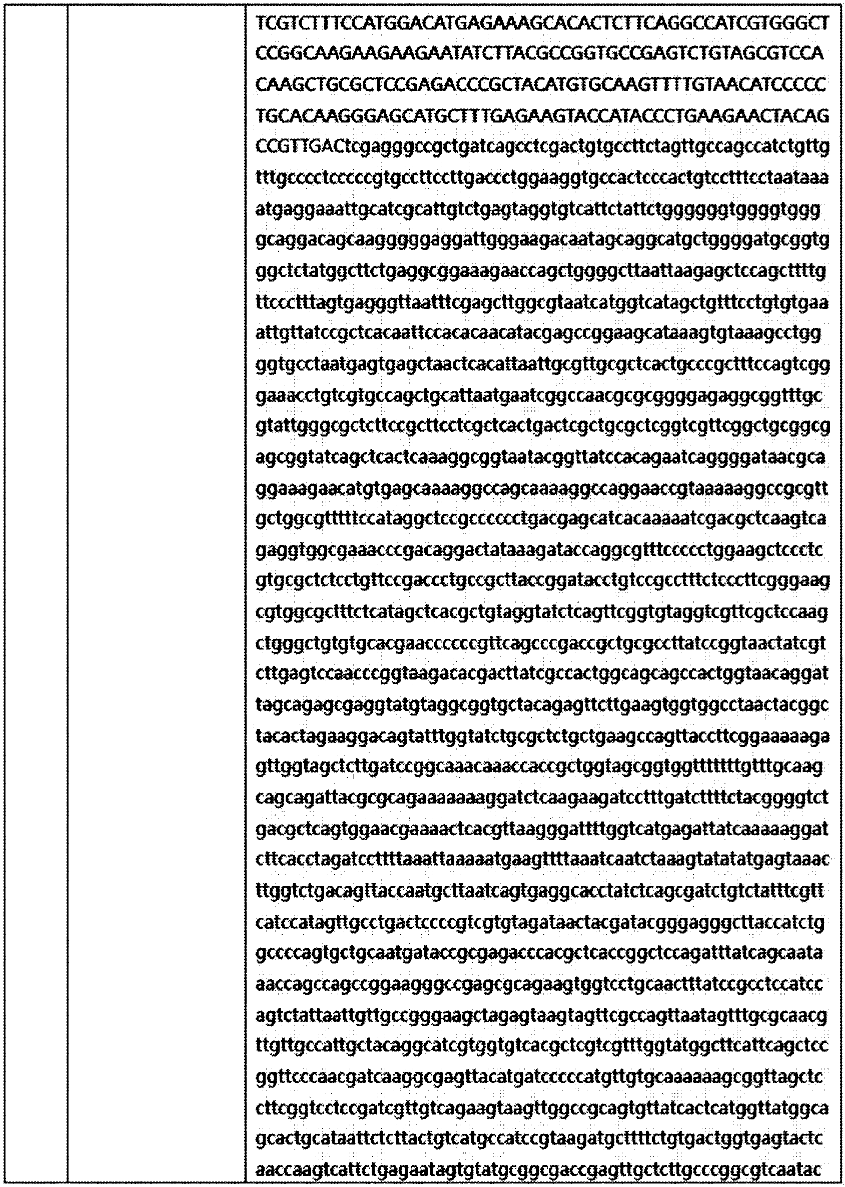

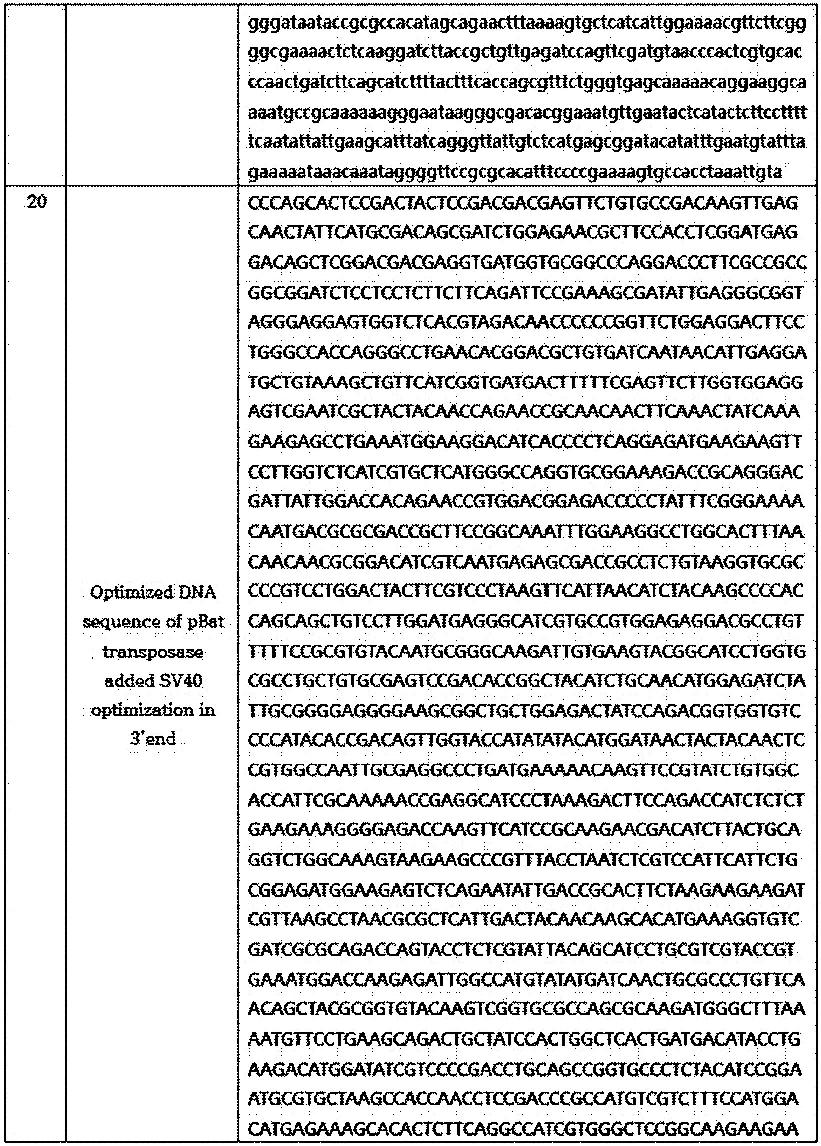

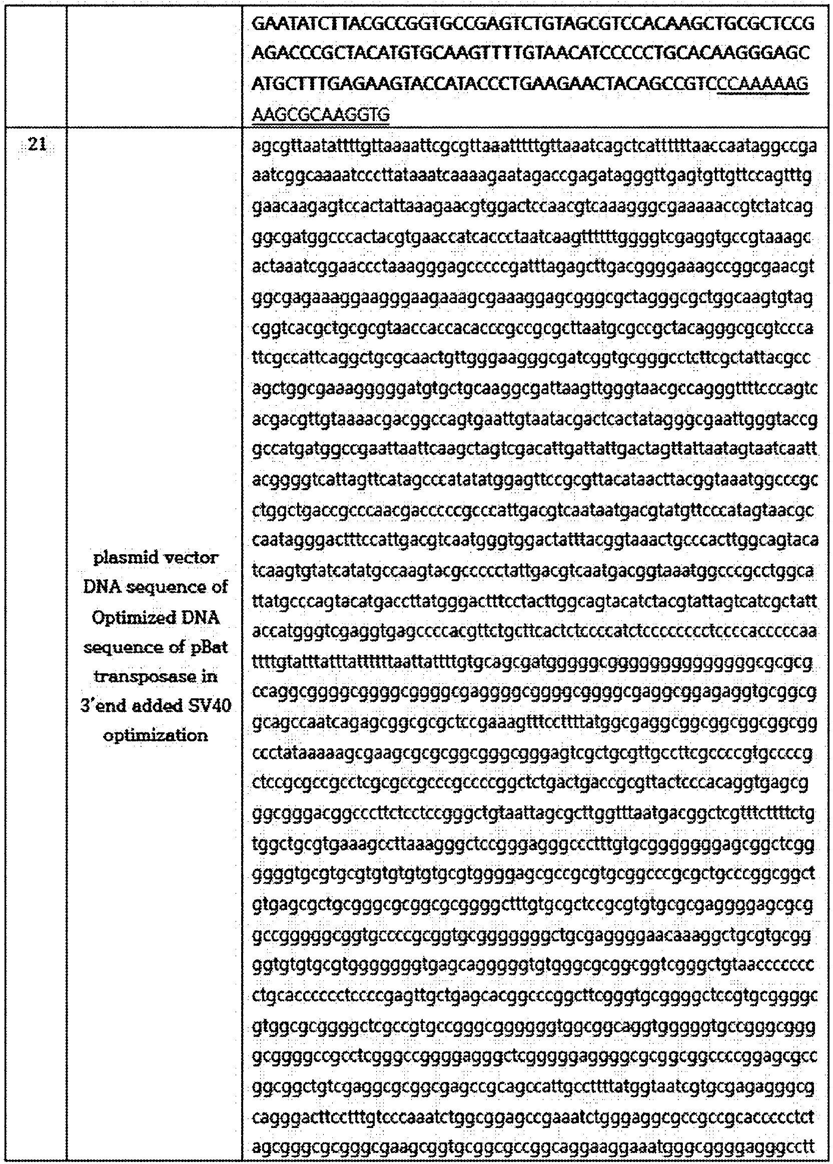

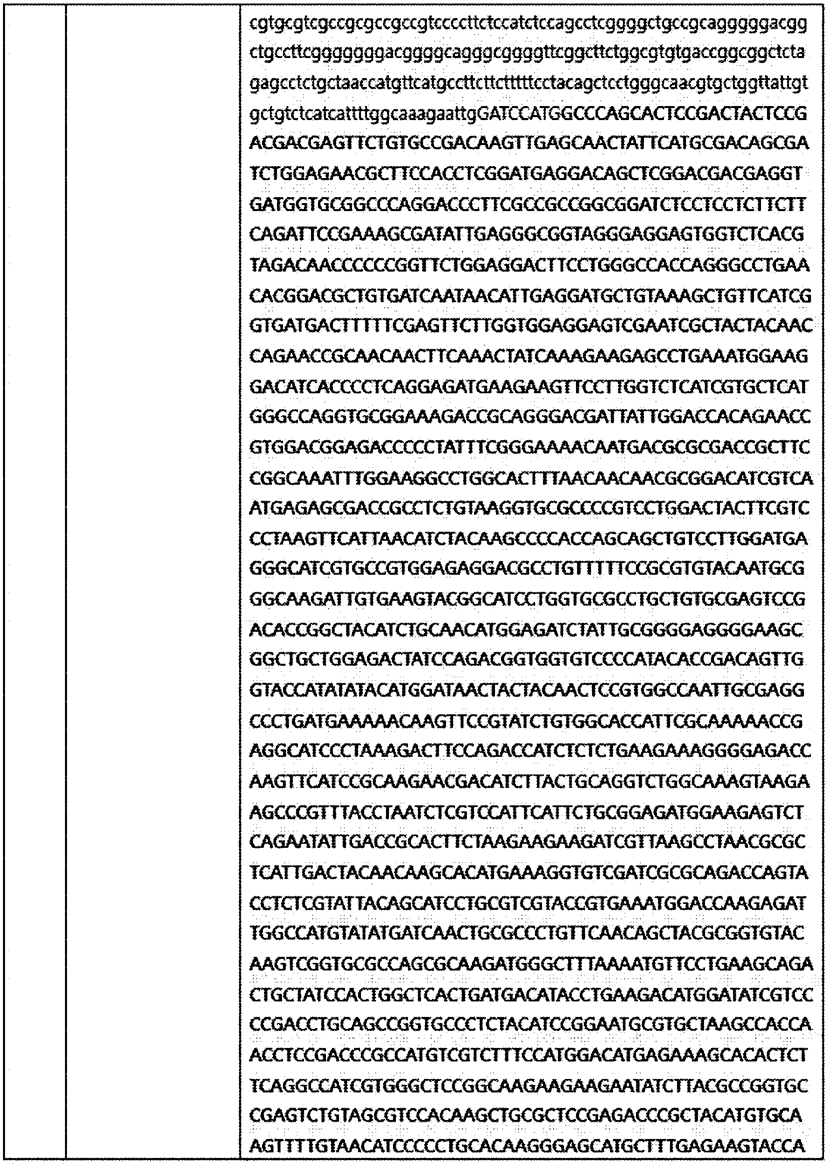

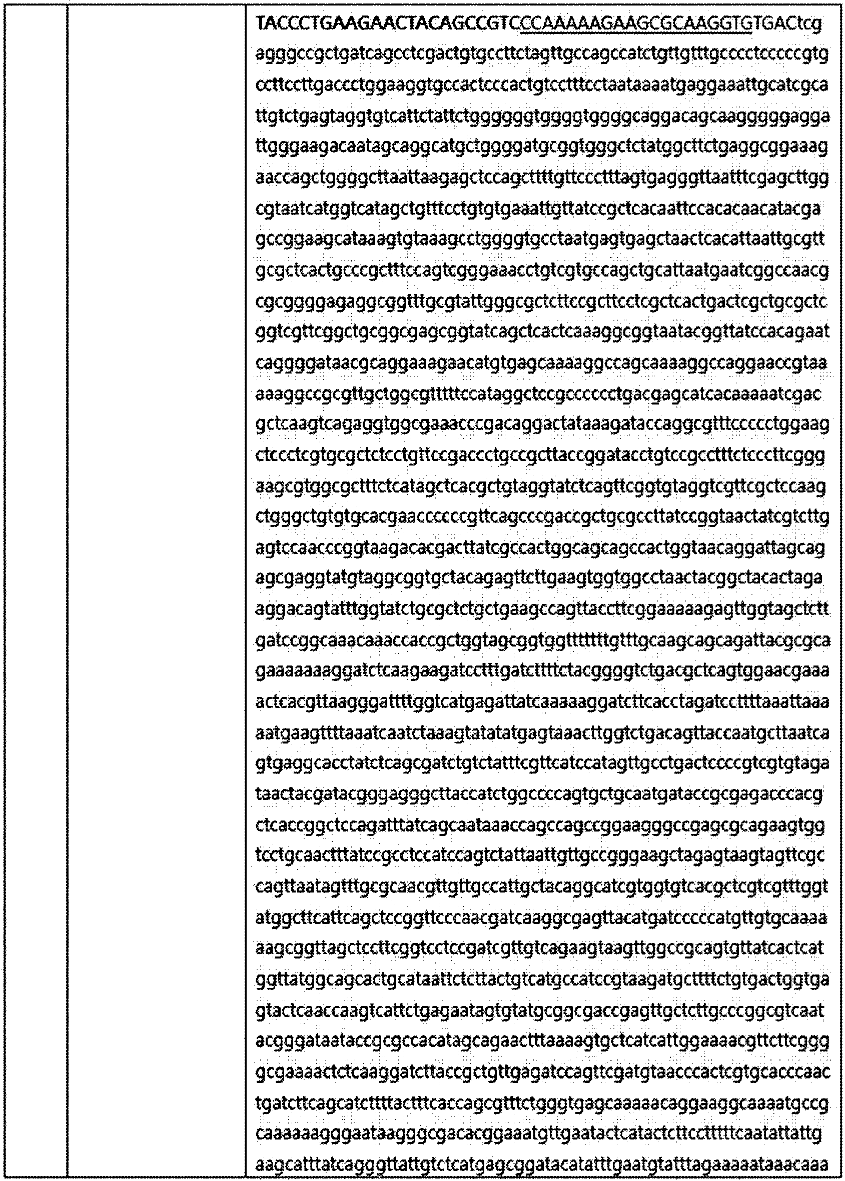

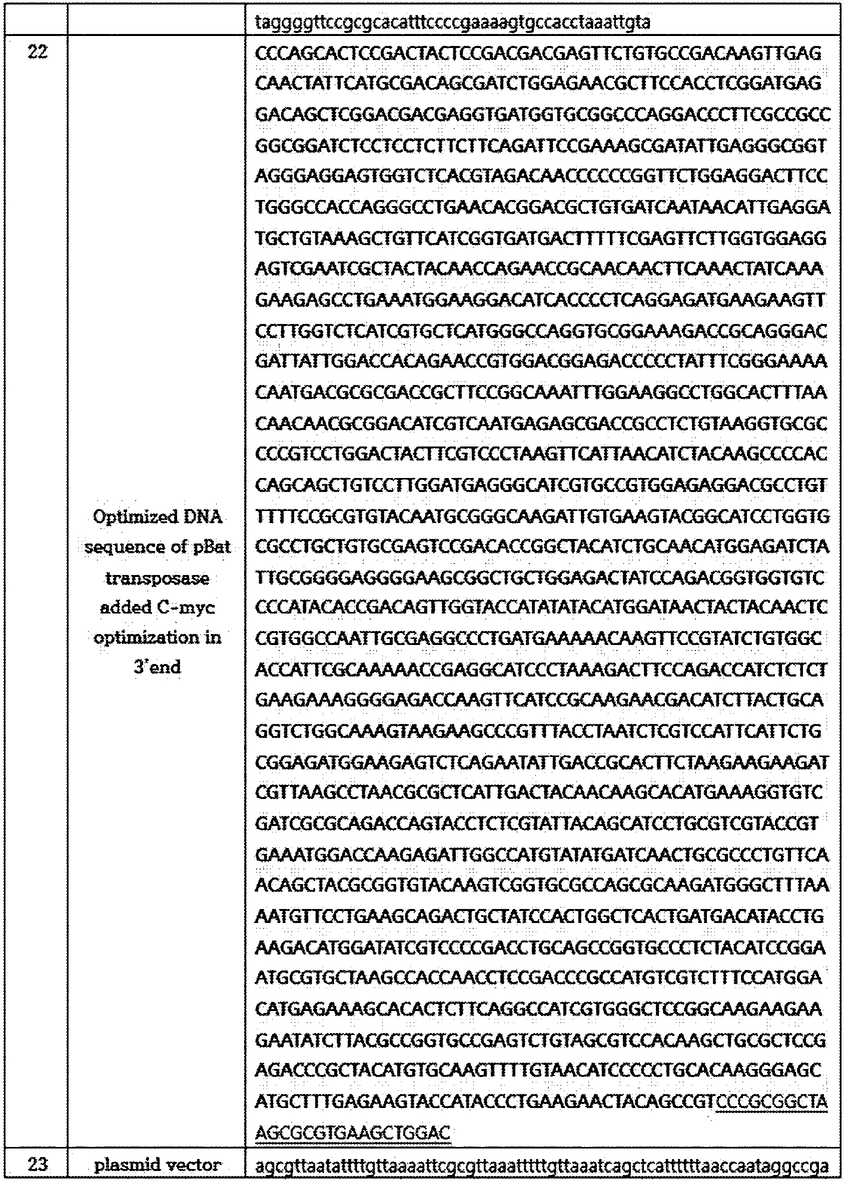

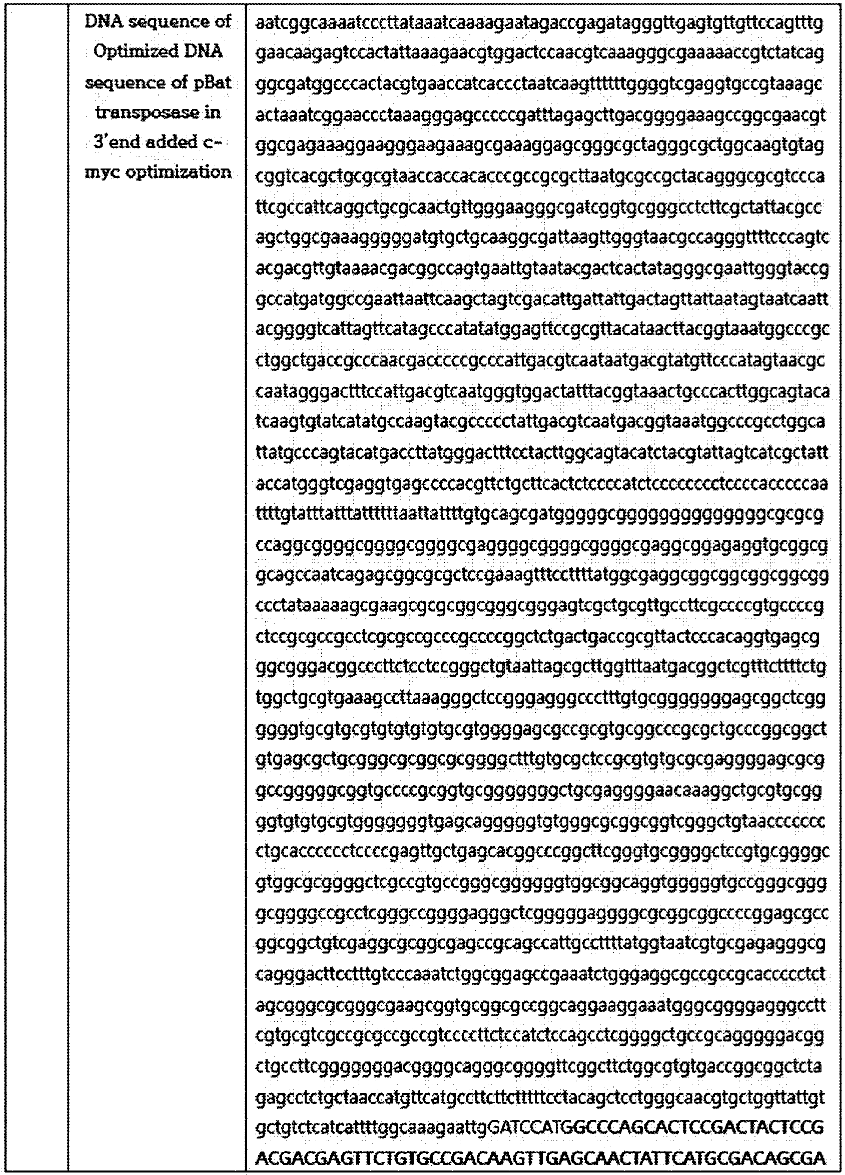

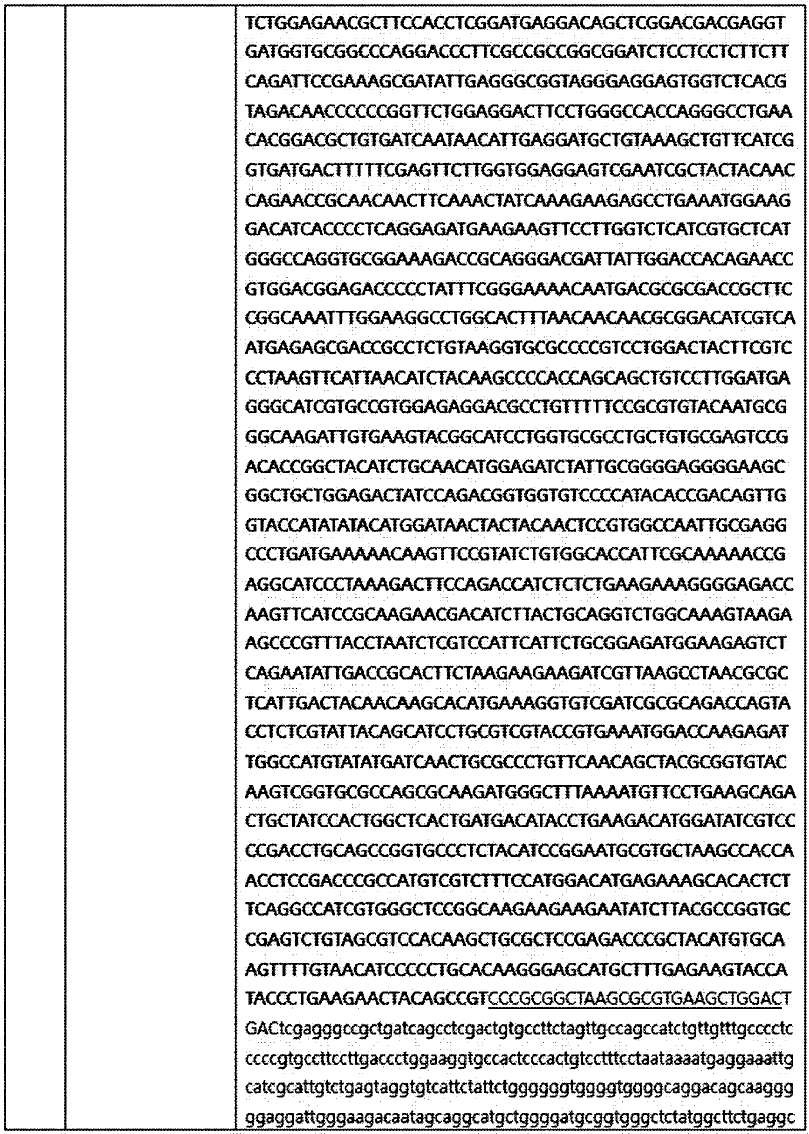

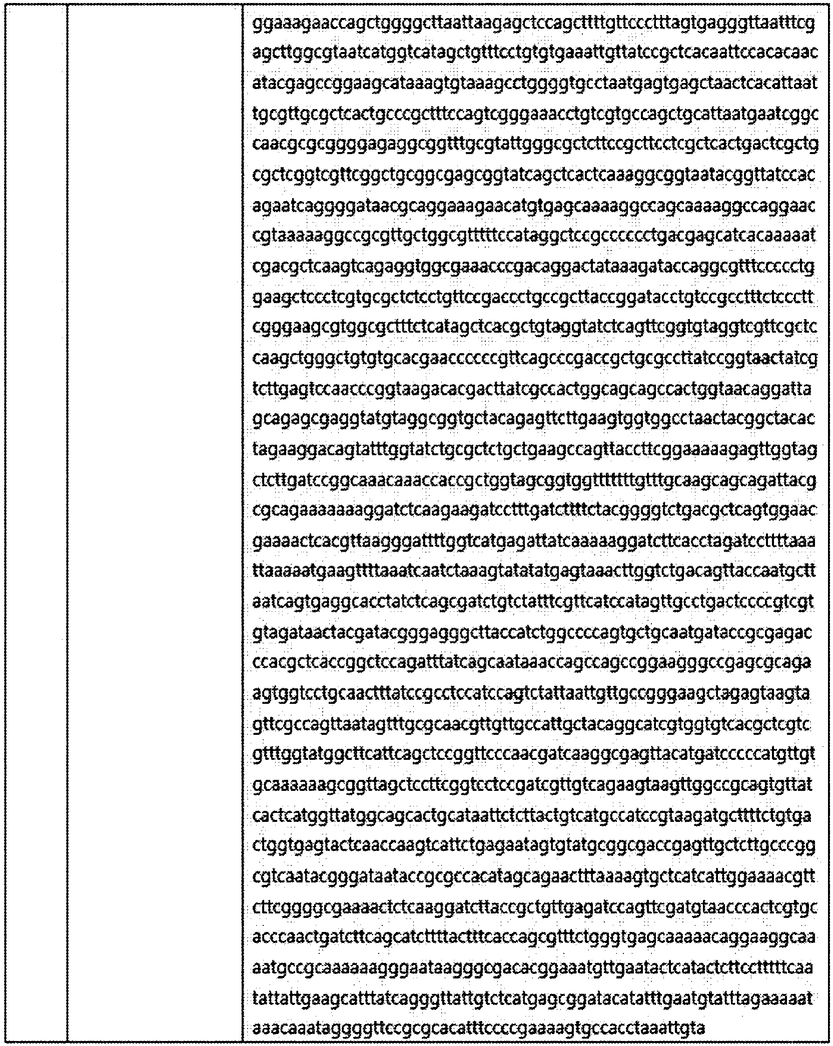

### [Industrial Applicability]

The present invention relates to a hyperactive transposase protein of a transposon system and uses thereof, and may be usefully applied to the development of genome-modified cell lines expressing various genes, as it enables effective gene delivery through enhanced activity of the transposase.

In addition, when T cells are genetically modified using the hyperactive transposase according to the present invention, the proportion of cytotoxic T (CD8⁺ T) cells exhibiting antitumor activity increases, as well as the proportion of memory-type T cells such as T_{CM} (central memory T cells) and T_{SCM} (stem cell-like memory T cells) with enhanced *in vivo* persistence, and thus the transposon system of the present invention is expected to enable the generation of TCR-T cells and CAR-T cells with excellent persistence *in vivo,* indicating industrial applicability.

## Claims

1. A transposase expression vector comprising a nucleic acid sequence encoding a transposase represented by SEQ ID NO: 2 or SEQ ID NO: 3.

2. The transposase expression vector of claim 1, wherein the nucleic acid sequence encoding the transposase further includes a nucleic acid sequence encoding a nuclear localization signal.

3. The transposase expression vector of claim 2, wherein the nucleic acid sequence encoding the nuclear localization signal is one or more of the nucleic acid sequences represented by SEQ ID NOs: 4 to 11.

4. The transposase expression vector of claim 2, wherein the nucleic acid sequence encoding the nuclear localization signal is included in a 5' to 3' direction at a 5' end or 3' end of the nucleic acid sequence encoding the transposase.

5. An mRNA encoding a transposase represented by SEQ ID NO: 12.

6. The mRNA of claim 5, wherein the mRNA is produced by performing *in vitro* transcription using the transposase expression vector of claim 1.

7. The mRNA of claim 6, wherein the transposase expression vector is a transposase expression vector including a nucleic acid sequence encoding a transposase represented by SEQ ID NO: 3.

8. A transposase expressed from the transposase expression vector of claim 1 or the mRNA encoding a transposase of claim 5.

9. The transposase of claim 8, wherein when the transposase expression vector is a transposase expression vector including a nucleic acid sequence encoding a transposase represented by SEQ ID NO: 3, the transposase is expressed from an mRNA produced by performing *in vitro* transcription using the transposase expression vector.

10. The transposase of claim 8, wherein the transposase includes an amino acid sequence represented by SEQ ID NO: 13.

11. A transposon system for delivering a target DNA, the system comprising:
a) a transposon vector including a target DNA inserted therein; and
b) a transposase expression vector including a nucleic acid sequence encoding a transposase represented by SEQ ID NO: 2 or SEQ ID NO: 3, or
an mRNA encoding a transposase represented by SEQ ID NO: 12, or
the transposase of claim 8.

12. The transposon system of claim 11, wherein the transposon vector and transposase expression vector or the mRNA encoding a transposase or the transposase are included in a mass ratio of 0.1 to 10:1.

13. A transposon kit for delivering a target DNA, the kit comprising:
the transposon system for delivering a target DNA of claim 11; and instructions.

14. A cell into which:
a) a transposon vector including a target DNA inserted therein; and
b) a transposase expression vector including a nucleic acid sequence encoding a transposase represented by SEQ ID NO: 2 or SEQ ID NO: 3, or
an mRNA encoding a transposase represented by SEQ ID NO: 12, or
the transposase of claim 8, is introduced.

15. The cell of claim 14, wherein the target DNA is cleaved from the transposon vector by the transposase within the cell, and the cleaved target DNA is inserted into the genome of the cell.

16. The cell of claim 14, wherein the cell is selected from the group consisting of a T cell, an NK cell, a B cell, a dendritic cell, a macrophage, and a mast cell.

17. The cell of claim 14, wherein the cell is co-cultured with feeder cells after the transposon vector is introduced.

18. The cell of claim 17, wherein the feeder cells are cells that have been irradiated with radiation.

19. The cell of claim 14, wherein the cell expresses the target DNA for more than seven or more days after the transposon vector is introduced.

20. A method of inserting a target DNA sequence into a genome of a cell, comprising: a step of introducing a) and b) into the cell.
a) a transposon vector including a target DNA inserted therein; and
b) a transposase expression vector including a nucleic acid sequence encoding a transposase represented by SEQ ID NO: 2 or SEQ ID NO: 3, or
an mRNA encoding a transposase represented by SEQ ID NO: 12, or
the transposase of claim 8.

21. The method of claim 20, wherein the introduction is performed through electroporation.

22. The method of claim 20, further comprising: after the introduction step, a step of co-culturing the cell into which the transposon vector is inserted, with feeder cells.

23. The method of claim 22, wherein the step of co-culturing the cell with feeder cells is performed immediately after the introduction step.
